(19) **Europäisches Patentamt / European Patent Office / Office européen des brevets**

(11) **EP 4 742 250 A2**

(12) # EUROPEAN PATENT APPLICATION

(43) Date of publication:
**13.05.2026 Bulletin 2026/20**

(21) Application number: **25226976.6**

(22) Date of filing: **28.12.2022**

(51) International Patent Classification (IPC):
***G16B 40/20*** (2019.01)

(52) Cooperative Patent Classification (CPC):
**C12Q 1/6827; G16B 5/10; G16B 20/20;
G16B 20/40; G16B 25/10; G16B 30/10;
G16B 40/20; G16H 10/40; G16H 20/10;
G16H 50/20; G16H 50/30; G16H 50/70;**
G16B 15/30

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: 29.12.2021 US 202163294813 P
29.12.2021 US 202163294816 P
29.12.2021 US 202163294820 P
29.12.2021 US 202163294827 P
29.12.2021 US 202163294828 P
29.12.2021 US 202163294830 P
10.06.2022 US 202263351283 P
10.06.2022 US 202263351299 P
10.06.2022 US 202263351317 P
18.10.2022 US 202217968285

(62) Document number(s) of the earlier application(s) in
accordance with Art. 76 EPC:
**22854642.0 / 4 457 818**

(71) Applicant: **ILLUMINA, INC.**
**San Diego, CA 92122 (US)**

(72) Inventors:
• **FIZIEV, Petko Plamenov**
**92122 San Diego (US)**
• **MCRAE, Jeremy Francis**
**92122 San Diego (US)**
• **FARH, Kai-How**
**92122 San Diego (US)**

(74) Representative: **Robinson, David Edward
Ashdown
Marks & Clerk LLP
Wytham Court, 11 West Way
Oxford OX2 0JB (GB)**

Remarks:
•This application was filed on 23-12-2025 as a
divisional application to the application mentioned
under INID code 62.
•Claims filed after the date of filing of the application/
the date of receipt of the divisional application (Rule
68(4) EPC).

(54) **OPTIMIZED BURDEN TEST BASED ON NESTED T-TESTS THAT MAXIMIZE SEPARATION BETWEEN CARRIERS AND NON-CARRIERS**

(57)     A computer-implemented method of performing an optimized burden test for a particular gene, in which an optimal combination of a maximum allele count and a minimum pathogenicity score threshold that maximize significance of burden testing for rare deleterious variants are determined using a grid search protocol. Each combination of maximum allele count and minimum pathogenicity score threshold is tested with a t-test to obtain effect size and p-value. The combination of allele count and pathogenicity score threshold with the most significant p-value is selected as the optimal parameters for the rare deleterious variant burden test for a particular gene.

EP 4 742 250 A2

Figure 1A

| 108 | Gene | Effect Size | Carrier Status |
|---|---|---|---|
| 118 | Gene A | Effect Size A | Carrier |
| 128 | Gene B | Effect Size B | NonCarrier |
| 138 | Gene C | Effect Size C | Carrier |
| 148 | Gene D | Effect Size F | Carrier |
| 158 | Gene E | Effect Size E | Carrier |

Output weighted
burden score
162

164

Rare Variant Polygenic Risk Score =
$\sum$ (Effect Size * Carrier Status)

182

Rare Variant Polygenic Risk Score for Phenotype Y =
Effect Size A + Effect Size C + Effect Size D + Effect Size E

# Figure 1B

**Description**

**PRIORITY APPLICATIONS**

This application claims the benefit of and priority to the following:

U.S. Provisional Patent Application No.: 63/294,813, titled "PERIODIC MASK PATTERN FOR REVELATION LANGUAGE MODELS," filed December 29, 2021 (Attorney Docket No. ILLM 1063-1/IP-2296-PRV);

U.S. Provisional Patent Application No.: 63/294,816, titled "CLASSIFYING MILLIONS OF VARIANTS OF UNCERTAIN SIGNIFICANCE USING PRIMATE SEQUENCING AND DEEP LEARNING," filed December 29, 2021 (Attorney Docket No. ILLM 1064-1/IP-2297-PRV);

U.S. Provisional Patent Application No.: 63/294,820, titled "IDENTIFYING GENES WITH DIFFERENTIAL SELECTIVE CONSTRAINT BETWEEN HUMANS AND NON-HUMAN PRIMATES," filed December 29, 2021 (Attorney Docket No. ILLM 1065-1/IP-2298-PRV);

U.S. Provisional Patent Application No.: 63/294,827, titled "DEEP LEARNING NETWORK FOR EVOLUTIONARY CONSERVATION," filed December 29, 2021 (Attorney Docket No. ILLM 1066-1/IP-2299-PRV);

U.S. Provisional Patent Application No.: 63/294,828, titled "INTER-MODEL PREDICTION SCORE RECALIBRATION," filed December 29, 2021 (Attorney Docket No. ILLM 1067-1/IP-2301-PRV);

U.S. Provisional Patent Application No.: 63/294,830, titled "SPECIES-DIFFERENTIABLE EVOLUTIONARY PROFILES," filed December 29, 2021 (Attorney Docket No. ILLM 1068-1/IP-2302-PRV);

U.S. Provisional Patent Application No.: 63/351,283, titled "OPTIMIZED BURDEN TEST BASED ON NESTED T-TESTS THAT MAXIMIZE SEPARATION BETWEEN CARRIERS AND NON-CARRIERS," filed June 10, 2022 (Attorney Docket No. ILLM 1070-1/IP-2368-PRV);

U.S. Provisional Patent Application No.: 63/351,299, titled "RARE VARIANT POLYGENIC RISK SCORES," filed June 10, 2022 (Attorney Docket No. ILLM 1071-1/IP-2378-PRV); and

U.S. Provisional Patent Application No.: 63/351,317, titled "COVARIATE CORRECTION INCLUDING DRUG USE FROM TEMPORAL DATA," filed June 10, 2022 (Attorney Docket No. ILLM 1073-1/IP-2387-PRV).

The priority applications are incorporated by reference as if fully set forth herein.

**FIELD OF THE TECHNOLOGY DISCLOSED**

The technology disclosed relates to artificial intelligence type computers and digital data processing systems and corresponding data processing methods and products for emulation of intelligence (*i.e.,* knowledge based systems, reasoning systems, and knowledge acquisition systems); and including systems for reasoning with uncertainty (*e.g*., fuzzy logic systems), adaptive systems, machine learning systems, and artificial neural networks. In particular, the technology disclosed relates to using deep convolutional neural networks to analyze ordered data.

**RELATED APPLICATIONS**

This application is related to US Nonprovisional Patent Application titled "RARE VARIANT POLYGENIC RISK SCORES" (Attorney Docket No. ILLM 1071-2/IP-2378-US), filed contemporaneously. The related application is hereby incorporated by reference for all purposes.
This application is related to US Nonprovisional Patent Application titled "COVARIATE CORRECTION INCLUDING DRUG USE FROM TEMPORAL DATA" (Attorney Docket No. ILLM 1073-2/IP-2387-US), filed contemporaneously. The related application is hereby incorporated by reference for all purposes.

**INCORPORATIONS BY REFERENCE**

The following are incorporated by reference for all purposes as if fully set forth herein:

Sundaram, L. et al. Predicting the clinical impact of human mutation with deep neural networks. Nat. Genet. 50, 1161-1170 (2018);

Jaganathan, K. et al. Predicting splicing from primary sequence with deep learning. Cell 176, 535-548 (2019);

US Patent Application No. 62/573,144, titled "TRAINING A DEEP PATHOGENICITY CLASSIFIER USING LARGE-SCALE BENIGN TRAINING DATA," filed October 16, 2017 (Attorney Docket No. ILLM 1000-1/IP-1611-PRV);

US Patent Application No. 62/573,149, titled "PATHOGENICITY CLASSIFIER BASED ON DEEP CONVOLUTIONAL NEURAL NETWORKS (CNNs)," filed October 16, 2017 (Attorney Docket No. ILLM 1000-2/IP-1612-PRV);

US Patent Application No. 62/573,153, titled "DEEP SEMI-SUPERVISED LEARNING THAT GENERATES LARGE-SCALE PATHOGENIC TRAINING DATA," filed October 16, 2017 (Attorney Docket No. ILLM 1000-3/IP-1613-PRV);

US Patent Application No. 62/582,898, titled "PATHOGENICITY CLASSIFICATION OF GENOMIC DATA USING DEEP CONVOLUTIONAL NEURAL NETWORKS (CNNs)," filed November 7, 2017 (Attorney Docket No. ILLM 1000-4/IP-1618-PRV);

US Patent Application No. 16/160,903, titled "DEEP LEARNING-BASED TECHNIQUES FOR TRAINING DEEP CONVOLUTIONAL NEURAL NETWORKS," filed on October 15, 2018 (Attorney Docket No. ILLM 1000-5/IP-1611-US);

US Patent Application No. 16/160,986, titled "DEEP CONVOLUTIONAL NEURAL NETWORKS FOR VARIANT CLASSIFICATION," filed on October 15, 2018 (Attorney Docket No. ILLM 1000-6/IP-1612-US);

US Patent Application No. 16/160,968, titled "SEMI-SUPERVISED LEARNING FOR TRAINING AN ENSEMBLE OF DEEP CONVOLUTIONAL NEURAL NETWORKS," filed on October 15, 2018 (Attorney Docket No. ILLM 1000-7/IP-1613-US);

US Patent Application No. 16/160,978, titled "DEEP LEARNING-BASED SPLICE SITE CLASSIFICATION," filed on October 15, 2018 (Attorney Docket No. ILLM 1001-4/IP-1680-US);

US Patent Application No. 16/407,149, titled "DEEP LEARNING-BASED TECHNIQUES FOR PRE-TRAINING DEEP CONVOLUTIONAL NEURAL NETWORKS," filed May 8, 2019 (Attorney Docket No. ILLM 1010-1/IP-1734-US);

US Patent Application No. 17/232,056, titled "DEEP CONVOLUTIONAL NEURAL NETWORKS TO PREDICT VARIANT PATHOGENICITY USING THREE-DIMENSIONAL (3D) PROTEIN STRUCTURES," filed on April 15, 2021, (Atty. Docket No. ILLM 1037-2/IP-2051-US);

US Patent Application No. 63/175,495, titled "MULTI-CHANNEL PROTEIN VOXELIZATION TO PREDICT VARIANT PATHOGENICITY USING DEEP CONVOLUTIONAL NEURAL NETWORKS," filed on April 15, 2021, (Atty. Docket No. ILLM 1047-1/IP-2142-PRV);

US Patent Application No. 63/175,767, titled "EFFICIENT VOXELIZATION FOR DEEP LEARNING," filed on April 16, 2021, (Atty. Docket No. ILLM 1048-1/IP-2143-PRV);

US Patent Application No. 17/468,411, titled "ARTIFICIAL INTELLIGENCE-BASED ANALYSIS OF PROTEIN THREE-DIMENSIONAL (3D) STRUCTURES," filed on September 7, 2021, (Atty. Docket No. ILLM 1037-3/IP-2051A-US);

U.S. Provisional Patent Application No.: 63/253,122, titled "PROTEIN STRUCTURE-BASED PROTEIN LANGUAGE MODELS," filed October 6, 2021 (Attorney Docket No. ILLM 1050-1/IP-2164-PRV);

U.S. Provisional Patent Application No.: 63/281,579, titled "PREDICTING VARIANT PATHOGENICITY FROM EVOLUTIONARY CONSERVATION USING THREE-DIMENSIONAL (3D) PROTEIN STRUCTURE VOXELS," filed November 19, 2021 (Attorney Docket No. ILLM 1060-1/IP-2270-PRV);

U.S. Provisional Patent Application No.: 63/281,592, titled "COMBINED AND TRANSFER LEARNING OF A VARIANT PATHOGENICITY PREDICTOR USING GAPED AND NON-GAPED PROTEIN SAMPLES," filed November 19, 2021 (Attorney Docket No. ILLM 1061-1/IP-2271-PRV);

U.S. Provisional Patent Application No.: 63/294,813, titled "PERIODIC MASK PATTERN FOR REVELATION LANGUAGE MODELS," filed December 29, 2021 (Attorney Docket No. ILLM 1063-1/IP-2296-PRV);

U.S. Provisional Patent Application No.: 63/294,816, titled "CLASSIFYING MILLIONS OF VARIANTS OF UNCERTAIN SIGNIFICANCE USING PRIMATE SEQUENCING AND DEEP LEARNING," filed December 29, 2021 (Attorney Docket No. ILLM 1064-1/IP-2297-PRV);

U.S. Provisional Patent Application No.: 63/294,820, titled "IDENTIFYING GENES WITH DIFFERENTIAL SELECTIVE CONSTRAINT BETWEEN HUMANS AND NON-HUMAN PRIMATES," filed December 29, 2021 (Attorney Docket No. ILLM 1065-1/IP-2298-PRV);

U.S. Provisional Patent Application No.: 63/294,827, titled "DEEP LEARNING NETWORK FOR EVOLUTIONARY CONSERVATION," filed December 29, 2021 (Attorney Docket No. ILLM 1066-1/IP-2299-PRV);

U.S. Provisional Patent Application No.: 63/294,828, titled "INTER-MODEL PREDICTION SCORE RECALIBRATION," filed December 29, 2021 (Attorney Docket No. ILLM 1067-1/IP-2301-PRV); and

U.S. Provisional Patent Application No.: 63/294,830, titled "SPECIES-DIFFERENTIABLE EVOLUTIONARY PROFILES," filed December 29, 2021 (Attorney Docket No. ILLM 1068-1/IP-2302-PRV).

## BACKGROUND

The subject matter discussed in this section should not be assumed to be prior art merely as a result of its mention in this section. Similarly, a problem mentioned in this section or associated with the subject matter provided as background should not be assumed to have been previously recognized in the prior art. The subject matter in this section merely represents different approaches, which in and of themselves can also correspond to implementations of the claimed technology.

Genomics, in the broad sense, also referred to as functional genomics, aims to characterize the function of every genomic element of an organism by using genome-scale assays such as genome sequencing, transcriptome profiling, and proteomics. Genomics arose as a data-driven science - it operates by discovering novel properties from explorations of genome-scale data rather than by testing preconceived models and hypotheses. Applications of genomics include finding associations between genotype and phenotype, discovering biomarkers for patient stratification, predicting the function of genes, and charting biochemically active genomic regions and residues such as transcriptional enhancers and single nucleotide polymorphisms (SNPs).

Genomics data are too large and too complex to be mined solely by visual investigation of pairwise correlations. Instead, analytical tools are required to support the discovery of unanticipated relationships, to derive novel hypotheses and models, and to make predictions. Unlike some algorithms, in which assumptions and domain expertise are hard coded, machine learning algorithms are designed to automatically detect patterns in data. Hence, machine learning algorithms are suited to data-driven sciences and, in particular, to genomics. However, the performance of machine learning algorithms can strongly depend on how the data are represented, that is, on how each variable (also called a feature) is computed. For instance, to classify a tumor as malign or benign from a fluorescent microscopy image, a preprocessing algorithm could detect cells, identify the cell type, and generate a list of cell counts for each cell type.

A machine learning model can take the estimated cell counts, which are examples of handcrafted features, as input features to classify the tumor. A central issue is that classification performance depends heavily on the quality and the relevance of these features. For example, relevant visual features such as cell morphology, distances between cells, or localization within an organ are not captured in cell counts, and this incomplete representation of the data may reduce classification accuracy.

Deep learning, a subdiscipline of machine learning, addresses this issue by embedding the computation of features into the machine learning model itself to yield end-to-end models. This outcome has been realized through the development of deep neural networks, machine learning models that comprise successive elementary operations, which compute increasingly more complex features by taking the results of preceding operations as input. Deep neural networks are able to improve prediction accuracy by discovering relevant features of high complexity, such as the cell morphology and spatial organization of cells in the above example. The construction and training of deep neural networks have been enabled by the explosion of data, algorithmic advances, and substantial increases in computational capacity, particularly through the use of graphical processing units (GPUs).

The goal of supervised learning is to obtain a model that takes features as input and returns a prediction for a so-called target variable. An example of a supervised learning problem is one that predicts whether an intron is spliced out or not (the target) given features on the RNA such as the presence or absence of the canonical splice site sequence, and the location of the splicing branchpoint or intron length. Training a machine learning model refers to learning its parameters, which commonly involves minimizing a loss function on training data with the aim of making accurate predictions on unseen data.

For many supervised learning problems in computational biology, the input data can be represented as a table with multiple columns, or features, each of which contains numerical or categorical data that are potentially useful for making predictions. Some input data are naturally represented as features in a table (such as temperature or time), whereas other input data need to be first transformed (such as deoxyribonucleic acid (DNA) sequence into k-mer counts) using a process called feature extraction to fit a tabular representation. For the intron-splicing prediction problem, the presence or absence of the canonical splice site sequence, the location of the splicing branchpoint and the intron length can be preprocessed features collected in a tabular format. Tabular data are standard for a wide range of supervised machine learning models, ranging from simple linear models, such as logistic regression, to more flexible nonlinear models, such as neural networks, and many others.

Logistic regression is a binary classifier, that is, a supervised learning model that predicts a binary target variable. Specifically, logistic regression predicts the probability of the positive class by computing a weighted sum of the input features mapped to the [0,1] interval using the sigmoid function, a type of activation function. The parameters of logistic regression, or other linear classifiers that use different activation functions, are the weights in the weighted sum. Linear classifiers fail when the classes, for instance, that of an intron spliced out or not, cannot be well discriminated with a weighted sum of input features. To improve predictive performance, new input features can be manually added by transforming or combining existing features in new ways, for example, by taking powers or pairwise products.

Neural networks use hidden layers to learn these nonlinear feature transformations automatically. Each hidden layer can be thought of as multiple linear models with their output transformed by a nonlinear activation function, such as the sigmoid function or the more popular rectified-linear unit (ReLU). Together, these layers compose the input features into relevant complex patterns, which facilitates the task of distinguishing two classes.

Deep neural networks use many hidden layers, and a layer is said to be fully-connected when each neuron receives inputs from all neurons of the preceding layer. Neural networks are commonly trained using stochastic gradient descent, an algorithm suited to training models on very large data sets. Implementation of neural networks using modern deep learning frameworks enables rapid prototyping with different architectures and data sets. Fully-connected neural networks can be used for a number of genomics applications, which include predicting the percentage of exons spliced in for a given sequence from sequence features such as the presence of binding motifs of splice factors or sequence conservation; prioritizing potential disease-causing genetic variants; and predicting cis-regulatory elements in a given genomic region using features such as chromatin marks, gene expression and evolutionary conservation.

Local dependencies in spatial and longitudinal data must be considered for effective predictions. For example, shuffling a DNA sequence or the pixels of an image severely disrupts informative patterns. These local dependencies set spatial or longitudinal data apart from tabular data, for which the ordering of the features is arbitrary. Consider the problem of classifying genomic regions as bound versus unbound by a particular transcription factor, in which bound regions are defined as high-confidence binding events in chromatin immunoprecipitation followed by sequencing (ChIP-seq) data. Transcription factors bind to DNA by recognizing sequence motifs. A fully-connected layer based on sequence-derived features, such as the number of k-mer instances or the position weight matrix (PWM) matches in the sequence, can be used for this task. As k-mer or PWM instance frequencies are robust to shifting motifs within the sequence, such models could generalize well to sequences with the same motifs located at different positions. However, they would fail to recognize patterns in which transcription factor binding depends on a combination of multiple motifs with well-defined spacing. Furthermore, the number of possible k-mers increases exponentially with k-mer length, which poses both storage and overfitting challenges.

A convolutional layer is a special form of fully-connected layer in which the same fully-connected layer is applied locally, for example, in a 6 bp window, to all sequence positions. This approach can also be viewed as scanning the sequence using multiple PWMs, for example, for transcription factors GATA1 and TAL1. By using the same model parameters across positions, the total number of parameters is drastically reduced, and the network is able to detect a motif at positions not seen during training. Each convolutional layer scans the sequence with several filters by producing a scalar value at every position, which quantifies the match between the filter and the sequence. As in fully-connected neural networks, a nonlinear activation function (commonly ReLU) is applied at each layer. Next, a pooling operation is applied, which aggregates the activations in contiguous bins across the positional axis, commonly taking the maximal or average activation for each channel. Pooling reduces the effective sequence length and coarsens the signal. The subsequent convolutional layer composes the output of the previous layer and is able to detect whether a GATA1 motif and TAL1 motif were present at some distance range. Finally, the output of the convolutional layers can be used as input to a fully-connected neural network to perform the final prediction task. Hence, different types of neural network layers (e.g., fully-connected layers and convolutional layers) can be combined within a single neural network.

Convolutional neural networks (CNNs) can predict various molecular phenotypes on the basis of DNA sequence alone. Applications include classifying transcription factor binding sites and predicting molecular phenotypes such as chromatin features, DNA contact maps, DNA methylation, gene expression, translation efficiency, RBP binding, and microRNA (miRNA) targets. In addition to predicting molecular phenotypes from the sequence, convolutional neural networks can be applied to more technical tasks traditionally addressed by handcrafted bioinformatics pipelines. For example, convolutional neural networks can predict the specificity of guide RNA, denoise ChIP-seq, enhance Hi-C data resolution, predict the laboratory of origin from DNA sequences and call genetic variants. Convolutional neural networks have also been employed to model long-range dependencies in the genome. Although interacting regulatory elements may be distantly located on the unfolded linear DNA sequence, these elements are often proximal in the actual 3D chromatin conformation. Hence, modelling molecular phenotypes from the linear DNA sequence, albeit a crude approximation of the chromatin, can be improved by allowing for long-range dependencies and allowing the model to implicitly learn aspects of the 3D organization, such as promoter-enhancer looping. This is achieved by using dilated convolutions, which have a receptive field of up to 32 kb. Dilated convolutions also allow splice sites to be predicted from sequence using a receptive field of 10 kb, thereby enabling the integration of genetic sequences across distances as long as typical human introns (*See* Jaganathan, K. et al. Predicting splicing from primary sequence with deep learning. Cell 176, 535-548 (2019)).

Different types of neural networks can be characterized by their parameter-sharing schemes. For example, fully-connected layers have no parameter sharing, whereas convolutional layers impose translational invariance by applying the same filters at every position of their input. Recurrent neural networks (RNNs) are an alternative to convolutional neural networks for processing sequential data, such as DNA sequences or time series, that implement a different parameter-sharing scheme. Recurrent neural networks apply the same operation to each sequence element. The operation takes as input the memory of the previous sequence element and the new input. It updates the memory and optionally emits an output, which is either passed on to subsequent layers or is directly used as model predictions. By applying the same model to each sequence element, recurrent neural networks are invariant to the position index in the processed sequence. For example, a recurrent neural network can detect an open reading frame in a DNA sequence regardless of the position in the sequence. This task requires the recognition of a certain series of inputs, such as the start codon followed by an in-frame stop codon.

The main advantage of recurrent neural networks over convolutional neural networks is that they are, in theory, able to carry over information through infinitely long sequences via memory. Furthermore, recurrent neural networks can naturally process sequences of widely varying length, such as mRNA sequences. However, convolutional neural networks combined with various tricks (such as dilated convolutions) can reach comparable or even better performances than recurrent neural networks on sequence-modelling tasks, such as audio synthesis and machine translation. Recurrent neural networks can aggregate the outputs of convolutional neural networks for predicting single-cell DNA methylation states, RBP binding, transcription factor binding, and DNA accessibility. Moreover, because recurrent neural networks apply a sequential operation, they cannot be easily parallelized and are hence much slower to compute than convolutional neural networks.

Each human has a unique genetic code, though a large portion of the human genetic code is common for all humans. In some cases, a human genetic code may include an outlier, called a genetic variant, that may be common among individuals of a relatively small group of the human population. For example, a particular human protein may comprise a specific sequence of amino acids, whereas a variant of that protein may differ by one amino acid in the otherwise same specific sequence.

Genetic variants may be pathogenetic, leading to diseases. Though most of such genetic variants have been depleted from genomes by natural selection, an ability to identify which genetic variants are likely to be pathogenic can help researchers focus on these genetic variants to gain an understanding of the corresponding diseases and their diagnostics, treatments, or cures. The clinical interpretation of millions of human genetic variants remains unclear. Some of the most frequent pathogenic variants are single nucleotide missense mutations that change the amino acid of a protein. However, not all missense mutations are pathogenic.

Models that can predict molecular phenotypes directly from biological sequences can be used as in silico perturbation tools to probe the associations between genetic variation and phenotypic variation and have emerged as new methods for quantitative trait loci identification and variant prioritization. These approaches are of major importance given that the majority of variants identified by genome-wide association studies of complex phenotypes are non-coding, which makes it challenging to estimate their effects and contribution to phenotypes. Moreover, linkage disequilibrium results in blocks of variants being co-inherited, which creates difficulties in pinpointing individual causal variants. Thus, sequence-based deep learning models that can be used as interrogation tools for assessing the impact of such variants offer a promising approach to finding potential drivers of complex phenotypes. One example includes predicting the effect of non-coding single-nucleotide variants and short insertions or deletions (indels) indirectly from the difference between two variants in terms of transcription factor binding, chromatin accessibility, or gene expression predictions. Another example includes predicting novel splice site creation from sequence or quantitative effects of genetic variants on splicing.

End-to-end deep learning approaches for variant effect predictions are applied to predict the pathogenicity of missense variants from protein sequence and sequence conservation data (*See* Sundaram, L. et al. Predicting the clinical impact of human mutation with deep neural networks. Nat. Genet. 50, 1161-1170 (2018), referred to herein as "PrimateAI"). PrimateAI uses deep neural networks trained on variants of known pathogenicity with data augmentation using cross-species information. In particular, PrimateAI uses sequences of wild-type and mutant proteins to compare the difference and decide the pathogenicity of mutations using the trained deep neural networks. Such an approach that utilizes the protein sequences for pathogenicity prediction is promising because it can avoid the circularity problem and overfitting to previous knowledge. However, compared to the adequate number of data to train the deep neural networks effectively, the number of clinical data available in ClinVar is relatively small. To overcome this data scarcity, PrimateAI uses common human variants and variants from primates as benign data while simulated variants based on trinucleotide context were used as unlabeled data.

PrimateAI outperforms prior methods when trained directly upon sequence alignments. PrimateAI learns important protein domains, conserved amino acid positions, and sequence dependencies directly from the training data consisting of about 120,000 human samples. PrimateAI substantially exceeds the performance of other variant pathogenicity prediction tools in differentiating benign and pathogenic de-novo mutations in candidate developmental disorder genes, and in reproducing prior knowledge in ClinVar. These results suggest that PrimateAI is an important step forward for variant classification tools that may lessen the reliance of clinical reporting on prior knowledge.

Central to protein biology is the understanding of how structural elements give rise to observed function. The surfeit of protein structural data enables the development of computational methods to systematically derive rules governing structural-functional relationships. However, the performance of these methods depends critically on the choice of protein structural representation.

Protein sites are microenvironments within a protein structure, distinguished by their structural or functional role. A site can be defined by a three-dimensional (3D) location and a local neighborhood around this location in which the structure or function exists. Central to rational protein engineering is the understanding of how the structural arrangement of amino acids creates functional characteristics within protein sites. Determination of the structural and functional roles of individual amino acids within a protein provides information to help engineer and alter protein functions. Identifying functionally or structurally important amino acids allows focused engineering efforts such as site-directed mutagenesis for altering targeted protein functional properties. Alternatively, this knowledge can help avoid engineering designs that would abolish a desired function.

Since it has been established that structure is far more conserved than sequence, the increase in protein structural data provides an opportunity to systematically study the underlying pattern governing the structural-functional relationships using data-driven approaches. A fundamental aspect of any computational protein analysis is how protein structural information is represented. The performance of machine learning methods often depends more on the choice of data representation than the machine learning algorithm employed. Good representations efficiently capture the most critical information while poor representations create a noisy distribution with no underlying patterns.

The surfeit of protein structures and the recent success of deep learning algorithms provide an opportunity to develop tools for automatically extracting task-specific representations of protein structures.

Out of more than 70 million possible missense variants in the human genome, the vast majority are of unknown clinical significance, with only ~0.1% annotated in clinical variant databases. An opportunity arises to understand the role of rare penetrant variants in common diseases. Accurate distinction of deleterious variants from those with benign consequence may result for the benefit of both precision medicine and targeted drug development.

## BRIEF DESCRIPTION OF THE DRAWINGS

The patent or application file contains at least one drawing executed in color. Copies of this patent or patent application publication with color drawing(s) will be provided by the Office upon request and payment of the necessary fee.

The color drawings also may be available in PAIR via the Supplemental Content tab. In the drawings, like reference characters generally refer to like parts throughout the different views. Also, the drawings are not necessarily to scale, with an emphasis instead generally being placed upon illustrating the principles of the technology disclosed. In the following description, various implementations of the technology disclosed are described with reference to the following drawings, in which.

Figure 1A is a schematic diagram illustrating a method of determining a weighted rare variant PRS.

Figure 1B illustrates an example calculation of a rare variant polygenic risk score for a particular plurality of genes and a particular phenotype.

Figure 2 shows an example computer system that can be used to implement the technology disclosed.

Figure 3 illustrates an example of genetic variants.

Figure 4 is an example illustrating phenotypic effects in response to changes in genotype.

Figure 5 illustrates a plurality of phenotypes corresponding to a cardiovascular disease patient X.

Figure 6 graphically contrasts genome-wide association studies for common variants versus rare variants.

Figure 7 illustrates genetic association tests for a particular individual common variant or particular aggregated rare variants at gene-resolution, respectively.

Figure 8 is a schematic diagram of a method for optimizing rare variant burden testing.

Figure 9 is a flow diagram that illustrates a process of a system for determining pathogenicity of variants.

Figure 10 shows an example processing architecture of the pathogenicity classifier, in accordance with one implementation of the technology disclosed.

Figure 11 shows an example computer system that can be used to implement the technology disclosed.

Figure 12 illustrates one implementation of determining a final pathogenicity score.

Figure 13 is a flow diagram of a process for correcting for multiple testing within each gene.

Figure 14 is a schematic diagram for a method of correcting phenotypic values for covariates.

Figure 15 is a schematic diagram representing predicting phenotypic shift in response to usage of a plurality of drugs on a plurality of phenotypes.

Figure 16 is a schematic diagram representing the experimental set-up to obtain drug usage patterns and phenotypic data for a particular cohort.

Figure 17 illustrates a plurality of phenotypes corresponding to a cardiovascular disease patient X.

Figure 18 is a graph quantifying the total number of significant gene-phenotype pairs identified for different types of burden tests.

Figure 19 is a collection of graphs illustrating rare deleterious variants that affect disease severity and age of onset identified by the pathogenicity classifier PrimateAI-3D.

Figure 20 is a graph of the average absolute Spearman correlation of different pathogenicity scores with phenotype values.

Figure 21 is a heatmap comparison of rare deleterious variants and common genome-wide association study variants.

Figure 22 is a further comparison of rare deleterious variants and common genome-wide association study variants.

Figure 23 illustrates the cholesterol pathway and total cholesterol distribution across all individuals in the UK Biobank cohort.

Figure 24 comprises measures of rare variant PRS performance.

Figure 25 is a graph of the enrichment of PRS outliers.

Figure 26 is a graph of PRS outliers for quantitative phenotypes.

Figure 27 comprises graphs of normalized total cholesterol distributions from two separate cohorts.

Figure 28 comprises graphs comparing rare variant PRS outliers and phenotypes between two separate cohorts.

Figure 29 comprises graphs illustrating performance results by ethnicity.

Figure 30 is a table comparing the effect sizes and frequencies for common PRS variants and rare PRS genes used for normal cholesterol levels.

Figure 31 is a graph of the mean fraction of phenotypic variance explained by different pathogenicity scoring methods on a set of 34 gene-phenotype pairs, which were selected based on their enrichment for rare missense and LoF variants.

Figure 32 is a heatmap of the enrichment of rare variants in GWAS genes for all pairwise comparisons between quantitative and clinical phenotypes.

Figure 33 comprises graphs of the distribution of the absolute values of the ratios between the average effect size of singleton LoF variants and the effect size of the most significant GWAS variant for the same gene.

Figure 34 shows the number of variants per individual in any of the genes from the gene-phenotype pairs that were significant at 5% false discovery rate.

Figure 35 shows a comparison of effect sizes in training data versus testing data splits.

Figure 36 comprises graphs comparing common variant and rare variant PRS subsets.

## DETAILED DESCRIPTION

The following discussion is presented to enable any person skilled in the art to make and use the technology disclosed and is provided in the context of a particular application and its requirements. Various modifications to the disclosed implementations will be readily apparent to those skilled in the art, and the general principles defined herein may be applied to other implementations and applications without departing from the spirit and scope of the technology disclosed. Thus, the technology disclosed is not intended to be limited to the implementations shown but is to be accorded the widest scope consistent with the principles and features disclosed herein.

The detailed description of various implementations will be better understood when read in conjunction with the appended drawings. To the extent that the figures illustrate diagrams of the functional blocks of the various implementations, the functional blocks are not necessarily indicative of the division between hardware circuitry. Thus, for example, one or more of the functional blocks (*e.g.*, modules, processors, or memories) may be implemented in a single piece of hardware (*e.g.*, a general purpose signal processor or a block of random-access memory, hard disk, or the like) or multiple pieces of hardware. Similarly, the programs may be stand-alone programs, may be incorporated as subroutines in an operating system, may be functions in an installed software package, and the like. It should be understood that the various implementations are not limited to the arrangements and instrumentality shown in the drawings.

The processing engines and databases of the figures, designated as modules, can be implemented in hardware or software, and need not be divided up in precisely the same blocks as shown in the figures. Some of the modules can also be implemented on different processors, computers, or servers, or spread among a number of different processors, computers, or servers. In addition, it will be appreciated that some of the modules can be combined, operated in parallel or in a different sequence than that shown in the figures without affecting the functions achieved. The modules in the figures can also be thought of as flowchart steps in a method. A module also need not necessarily have all its code disposed contiguously in memory; some parts of the code can be separated from other parts of the code with code from other modules or other functions disposed in between.

The technologies disclosed can be used to improve the quality of polygenic risk scoring for rare variants. The technology disclosed can be used to identify patients at the extreme of a phenotypic spectrum who are at the greatest risk for severe, early-onset disease and receive the greatest benefit from clinical intervention. The researchers presenting these disclosures show that outlier phenotypes associated with severe, early-onset disease are better explained by rare penetrant variants than by the collective action of many common variants with small effect sizes. Unlike common variants, which have predominantly been depleted of deleterious consequence by natural selection over the course of many generations, rare variants are largely unfiltered, and preserve the potential to exert highly penetrant effects in complex traits and diseases. The technology disclosed comprises a novel weighted sum model for the quantification of contribution of rare, pathogenic variants to a phenotype response.

**Introduction**

The technology disclosed develops a complementary, rare variant polygenic risk score model, based on a weighted sum of rare deleterious variants from multiple phenotype-associated genes. Although individual genome-wide association study (GWAS) variants confer effects that tend to be too mild for clinical actionability, polygenic risk scores combining signal from hundreds to millions of common variants have demonstrated significant efficacy for predicting patients at phenotypic extremes who are at high risk for disease. However, existing common variant polygenic risk score models have largely excluded rare variants due to challenges in interpreting variants of unknown significance and imprecision in their effect size estimation. Compared to existing common variant polygenic risk score models, the disclosed technology comprises a rare variant polygenic risk score model configured to aggregate risk across genes based on whether individuals carry rare deleterious variants in each gene.

An individual's genotype contributes to the individual's phenotype (where the phenotype is defined as one or more observable physical properties of an individual); thus, there is a statistical correlation between genotype and phenotype that may be used to aid in the prediction of a physical trait, abnormality, or presence of disease in an individual with a particular variant or group of variants. While certain genetic disorders can be caused by a variant in a single gene (*i.e.,* a monogenic genetic disorder), genetic disorders caused by variants in multiple genes (*i.e.,* a polygenic genetic disorder) are more common. Thus, a robust method for determining polygenic risk scores for rare, deleterious variants likely to cause severe disease is needed.

Recent large-scale genome and exome sequencing studies of healthy individuals from the general population have revealed that the average person carries dozens of potentially deleterious rare variants that have arisen through recent mutations. Unlike common variants, which have predominantly been depleted of deleterious consequence by natural selection over the course of many generations, rare variants are largely unfiltered, and preserve the potential to exert highly penetrant effects in complex traits and diseases. The public release of 200,643 UK Biobank exomes, together with rapid advances in the accuracy of variant pathogenicity prediction, creates the opportunity to examine the impact of rare penetrant variants on a comprehensive set of common human diseases and complex traits, as well as offering a glimpse into the potential utility of personal genome sequencing for the general population.

Recent exponential human population growth has created an abundance of rare variants via randomly occurring mutations, without providing adequate time for natural selection to deplete those with deleterious consequences, in contrast with common variants identified in GWAS. We set out to test that at each GWAS locus containing common variants associated with mild clinical risk, rare deleterious variants with far greater severity should also exist, forming an allelic series where a variant's severity is inversely related to its frequency in the population.

Despite the high penetrance of rare deleterious variants, their rarity limits their ability to explain phenotypic variance to a small fraction of the population, since the majority of individuals do not carry a rare deleterious variant for a given phenotype. Hence, one implementation of the rare variant PRS described in the technology disclosed performs only about one-twentieth as well as the common variant PRS in terms of variance explained across the entire population. However, when considering individuals at phenotypic extremes this trend was reversed: individuals with an outlier phenotype (z-score $\geq$3) were 3-fold more likely than the baseline population to have a rare variant PRS score in the 1st or 99th percentile, compared to 1.8-fold for common variant PRS.

A significant barrier to the clinical adoption of common variant PRS models has been their limited generalizability between populations with different ancestry. These issues stem from the incorporation of variants that are disease-associated, but non-causal, as predictors in common variant PRS models due to linkage disequilibrium. While the effects of causal variants may be expected to generalize between cohorts, there is no guarantee that the correlation between the true causal variant and the variant used in the model will be preserved; rather, these correlations are affected by differences in population ancestry as well as technical artifacts in genotyping and imputation. In comparison, the rare variant PRS model directly uses rare deleterious variants as the predictors, which given their rarity and recent history in the population, are largely unaffected by the linkage disequilibrium issues that make it difficult to distinguish causation from correlation for common variants. Rather, the challenge for predicting rare variant polygenic risk lies in the accurate interpretation of the effects of variants of uncertain significance (VUS), which is crucial both for identifying genes with significant rare variant associations, as well as estimating the effect size of a rare deleterious variant for a given clinical phenotype. Because nearly half of the rare deleterious variants that appear in rare variant PRS genes are seen in only one individual in the entire 200,643 UK Biobank exome cohort, this problem may seem intractable for traditional statistical analysis, but recent advances employing deep learning, high-throughput experimental assays, and variant information from closely related primate species have each demonstrated progress towards solving the VUS problem on a genome-wide scale. Although the exceptional allelic heterogeneity underlying rare variant PRS genes can be a challenge from the standpoint of variant effect prediction, paradoxically, it is also the key to the robustness and portability of the rare variant PRS model across different cohorts: by integrating signal across thousands of unique rare deleterious variants per gene, the rare variant PRS attains the desirable property of smoothing out the effects of any variant-specific artifacts that may be present in the data.

The extent to which rare and common genetic variants contribute risk for common diseases and complex traits has been a decades-long standing debate in the field of human genetics. The technology disclosed helps to reconcile these viewpoints, by demonstrating the presence of rare penetrant variants at a large fraction of GWAS loci, and quantifying the relative contributions of rare and common variants to total population variance as well as to outlier individuals who are at the greatest risk for severe, early-onset disease.

From the perspective of precision medicine, the UK Biobank cohort, being a representative cross-section of adults from the UK population at large, also presents a unique opportunity to characterize the burden of rare penetrant variants and their effects in the general population. Across the 500 genes that were significant for one or more of the 90 clinical and quantitative phenotypes studied in one implementation of the technology disclosed, 86% of individuals carried at least one rare deleterious variant, with an average of 2.03 rare deleterious variants per individual. Some implementations of the technology disclosed show that these rare deleterious variants have highly penetrant effects, contributing effect sizes that were on average 10-fold greater than common GWAS variants present at the same loci. On average, 5.2% of individuals carried a rare deleterious variant for a given phenotype, and 0.4% of individuals carried a rare penetrant variant for a given gene.

The technology disclosed augments the rare variant burden testing approach to maximize power and filter out benign missense variables. In some implementations of the technology disclosed, burden test power is maximized by performing a first grid search over the allele counts of the observed variants in order to find the cutoff for maximum allele count that maximizes the significance of the burden test and benign missense variants are filtered out by performing a second grid search over a plurality of cutoffs for pathogenicity score (*e.g.,* PrimateAI-3D scores) thresholds to find the optimal threshold for pathogenicity score that maximizes the significance of the burden test.

The technology disclosed develops a rare deleterious variant polygenic risk score model based on a weighted sum of rare deleterious variants from multiple phenotype-associated genes. Individual genome-wide association study variants tend to confer effects that are too mild for clinical actionability and genetic disorders caused by variants in multiple genes as compared to monogenic disorders. Therefore, polygenic risk score models that combine signal from a plurality of variants are often more efficacious for predicting patients who are at a high risk of disease. However, existing polygenic risk score models are predominantly optimized for common variants and exclude rare variants due to difficulty interpreting variants of unknown significance, challenges with low-powered studies, and imprecise effect size estimation due to small sample size of rare variants. The technology disclosed improves upon prior polygenic risk score models for the inclusion of rare variants by aggregating risk on a gene-resolution basis based on whether individuals carry at least one rare deleterious variant within a particular gene.

The disclosed rare polygenic risk scores are computed as a burden test in which a strength of association is quantified of genes associated with a phenotype and a contribution of rare variants to a phenotype response. Data is obtained representing variant carrier status and specified measured phenotype values for a cohort of individuals. A burden score is calculated for each of the associated genes using the data obtained, wherein the burden score identifies consequential, non-random association in the cohort between carrier status of each of the associated genes and a phenotype response to presence in the associated genes of one or more rare pathogenic variants. Carrier status is a Boolean variable determined by presence or lack of presence of one or more rare pathogenic variants in a particular gene. Pathogenicity of particular rare variants is determined by predicted impact of the particular rare variant on function when the gene is expressed as a protein, wherein the predicted impact is determined as a pathogenicity score by a convolutional neural network pathogenicity classifier. Rareness of the particular rare variants is determined by occurrence below a predetermined threshold of the particular rare variant in a population (*i.e.,* maximum allele count).

Strength of association is quantified as an effective strength score respective to carrier status of rare pathogenic variants for a respective gene and a respective phenotype response to carrier status of rare pathogenic variants for the respective gene at per-gene resolution. Scores for contribution of the carrier status of subjects in the cohort across the associated genes to the phenotype response can be determined based on the effective strength scores. In some implementations of the technology disclosed, the specified phenotype is a quantitative biomarker phenotype and the effective strength scores for the consequential, non-random association for genes are determined for the quantitative biomarker phenotype using a two-tailed t-test on a linear regression component of the burden score. The two-tailed t-test produces p-values to determine whether or not the difference between average phenotype measurements of carriers and non-carriers is significant at a predetermined significance level.

In other implementations of the technology disclosed, the specified phenotype is a categorical clinical diagnosis phenotype and the effective strength scores for the consequential, non-random association for genes are determined for the categorical clinical diagnosis phenotype as a beta coefficient for the carrier status. The beta coefficient is determined using a logistic regression component of the burden score wherein the logistic regression component is fitted to predict a clinical diagnosis label from a binary indicator variable corresponding to carrier status and a plurality of covariates.

In some implementations of the technology disclosed, each burden test respective to a particular gene and a particular phenotype is both optimized for the particular gene and corrected for the particular phenotype value. Some implementations of the technology disclosed may involve either optimization for the particular gene or correction for the particular phenotype value, but not the other.

Further implementations of the technology disclosed optimize the rare variant burden test by using nested t-tests that maximize separation between carriers and noncarriers, burden test parameters are optimized with respect to a particular gene. Burden test power is maximized by performing a first grid search over the allele counts of the observed variants in order to find the cutoff for maximum allele count that maximizes the significance of the burden test and benign missense variants are filtered out by performing a second grid search over a plurality of cutoffs for pathogenicity score (e.g., PrimateAI-3D scores) thresholds to find the optimal threshold for pathogenicity score that maximizes the significance of the burden test.

In some implementations of the technology disclosed, the grid search procedure involves searching a plurality of allele counts and a plurality of pathogenicity score thresholds wherein the grid search comprises the generation of a plurality of combinations of allele counts and pathogenicity score thresholds from the plurality of allele counts and the plurality of pathogenicity score thresholds, identification of a plurality of groups of rare pathogenic variants corresponding to the plurality of combinations of allele counts and pathogenicity score thresholds, burden testing of plurality of groups of rare pathogenic variants in dependence upon a carrier status that separates carriers of a particular group of rare pathogenic variants in a cohort of individuals from non-carriers of the particular group of rare pathogenic variants in the cohort of individuals, and determination of a plurality of effect sizes and p-values corresponding to the plurality of combinations of allele counts and pathogenicity score thresholds.

A particular combination of an allele count and a pathogenicity score threshold with the most significant p-value is selected to be used as the optimal combination for a specific gene. Respective genes may or may not have similar maximum allele count thresholds and/or minimum pathogenicity score thresholds that result in the optimum separation between carriers and noncarriers of each respective gene. Pathogenicity score thresholds correspond to pathogenicity score quantiles of pathogenicity scores determined for rare pathogenic variants in the groups of rare pathogenic variants. In some implementations of the technology disclosed, the pathogenicity scores are generated by a convolutional neural network pathogenicity classifier such as PrimateAI-3D. In other implementations of the technology disclosed, a wide range of additional AI, machine learning, and deep learning models are employed to generate a pathogenicity score.

The grid search procedure described above requires correction for multiple-testing on generated false discovery rate-corrected p-values. In some implementations of the technology disclosed, multiple testing correction is performed using a Benjamini-Hochberg procedure. In other implementations of the technology disclosed, multiple testing correction is performed using an adaptive permutation test procedure.

Further implementations of the technology disclosed apply covariate correction to drug use across temporally distributed detection points. The burden test input data is corrected with respect to measured phenotype values. Phenotype response values are covariate-corrected for a plurality of covariates and drug-usage corrected for a plurality of drugs. In some implementations of the technology disclosed, a phenotypic shift in response to a plurality of drugs on a plurality of phenotypes of a cohort of individuals with a plurality of confounders is predicted to generate covariate-corrected and drug-usage corrected phenotype measurements.

In some implementations of the technology disclosed, the phenotypic shift is predicted in response to usage of a plurality of drugs on a plurality of phenotypes of a cohort of individuals with a plurality of confounders (e.g., age, sex, genetic principal components, diet, and smoking status) on a per-phenotype basis for a cohort of individuals and phenotype measurements for the plurality of phenotypes, covariate measurements for the plurality of confounders, and drug usage patterns for the plurality of drugs for each individual within the cohort at two separate time points.

Phenotype measurements are covariate-corrected for the first and second time points based on the covariate measurements by regressing out the covariate measurements by fitting a first regression model, thereby generating covariate-corrected phenotype measurements for the first and second time points. A delta is determined based on a difference between the covariate-corrected phenotype measurements for the first and second time points. For each of the drug usage patterns, a second regression model is fit that uses the delta to predict a phenotypic shift in response to usage of the plurality of drugs on the covariate-corrected phenotype measurements.

The second regression model is a forward selection stepwise regression model that repeatedly predicts the delta by successively and cumulatively including the phenotypic shift for each of the drug usage patterns. The second regression model has a binary indicator independent variable for each of the drug usage patterns, wherein the drug usage patterns include not taking a drug at the first and second time points, start taking the drug between first and second time points, stop taking the drug between the first and second time points, and taking the drug at the first and second time points. In some implementations of the technology disclosed, the covariate correction, the delta determination, the phenotypic shift prediction, and the drug usage correction are executed on a per-drug basis for drugs in the plurality of drugs. In other implementations of the technology disclosed, the covariate correction, the delta determination, the phenotypic shift prediction, and the drug usage correction are executed on a per-drug category basis (e.g., statins, NSAIDs, opioids, and so on) for drug categories in the plurality of drug categories.

The second regression model further models a phenotypic shift in response to time passed between the first and second time points on a per-individual basis for individuals in the cohort of individuals, and a phenotypic shift in response to regression to a mean between the first and second time points. The second regression model is jointly fitted for a set of relevant drugs in the plurality of drugs.

In some implementations of the technology disclosed, drug usage correction is implemented by fitting a third regression model, including drug usage-correcting the phenotype measurement for the first time point based on a first binary indicator independent variable for a first drug usage pattern of start taking the drug between first and second time points, a second binary indicator independent variable for a second drug usage pattern of not taking a drug at the first and second time points, and a drug-specific binary indicator independent variable that encodes whether an individual was taking a particular drug at the first time point.

A fourth regression model is fitted, which includes drug usage-correcting the phenotype measurement for the second time point based on a third binary indicator independent variable for a third drug usage pattern of stop taking the drug between the first and second time points, a fourth binary indicator independent variable for a fourth drug usage pattern of taking the drug at the first and second time points, and a drug-specific binary indicator independent variable that encodes whether an individual was taking a particular drug at the second time point.

Rank-based inverse normal transformation may be applied to the drug usage-corrected phenotype measurements for the first and second time points, and generating normalized-drug usage-corrected phenotype measurements for the first and second time points. The normalized-drug usage-corrected phenotype measurements are then covariate-corrected for the first and second time points, and generating covariate-corrected-normalized-drug usage-corrected phenotype measurements for the first and second time points. The covariate-corrected-normalized-drug usage-corrected phenotype measurements may be used to generate rare variant polygenic risk scores wherein measurements for the phenotype of interest are corrected for phenotypic shift in response to covariates and drug usage patterns.

In some implementations of the technology disclosed, the constructed regression models may be used to detect drug-phenotype associations such as unwanted side effects and wanted target effects.

## Rare Variant Polygenic Risk Scores (PRS)

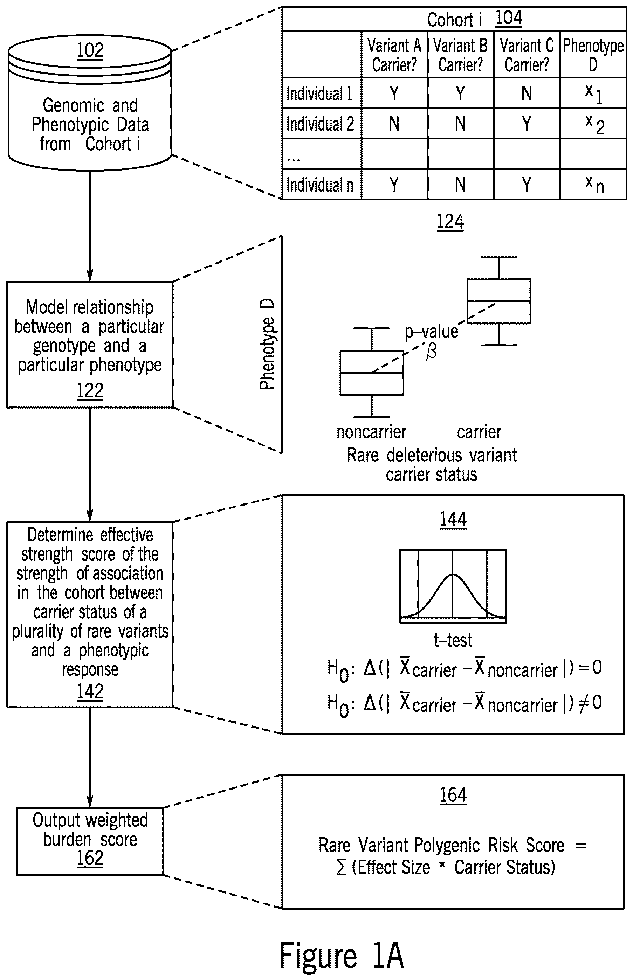

Figure 1A is a schematic diagram illustrating a method of determining a weighted rare variant PRS. A database 102 comprises genomic and phenotypic data corresponding to a group of individuals belonging to cohort *i*. Cohort *i* 104 comprises $N$ individuals who have undergone genetic sequencing and testing for a plurality of phenotypic measurements. Sequencing data for individuals within cohort i 104 comprises data corresponding to a plurality of variant carrier statuses. A particular gene may possess three known possible rare deleterious variants (*e.g.,* variant A, variant B, and variant C) of which an individual may be a carrier (*i.e.,* the individual possesses a respective variant, therefore they are a carrier of that variant) or a noncarrier (*i.e.,* the individual does not possess the respective variant, therefore they are not a carrier of that variant). A person skilled in the art will recognize that variant A, variant B, and variant C are given as an illustrative example and a gene may possess any number of rare deleterious variants. The technology disclosed defines a variant as 'rare' if the variant occurrence rate within a population is below a predetermined threshold and 'deleterious' if the pathogenicity of the variant is predicted to have a measured impact on impact when the gene is expressed as a protein above a predetermined threshold. The respective predetermined thresholds for both rarity and pathogenicity are gene-specific (*i.e.,* each individual gene will correspond to an individually determined rarity threshold and pathogenicity threshold that may differ from another gene).

In addition to genomic data obtained from genome and exome sequencing for each individual within cohort *i* 104, phenotypic data is also available for each individual (*i.e.,* individual $N$ has a measured value of $x_n$ for phenotype D). By constructing a model 122 for the relationship between a particular genotype and a particular phenotype, it is possible to measure the effect size of the particular genotype on the particular phenotype. Box-and-whisker plot 124 illustrates a representative genetic association test in which the effect of carrier status for one or more particular rare deleterious variants on phenotype D is measured in the form of both *p*-value (*i.e.,* the degree of significance when comparing the average phenotype value between carriers and noncarriers) and $\beta$ coefficient (*i.e.,* the weight estimate resulting from a regression line connecting the average phenotype value of carriers and the average phenotype value of noncarriers where the underlying data have been standardized so that the variance of the dependent and independent variables are equal to one).

In some implementations of the technology, wherein the particular phenotype is a quantitative biomarker phenotype, the strength of association is quantified as the *p*-value as determined by a two-tailed *t*-test. In other implementations of the technology disclosed, wherein the particular phenotype is a qualitative phenotype (*e.g.*, a categorical clinical diagnosis phenotype), the strength of association is quantified as a $\beta$ coefficient of a logistic regression.

In the technology disclosed, carrier status is defined on a gene-resolution basis rather than a variant-resolution basis. For a particular gene, an individual is defined as a carrier if the individual possesses at least one rare deleterious variant within the particular gene (*i.e.,* an individual who is a carrier for two rare deleterious variants is not differentiated from an individual who is a carrier for one rare deleterious variant, whereas an individual who is a carrier for zero rare deleterious variants is differentiated as a noncarrier). A determination 142 can be made for the effective strength score of the strength of association in cohort *i* 104 between the carrier status of a plurality of rare variants and the phenotypic response.

Graph 144 comprises a *t*-test for the determination of the effective strength score of the strength of association in cohort *i* 104 between the carrier status of a plurality of rare variants and the phenotypic response. The null hypothesis for the *t*-test states that the absolute value of the difference between the sample mean of carriers within cohort *i* and the sample mean of noncarriers within cohort *i* 104 is equal to zero. The alternative hypothesis for the *t*-test states that the absolute value of the difference between the sample mean of carriers within cohort *i* 104 and the sample mean of noncarriers within cohort *i* 104 is not equal to zero. The decision to accept or reject the null hypothesis is driven by a particular significance level $\alpha$ and the resulting p-value respective to $\alpha$. A respective *t*-test can be performed for a plurality of respective genes to obtain a plurality of gene-specific effective strength scores (measured by *p*-value or $\beta$ coefficient, as represented in chart 124) for a particular shared phenotypic response.

A weighted burden score 162 can be generated from the plurality of respective gene-specific effective strength scores. This calculation, described as a rare variant PRS, is shown in equation 164 wherein a rare variant PRS is equivalent to the summation of the product of effect size and carrier status for a particular gene for a plurality of genes.

The discussion now provides an example of using a plurality of respective gene-specific effective strength scores to generate a rare variant PRS for a particular individual, a particular plurality of respective genes sequenced for the particular individual, and a particular phenotype of interest.

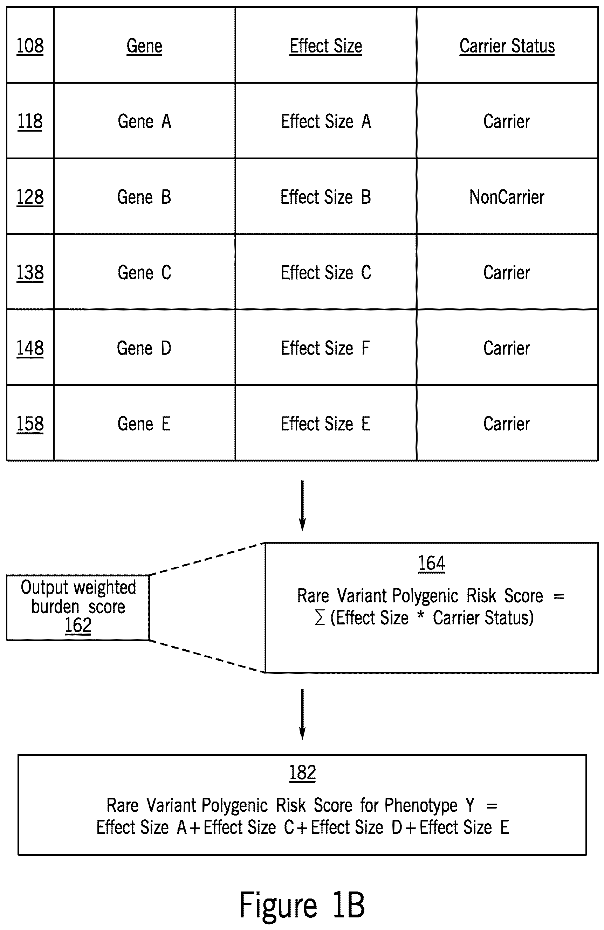

Figure 1B illustrates an example calculation of a rare variant polygenic risk score for a particular plurality of genes and a particular phenotype. Header row 108 comprises a group of column labels corresponding to gene, effect size, and carrier status for a particular individual. Genes included within the gene column are Gene A (row 118), Gene B (row 128), Gene C (row 138), Gene D (row 148), and Gene E (row 158), where Genes A-E can be assumed to be implicated genes for a particular Phenotype Y. Effect sizes for each gene, where effect size A is respective to Gene A (row 118), effect size B is respective to Gene B (row 128), effect size C is respective to Gene C (row 138), effect size D is respective to Gene D (row 148), and effect size E is respective to Gene E (row 158). Carrier status is a Boolean variable describing the presence of at least one identified rare deleterious variant within the particular gene (e.g., assume variants A, B, and C detected in cohort *i* 104 are particular to Gene A (row 118), thus, each individual listed in the table for cohort *i* 104 would be considered a carrier for Gene A). The individual whose genome is characterized in Figure 1B is a carrier for at least one rare deleterious variant in Gene A (row 118), Gene C (row 138), Gene D (row 148), and Gene E (row 158), respectively. The individual is not a carrier for any rare deleterious variants in Gene B (row 128).

To output a weighted burden score 162, equation 164 is applied. Equation 164 for the particular individual can be considered equivalent to equation 182, wherein the particular individual has a rare variant PRS for a particular Phenotype Y that is equivalent to the sum of the effect size A respective to Gene A (row 118), effect size C respective to Gene C (row 138), effect size D respective to Gene D (row 148), and effect size E respective to Gene E (row 158). For each respective gene that the particular individual is a carrier for at least one rare deleterious variant, carrier status is equal to one (*i.e.,* Effect Size * 1 = Effect Size). Effect size B respective to Gene B (row 128) is not shown in equation 182 as the particular individual is not a carrier for at least one rare deleterious variant; hence, carrier status is equal to zero (*i.e.,* Effect Size * 0 = 0) and the effect size B respective to Gene B (row 128) is not present in equation 182.

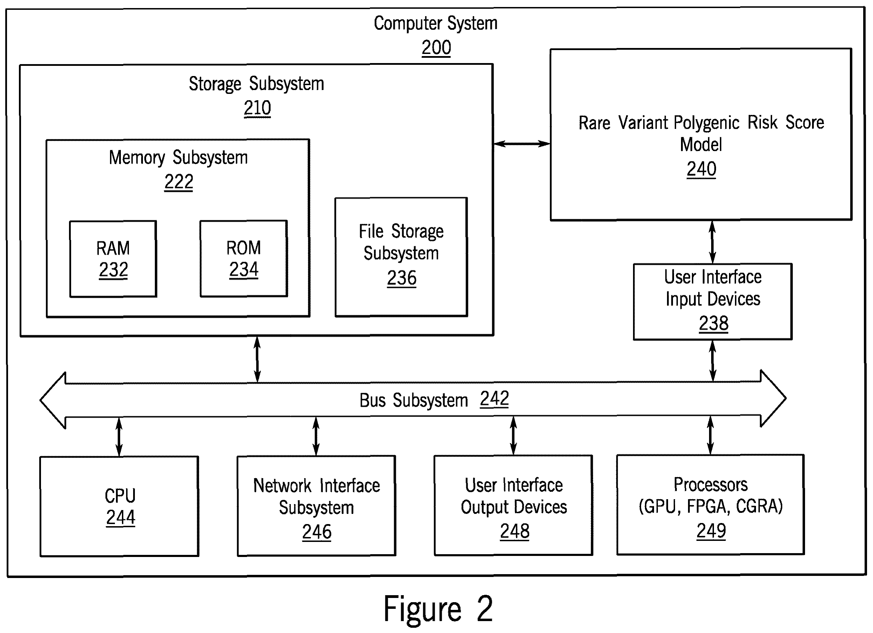

Figure 2 shows an example computer system 200 that can be used to implement the technology disclosed. Computer system 200 includes at least one central processing unit (CPU) 244 that communicates with a number of peripheral devices via bus subsystem 242. These peripheral devices can include a storage subsystem 210 including, for example, memory devices and a file storage subsystem 236, user interface input devices 238, user interface output devices 248, and a network interface subsystem 246. The input and output devices allow user interaction with computer system 200. Network interface subsystem 246 provides an interface to outside networks, including an interface to corresponding interface devices in other computer systems.

In one implementation, the rare variant PRS model 240 is communicably linked to the storage subsystem 210 and the user interface input devices 238.

User interface input devices 238 can include a keyboard; pointing devices such as a mouse, trackball, touchpad, or graphics tablet; a scanner; a touch screen incorporated into the display; audio input devices such as voice recognition systems and microphones; and other types of input devices. In general, use of the term "input device" is intended to include all possible types of devices and ways to input information into computer system 200.

User interface output devices 248 can include a display subsystem, a printer, a fax machine, or non-visual displays such as audio output devices. The display subsystem can include an LED display, a cathode ray tube (CRT), a flat-panel device such as a liquid crystal display (LCD), a projection device, or some other mechanism for creating a visible image. The display subsystem can also provide a non-visual display such as audio output devices. In general, use of the term "output device" is intended to include all possible types of devices and ways to output information from computer system 200 to the user or to another machine or computer system.

Storage subsystem 210 stores programming and data constructs that provide the functionality of some or all of the modules and methods described herein. These software modules are generally executed by processors 249.

Processors 249 can be graphics processing units (GPUs), field-programmable gate arrays (FPGAs), application-specific integrated circuits (ASICs), and/or coarse-grained reconfigurable architectures (CGRAs). Processors 249 can be hosted by a deep learning cloud platform such as Google Cloud Platform™, Xilinx™, and Cirrascale™. Examples of processors 249 include Google's Tensor Processing Unit (TPU)™, rackmount solutions like GX4 Rackmount Series™, GX2 Rackmount Series™, NVIDIA DGX-1™, Microsoft' Stratix V FPGA™, Graphcore's Intelligent Processor Unit (IPU)™, Qualcomm's Zeroth Platform™ with Snapdragon processors™, NVIDIA's Volta™, NVIDIA's DRIVE PX™, NVIDIA's JETSON TX1/TX2 MODULE™, Intel's Nirvana™, Movidius VPU™, Fujitsu DPI™, ARM's DynamicIQ™, IBM TrueNorth™, Lambda GPU Server with Testa V100s™, and others.

Memory subsystem 222 used in the storage subsystem 210 can include a number of memories including a main random-access memory (RAM) 232 for storage of instructions and data during program execution and a read only memory (ROM) 234 in which fixed instructions are stored. A file storage subsystem 236 can provide persistent storage for program and data files, and can include a hard disk drive, a floppy disk drive along with associated removable media, a CD-ROM drive, an optical drive, or removable media cartridges. The modules implementing the functionality of certain implementations can be stored by file storage subsystem 236 in the storage subsystem 210, or in other machines accessible by the processor.

Bus subsystem 242 provides a mechanism for letting the various components and subsystems of computer system 200 communicate with each other as intended. Although bus subsystem 242 is shown schematically as a single bus, alternative implementations of the bus subsystem can use multiple busses.

Computer system 200 itself can be of varying types including a personal computer, a portable computer, a workstation, a computer terminal, a network computer, a television, a mainframe, a server farm, a widely-distributed set of loosely networked computers, or any other data processing system or user device. Due to the ever-changing nature of computers and networks, the description of computer system 200 depicted in Figure 2 is intended only as a specific example for purposes of illustrating the preferred implementations of the present invention. Many other configurations of computer system 200 are possible having more or less components than the computer system depicted in Figure 2.

### Alternative Rare Deleterious Polygenic Risk Score Model

In one implementation, the technology disclosed can use a different rare deleterious polygenic risk score model than the one discussed above. Instead of using a weighted sum of rare deleterious variants, where the weight assigned to a variant in a gene was equal to the effect size observed across all rare deleterious variants in that gene, *i.e.,* the same for all variants in a gene, the technology disclosed can estimate per-variant weights within each significant gene. The per-variant weights can be estimated from individuals carrying the rare deleterious variants for a significant gene, via linear regression of variant pathogenicity score (*e.g.*, PrimateAI-3D) and log10-transformed allele frequency against individuals' trait values. This can improve performance by 18% in terms of variance explained, in some implementations.

### Genotype

The method of calculating a polygenic risk score described in Figure 1 involves data associated with an individual's genotype, determined by genotyping arrays (*i.e.,* whole-genome sequencing of a particular individual) and exome sequencing (*i.e.,* sequencing of the protein-coding regions of a particular individual's genome). The discussion now turns to details of genomic data, their associated features, and the relationship between genotype and phenotype for a particular individual.

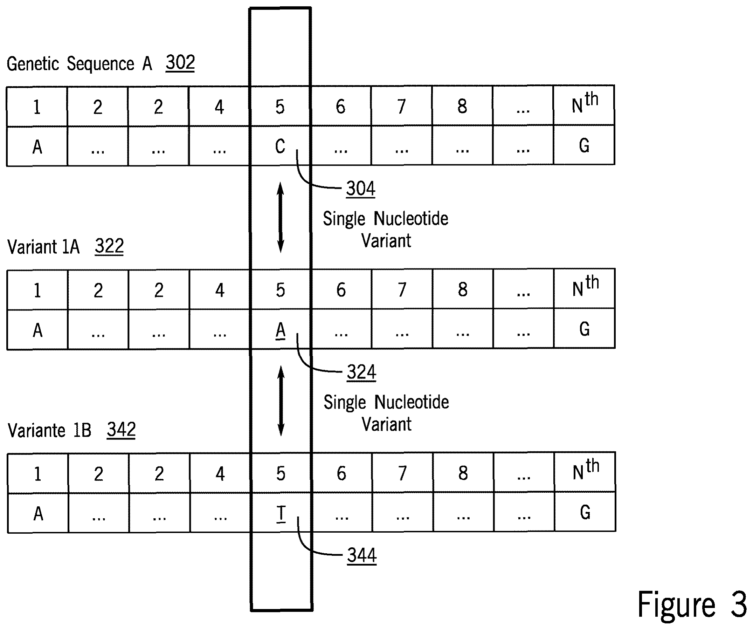

Figure 3 illustrates an example of genetic variants. An example reference genetic sequence A 302 possesses a length of *N* wherein each sequence position comprises a nucleotide base of either thymine, adenine, guanine, or cytosine. The reference genetic sequence A 302 can be considered the native, or normal, genetic sequence for a segment of a particular gene. The reference genetic sequence A 302 is compared with two example variant sequences 1A 322 and 1B 342 in which the variant sequences possess a respective single nucleotide variant in the fifth base position but otherwise possess an identical composition to the reference sequence. For example, a single nucleotide substitution is shown as adenine 324 in variant 1A 322 and thymine 344 in variant 1B 342 as compared to cytosine 304 in the reference genetic sequence A 302. When reference genetic sequence A 302, variant 1A 322, and variant 1B 342 are transcribed and translated into amino acid units of a protein, single nucleotide variants 324 and 344 may respectively result in at least one similar or different amino acid as compared to the amino acid(s) present in the native protein. If variant 1A 322 or variant 1B 342 respectively results in at least one changed amino acid in the translated protein, the respective variant may also result in a changed phenotype for an individual possessing the respective variant.

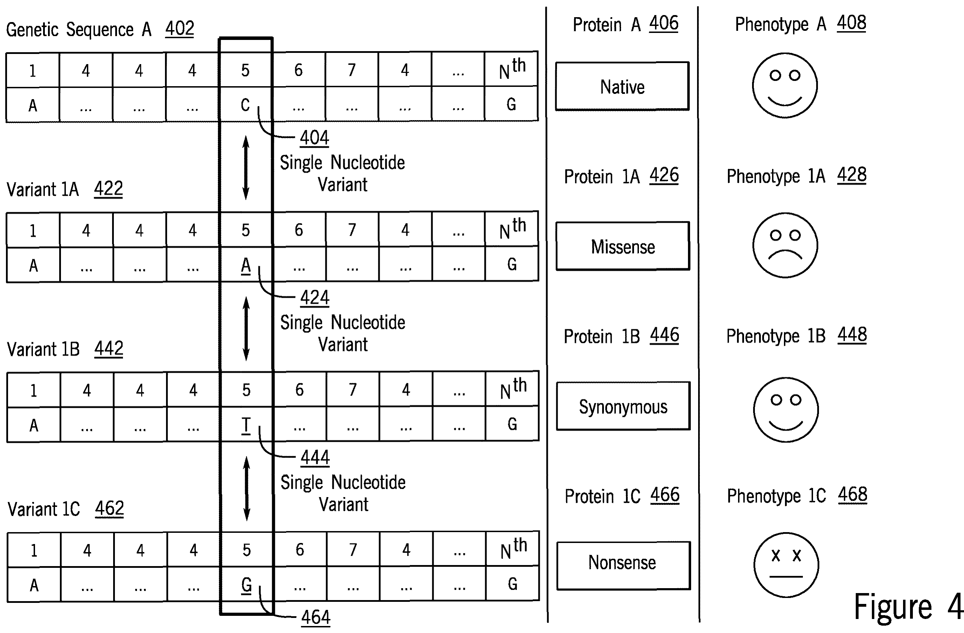

Figure 4 is an example illustrating phenotypic effects in response to changes in genotype. An example reference genetic sequence A 402 possesses a length of *N* wherein each sequence position comprises a nucleotide base of either thymine, adenine, guanine, or cytosine. The reference genetic sequence A 402 can be considered the native, or normal, genetic sequence for a segment of a particular gene. The reference genetic sequence A 402 is compared with three example variant sequences 1A 422, 1B 442, and 1C 462 in which the variant sequences possess a respective single nucleotide variant in the fifth base position but otherwise possess an identical composition to the reference sequence. For example, a single nucleotide substitution is shown as adenine 404 in variant 1A 422, thymine 444 in variant 1B 442, and guanine 464 in variant 1C 462 as compared to cytosine 404 in the reference genetic sequence A 402.

Reference genetic sequence A 402 results in protein A 406, the native protein composition for the particular gene comprising reference genetic sequence A 402. Individuals possessing native protein A 406 will present with phenotype A 408, a healthy phenotype. Variant 1A 422 results in mutant protein 1A 426, where mutant protein 1A 426 comprises a missense mutation resulting in a differing protein structure and function from the native protein A 406. Individuals possessing missense protein 1A 406 will present with phenotype 1A 428, a disease phenotype. Variant 1B 442 results in mutant protein 1B 446, where mutant protein 1B 446 comprises a synonymous mutation resulting in no change to protein structure and function as compared to the native protein A 406 (*i.e.,* no amino acid changes occurred in response to the single nucleotide variant in position five of variant 1B 442). Individuals possessing synonymous protein 1B 446 will present with phenotype 1B 448, a healthy phenotype similar to phenotype A 408. Variant 1C 462 results in mutant protein 1C 466, where mutant protein 1C 466 comprises a nonsense mutation resulting in a truncated, nonfunctional protein structure and function as compared to native protein A 406. Individuals possessing nonsense protein 1C 466 will present with phenotype 1C 468, likely resulting in a nonviable embryo or significantly reduced lifespan.

A person skilled in the art will recognize that variants 1A 422, 1B 442, and 1C 462 are listed as simplified examples and potential phenotypic representations span a wide spectrum rather than a limited number of discrete representations. Moreover, a person skilled in the art will also recognize that many phenotypic responses occur due a plurality of variants within a single gene, or a combined polygenic variant effect. Many implementations of the technology disclosed specifically address polygenic risk scoring for a particular phenotypic response associated with severe genetic disorders known to cause substantial detriment to an individual's quality of life and/or life expectancy.

## Phenotype

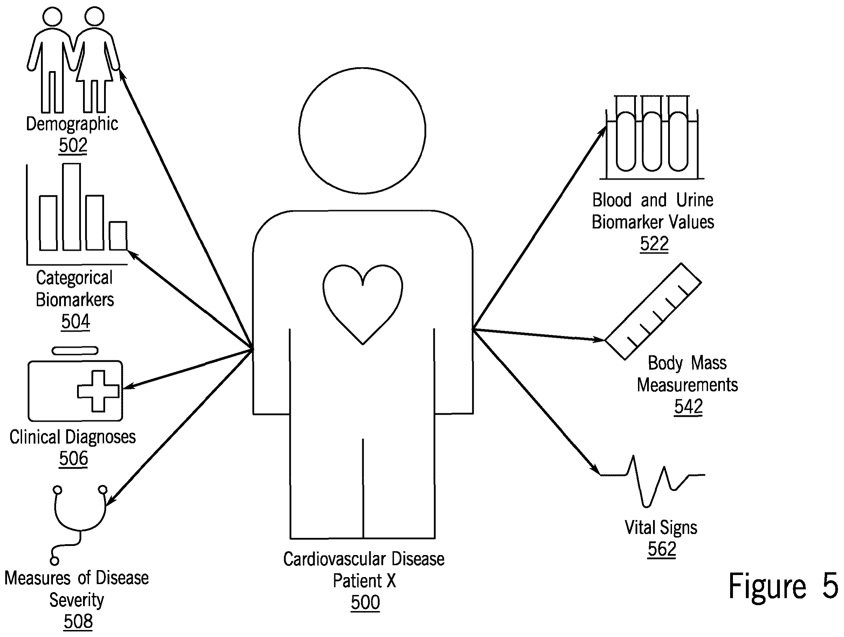

The discussion now turns to phenotypic presentations in further detail. While Figure 4 illustrates a plurality of phenotypic examples at a broad, organismal level, Figure 5 now illustrates a plurality of phenotypic examples at a targeted, measurable level capable of being described either as a quantitative biomarker value or a categorical clinical diagnosis.

Note that this Application uses "quantitative biomarker value" and "quantitative phenotype" interchangeably. Note that this Application also uses "categorical clinical diagnosis" and "qualitative phenotype" interchangeably.

Figure 5 illustrates a plurality of phenotypes corresponding to a cardiovascular disease patient X 500. Cardiovascular disease patient X 500 can be described by a plurality of observable phenotypes in response to a plurality of genetic variants within patient X's genome. These phenotypes may be qualitative or quantitative. Qualitative phenotypes may include demographic outputs 502 (*e.g.*, ancestry, biological sex, and so on), categorical biomarkers 504 (*e.g.,* a binary variable for presence of a particular protein in the blood, binary categorization of a patient's metabolite level in the blood as determined by a decision threshold value, or a multiclass variable for presence of a particular histological morphology), clinical diagnoses 506 (*e.g.,* a binary variable for cardiovascular disease diagnosis, a binary variable for a particular cancer diagnosis, and so on), or measures of disease severity (*e.g.*, progressive stages of tumor metastasis, classifications of disease subtype such as refractory celiac disease type 1 versus type 2, and so on).

Quantitative phenotypes may include exact blood or urine biomarker values 522 (*e.g.*, creatine, cholesterol, low-density lipoprotein (LDL), triglycerides, glucose, and so on), body mass measurements 542 (*e.g.,* height, weight, body fat percentage, body mass index, and so on), or vital signs (*e.g.*, resting heart rate, systolic and diastolic blood pressure, respiratory rate, and so on). A person skilled in the art will recognize that the quantitative and qualitative phenotypes listed for cardiovascular disease patient X 500 are non-limiting examples and there is an unlimited number of observable values that can be employed to describe individual health and composition.

## Gene-Phenotype Association Tests

The discussion so far covers the definitions and contexts for genomic and phenotypic data used to describe a particular individual. Figure 1 describes, at a high level, a method of determining genetic association for a particular rare deleterious variant carrier status at a gene-resolution level for a particular phenotype. The discussion now revisits this method in more detail via the introduction of genome-wide association studies and their implementation for both common and rare variants. In contrast to traditional genome-wide association studies for rare variants, the discussion turns to a novel methodology with improved utility wherein a plurality of rare variant burden testing for a plurality of particular genes comprising at least one particular rare deleterious variant is implemented to determine a rare variant polygenic risk score for the plurality of particular genes and a particular phenotype. The discussion focuses specifically on rare deleterious variants due to the statistical likelihood of rare deleterious variants to be implicated in severe, early-onset genetic disease.

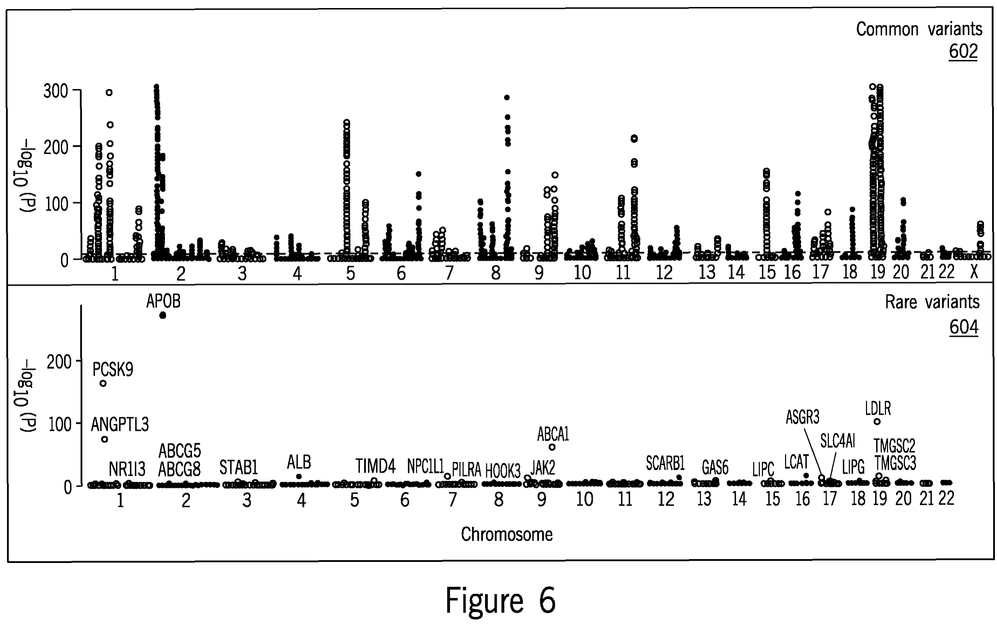

Figure 6 graphically contrasts genome-wide association studies for common variants versus rare variants. Graphs 602 and 604 describe genome-wide associations for cholesterol levels and common variants or rare variants, respectively. For both graph 602 and 604, alternating colors indicate successive chromosomes. Graph 602 shows individual common variants as data points whereas graph 604 shows individual genes (*i.e.,* all rare variants for a particular gene condensed into a single carrier status as shown in model 122). The common variant genome-wide association study shown in graph 602 finds many locations within the genome that are strongly associated with cholesterol levels. Due to the fact that common variants near each other are frequently correlated, the associations occur as peaks with many associated variants. As a result, detecting any causal variants within a particular associated locus can require considerable effort.

Graph 604 shows the rare variant genome-wide association study results for the 19,500 protein-coding genes in the human genome, where a small number of rare variants are strongly associated. Due to their rarity, the rare variant genome-wide association study is less well-powered compared to the common variant genome-wide association study shown in graph 602. Rare variants typically are not correlated with each other; therefore, the effects will not extend into nearby genes. Hence, significant genes appear isolated rather than in clusters within graph 602. Variants which alter protein sequence are specifically tested, meaning causal variants can be identified for the significant genes identified by the rare variant genome-wide association study.

Whereas Figure 6 illustrates a plurality of genetic associations on a genome-wide level, the discussion now turns to genetic association testing for a particular individual variant (*i.e.,* the consequential, non-random association of a particular individual common variant allele dosage and a phenotype response to presence in the associated gene of the particular individual common variant allele dosage value) or a particular aggregation of variants (*i.e.,* the consequential, non-random association in the cohort between carrier status of each of the associated genes and a phenotype response to presence in the associated genes of one or more rare pathogenic variants).

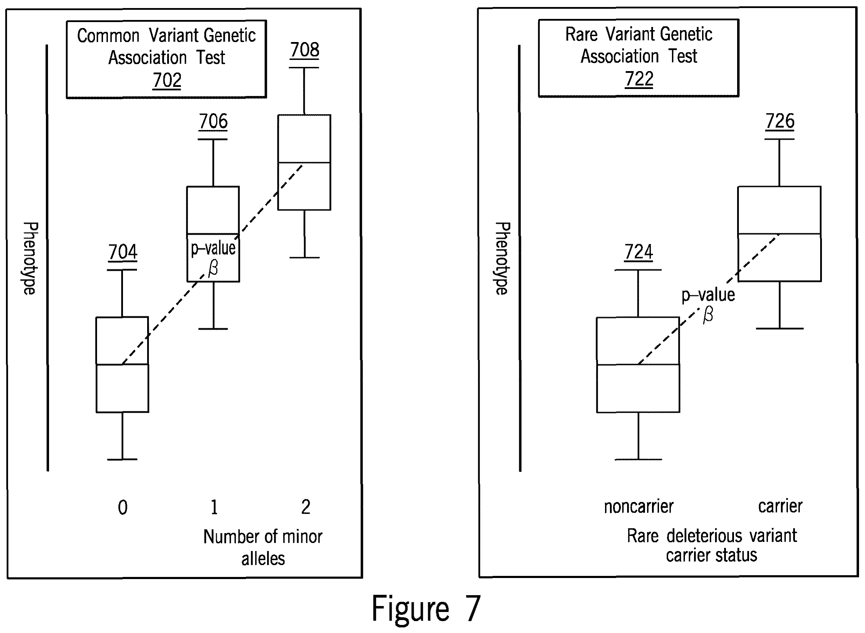

Figure 7 illustrates genetic association tests for a particular individual common variant or particular aggregated rare variants at gene-resolution, respectively. For the single common variant genetic association test 702 from genotyping arrays, each individual variant can be evaluated to ask the relationship between the number of minor alleles (wherein box plots 704, 706, and 708 are respective to phenotypic value distributions for zero, one, or two minor alleles, respectively) for the individual common variant and a phenotype of interest (e.g., cholesterol). A linear regression model can be built between the mean values for each minor allele count value to obtain a p-value and $\beta$ coefficient measuring the effective strength score of association for the particular variant and phenotype response.

In contrast, for rare variants obtained from exome sequencing, testing per individual variants is not useful as the occurrence rate for each rare variant is too low. To test genetic association for rare variants, a burden test 722 is performed on aggregated variants within a particular gene as demonstrated in model 122. In comparison to the single common variant genetic association test 702, the aggregated rare variant burden test 722 measures the strength of association between carrier status for at least one rare variant within a gene (wherein box plots 724 and 726 are respective to phenotypic value distributions for noncarriers and carriers, respectively) and the phenotype of interest. In addition to filtering for rarity, only deleterious variants are included as determined by a pathogenicity measure threshold value.

In some implementations of the technology disclosed, the phenotype response of interest is a categorical clinical diagnosis. Thus, the effective strength scores for the consequential, non-random association for genes are determined for the categorical clinical diagnosis phenotype as a beta coefficient for the carrier status, wherein the beta coefficient is determined using a logistic regression component of the burden score.

## Optimization of Rare Variant Burden Testing

The discussion now revisits the concept of an individually determined rarity threshold (*i.e.,* maximum allele count) and pathogenicity score threshold for a particular gene. Previous figures have described genetic association tests of a particular plurality of rare deleterious variants for a particular gene on a particular phenotype, wherein the maximum rarity threshold and minimum pathogenicity score threshold are specific to the particular gene and may differ from the maximum rarity threshold and minimum pathogenicity score threshold for a different particular gene.

Note that the Application uses the terms "rarity threshold", "maximum allele count", "allele count threshold", and "AC" interchangeably. Note that the Application also uses the terms "pathogenicity threshold", "pathogenicity score threshold"", "minimum pathogenicity score threshold", and "PST" interchangeably.

The discussion now turns to the technology disclosed for determining the particular combination of an allele count and a pathogenicity score threshold that has a most significant *p*-value and using the particular combination as the optimal combination. Each *p*-value for each combination of allele counts and pathogenicity score thresholds within the plurality of combinations of allele counts and pathogenicity score thresholds is corrected to account for the multiple tests (*i.e., t*-tests for genetic association tests of a particular plurality of rare deleterious variants for a particular gene on a particular phenotype performed within a grid search over a the plurality of combinations of allele counts and pathogenicity score thresholds).

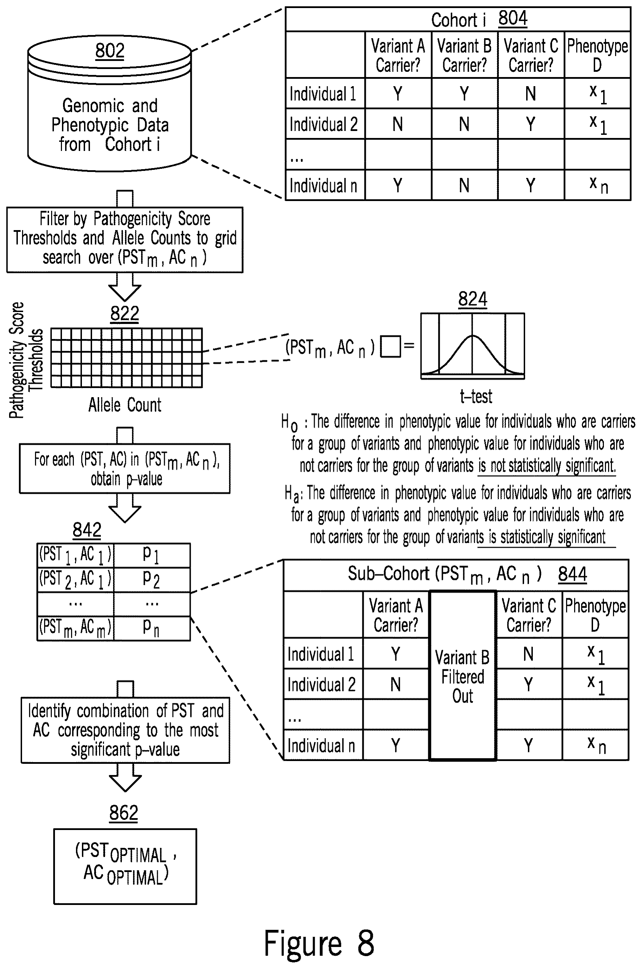

Figure 8 is a schematic diagram of a method for optimizing rare variant burden testing. A database 802 comprises genomic and phenotypic data corresponding to a group of individuals belonging to cohort *i*. Cohort *i* 804 comprises *N* individuals who have undergone genetic sequencing and testing for a plurality of phenotypic measurements. Sequencing data for individuals within cohort *i* 804 comprises data corresponding to a plurality of variant carrier statuses. A particular gene may possess three known possible rare deleterious variants (*e.g.*, variant A, variant B, and variant C) of which an individual may be a carrier (*i.e.,* the individual possesses a respective variant, therefore they are a carrier of that variant) or a noncarrier (*i.e.,* the individual does not possess the respective variant, therefore they are not a carrier of that variant). A person skilled in the art will recognize that variant A, variant B, and variant C are given as an illustrative example and a gene may possess any number of rare deleterious variants. The technology disclosed defines a variant as 'rare' if the variant occurrence rate within a population is below a predetermined threshold and 'deleterious' if the pathogenicity of the variant is predicted to have a measured impact on impact when the gene is expressed as a protein above a predetermined threshold. The respective predetermined thresholds for both rarity and pathogenicity are gene-specific (*i.e.,* each individual gene will correspond to an individually determined rarity threshold and pathogenicity threshold that may differ from another gene).

In addition to genomic data obtained from genome and exome sequencing for each individual within cohort *i* 804, phenotypic data is also available for each individual (*i.e.,* individual *N* has a measured value of $x_n$ for phenotype D).

To determine the optimal threshold for rarity and pathogenicity, a grid search 822 is performed to search over a space comprising all possible pathogenicity score thresholds (PST) and maximum allele count thresholds (AC). Within grid 822, each individual test (corresponding to PST value *m* and AC value *N*) is a t-test 824. For each t-test 824, the null hypothesis states that the difference in phenotypic value for individuals who are carriers for a group of variants and phenotypic value for individuals who are not carriers for the group of variants is not statistically significant. The alternative hypothesis states that the difference in phenotypic value for individuals who are carriers for a group of variants and phenotypic value for individuals who are not carriers for the group of variants is statistically significant. The *t*-test is iteratively performed over the entire grid for each possible combination of PST and AC values to obtain a *p*-value for each *t*-test at a uniform significance level. Within the plurality of PST value and AC value combinations and associated *p*-values 842, a sub-cohort ($PST_m$, $AC_n$) 844 contains genomic and phenotypic data for *N* individuals that meet the specified PST value *m* and AC value *N* (*i.e.,* if variant B does not meet the specified threshold for pathogenicity or allele count for a particular t-test, carrier status for variant B is not considered within the aggregated binary carrier status variable).

The combination of PST and AC corresponding to the most significant *p*-value is output as the optimal PST and AC combination 862 for the particular gene. In some implementations of the technology disclosed, rare variant burden tests are performed following optimization at the determined optimal PST and AC combination 862. Thus, the method of performing an optimized burden test is performed such that all optimized burden tests for the particular gene will be initiated such that an optimal combination of a maximum allele count and a minimum pathogenicity score threshold that maximizes significance of burden testing effects of rare pathogenic variants in a particular gene on a particular phenotype are employed as parameters for the burden test for the particular gene.

### Protein Structure-Based Pathogenicity Determination

The discussion thus far has described a rare variant polygenic risk score model configured to aggregate risk across genes based on whether individuals carry rare deleterious variants in each gene. The rare variant polygenic risk score model described in Figure 1, Figure 6, Figure 7, and Figure 8 are performed at an optimal combination of a maximum allele count and a minimum pathogenicity score threshold that maximizes significance of burden testing effects of rare pathogenic variants in a particular gene on a particular phenotype.

The discussion now turns to a description of systems for protein structure-based pathogenicity determination, wherein one implementation of the technology disclosed implements a convolutional neural network pathogenicity classifier configured to determine pathogenicity of variants. A pathogenicity score output corresponding to a particular variant for a particular gene may be implemented as the pathogenicity score threshold for a particular gene in a particular t-test 824. A plurality of *m* pathogenicity score thresholds may be tested within a grid search 822 to determine an optimal minimum pathogenicity score threshold such as the optimal pathogenicity score threshold 862.

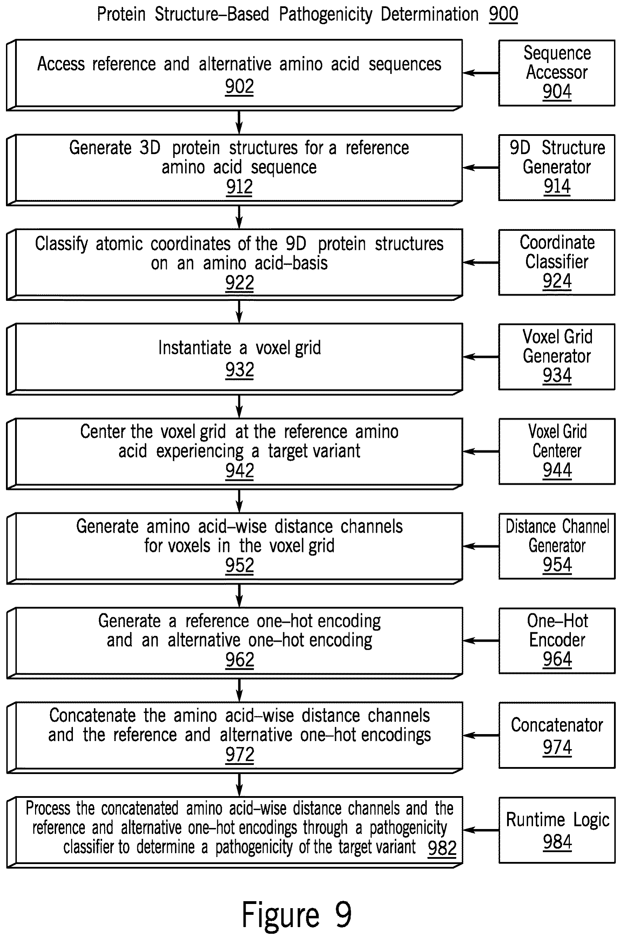

Figure 9 is a flow diagram 900 that illustrates a process of a system for determining pathogenicity of variants. At step 902, a sequence accessor 904 of the system accesses reference and alternative amino acid sequences. At 912, a 3D structure generator 914 of the system generates 3D protein structures for a reference amino acid sequence. In some implementations, the 3D protein structures are homology models of human proteins. In one implementation, a so-called SwissModel homology modelling pipeline provides a public repository of predicted human protein structures. In another implementation, a so-called HHpred homology modelling uses a tool called Modeller to predict the structure of a target protein from template structures.

Proteins are represented by a collection of atoms and their coordinates in 3D space. An amino acid can have a variety of atoms, such as carbon atoms, oxygen (O) atoms, nitrogen (N) atoms, and hydrogen (H) atoms. The atoms can be further classified as side chain atoms and backbone atoms. The backbone carbon atoms can include alpha-carbon (Cα) atoms and β-carbon (Cβ) atoms.

At step 922, a coordinate classifier 924 of the system classifies 3D atomic coordinates of the 3D protein structures on an amino acid-basis. In one implementation, the amino acid-wise classification involves attributing the 3D atomic coordinates to the twenty-one amino acid categories (including stop or gap amino acid category). In one example, an amino acid-wise classification of alpha-carbon atoms can respectively list alpha-carbon atoms under each of the twenty-one amino acid categories. In another example, an amino acid-wise classification of β-carbon atoms can respectively list β-carbon atoms under each of the twenty-one amino acid categories.

In yet another example, an amino acid-wise classification of oxygen atoms can respectively list oxygen atoms under each of the twenty-one amino acid categories. In yet another example, an amino acid-wise classification of nitrogen atoms can respectively list nitrogen atoms under each of the twenty-one amino acid categories. In yet another example, an amino acid-wise classification of hydrogen atoms can respectively list hydrogen atoms under each of the twenty-one amino acid categories.

A person skilled in the art will appreciate that, in various implementations, the amino acid-wise classification can include a subset of the twenty-one amino acid categories and a subset of the different atomic elements.

This application uses voxels and voxelization as non-limiting examples. A person skilled in the art will appreciate that, in various implementations, different formats of arranging and processing data, such as features, vectors, arrays, of various dimensionalities, can be used as alternatives or as combinations.

At step 932, a voxel grid generator 934 of the system instantiates a voxel grid. The voxel grid can have any resolution, for example, 3x3x3, 5x5x5, 7x7x7, and so on. Voxels in the voxel grid can be of any size, for example, one angstrom (Å) on each side, two Å on each side, three Å on each side, and so on. One skilled in the art will appreciate that these example dimensions refer to cubic dimensions because voxels are cubes. Also, one skilled in the art will appreciate that these example dimensions are non-limiting, and the voxels can have any cubic dimensions.

At step 942, a voxel grid centerer 944 of the system centers the voxel grid at the reference amino acid experiencing a target variant at the amino acid level. In one implementation, the voxel grid is centered at an atomic coordinate of a particular atom of the reference amino acid experiencing the target variant, for example, the 3D atomic coordinate of the alpha-carbon atom of the reference amino acid experiencing the target variant.

## Distance Channels

The voxels in the voxel grid can have a plurality of channels (or features). In one implementation, the voxels in the voxel grid have a plurality of distance channels (e.g., twenty-one distance channels for the twenty-one amino acid categories, respectively (including stop or gap amino acid category)). At step 952, a distance channel generator 954 of the system generates amino acid-wise distance channels for the voxels in the voxel grid. The distance channels are independently generated for each of the twenty-one amino acid categories.

Consider, for example, the Alanine (A) amino acid category. Further consider, for example, that the voxel grid is of size 3x3x3 and has twenty-seven voxels. Then, in one implementation, an Alanine distance channel includes twenty-seven distance values for the twenty-seven voxels in the voxel grid, respectively. The twenty-seven distance values in the Alanine distance channel are measured from respective centers of the twenty-seven voxels in the voxel grid to respective nearest atoms in the Alanine amino acid category.

In one example, the Alanine amino acid category includes only alpha-carbon atoms and therefore the nearest atoms are those Alanine alpha-carbon atoms that are most proximate to the twenty-seven voxels in the voxel grid, respectively. In another example, the Alanine amino acid category includes only β-carbon atoms and therefore the nearest atoms are those Alanine β-carbon atoms that are most proximate to the twenty-seven voxels in the voxel grid, respectively.

In yet another example, the Alanine amino acid category includes only oxygen atoms and therefore the nearest atoms are those Alanine oxygen atoms that are most proximate to the twenty-seven voxels in the voxel grid, respectively. In yet another example, the Alanine amino acid category includes only nitrogen atoms and therefore the nearest atoms are those Alanine nitrogen atoms that are most proximate to the twenty-seven voxels in the voxel grid, respectively. In yet another example, the Alanine amino acid category includes only hydrogen atoms and therefore the nearest atoms are those Alanine hydrogen atoms that are most proximate to the twenty-seven voxels in the voxel grid, respectively.

Like the Alanine distance channel, the distance channel generator 954 generates a distance channel (*i.e.*, a set of voxel-wise distance values) for each of the remaining amino acid categories. In other implementations, the distance channel generator 954 generates distance channels only for a subset of the twenty-one amino acid categories.

In other implementations, the selection of the nearest atoms is not confined to a particular atom type. That is, within a subject amino acid category, the nearest atom to a particular voxel is selected, irrespective of the atomic element of the nearest atom, and the distance value for the particular voxel calculated for inclusion in the distance channel for the subject amino acid category.

In yet other implementations, the distance channels are generated on an atomic element-basis. Instead of or in addition to having the distance channels for the amino acid categories, distance values can be generated for atom element categories, irrespective of the amino acids to which the atoms belong. Consider, for example, that the atoms of amino acids in the reference amino acid sequence span seven atomic elements: carbon, oxygen, nitrogen, hydrogen, calcium, iodine, and sulfur. Then, the voxels in the voxel grid are configured to have seven distance channels, such that each of the seven distance channels have twenty-seven voxel wise distance values that specify distances to nearest atoms only within a corresponding atomic element category. In other implementations, distance channels for only a subset of the seven atomic elements can be generated. In yet other implementations, the atomic element categories and the distance channel generation can be further stratified into variations of a same atomic element, for example, alpha-carbon ($C_\alpha$) atoms and β-carbon ($C_\beta$) atoms.

In yet other implementations, the distance channels can be generated on an atom type-basis, for example, distance channels only for side chain atoms and distance channels only for backbone atoms.

The nearest atoms can be searched within a predefined maximum scan radius from the voxel centers (e.g., six angstrom (Å)). Also, multiple atoms can be nearest to a same voxel in the voxel grid.

The distances are calculated between 3D coordinates of the voxel centers and 3D atomic coordinates of the atoms. Also, the distance channels are generated with the voxel grid centered at a same location (e.g., centered at the 3D atomic coordinate of the alpha-carbon atom of the reference amino acid experiencing the target variant).

The distances can be Euclidean distances. Also, the distances can be parameterized by atom size (or atom influence) (e.g., by using Lennard-Jones potential and/or Van der Waals atom radius of the atom in question). Also, the distance values can be normalized by the maximum scan radius, or by a maximum observed distance value of the furthest nearest atom within a subject amino acid category or a subject atomic element category or a subject atom type category. In some implementations, the distances between the voxels and the atoms are calculated based on polar coordinates of the voxels and the atoms. The polar coordinates are parameterized by angles between the voxels and the atoms. In one implementation, this angel information is used to generate an angle channel for the voxels (*i.e.,* independent of the distance channels). In some implementations, angles between a nearest atom and neighboring atoms (*e.g.*, backbone atoms) can be used as features that are encoded with the voxels.

## Reference Allele and Alternative Allele Channels

The voxels in the voxel grid can also have reference allele and alternative allele channels. At step 962, a one-hot encoder 964 of the system generates a reference one-hot encoding of a reference amino acid in the reference amino acid sequence and an alternative one-hot encoding of an alternative amino acid in an alternative amino acid sequence. The reference amino acid experiences the target variant. The alternative amino acid is the target variant. The reference amino acid and the alternative amino acid are located at a same position respectively in the reference amino acid sequence and the alternative amino acid sequence. The reference amino acid sequence and the alternative amino acid sequence have the same position-wise amino acid composition with one exception. The exception is the position that has the reference amino acid in the reference amino acid sequence and the alternative amino acid in the alternative amino acid sequence.

At step 972, a concatenator 974 of the system concatenates the amino acid-wise distance channels and the reference and alternative one-hot encodings. In another implementation, the concatenator 974 concatenates the atomic element-wise distance channels and the reference and alternative one-hot encodings. In yet another implementation, the concatenator 974 concatenates the atomic type-wise distance channels and the reference and alternative one-hot encodings.

At step 982, runtime logic 984 of the system processes the concatenated amino acid-wise/atomic element-wise/atomic type-wise distance channels and the reference and alternative one-hot encodings through a pathogenicity classifier (pathogenicity determination engine) to determine a pathogenicity of the target variant, which is in turn inferred as a pathogenicity determination of the underlying nucleotide variant that creates the target variant at the amino acid level. The pathogenicity classifier is trained using labelled datasets of benign and pathogenic variants, for example, using the backpropagation algorithm. Additional details about the labelled datasets of benign and pathogenic variants and example architectures and training of the pathogenicity classifier can be found in commonly owned US Patent Application Nos. 16/160,903; 16/160,986; 16/160,968; and 16/407,149.

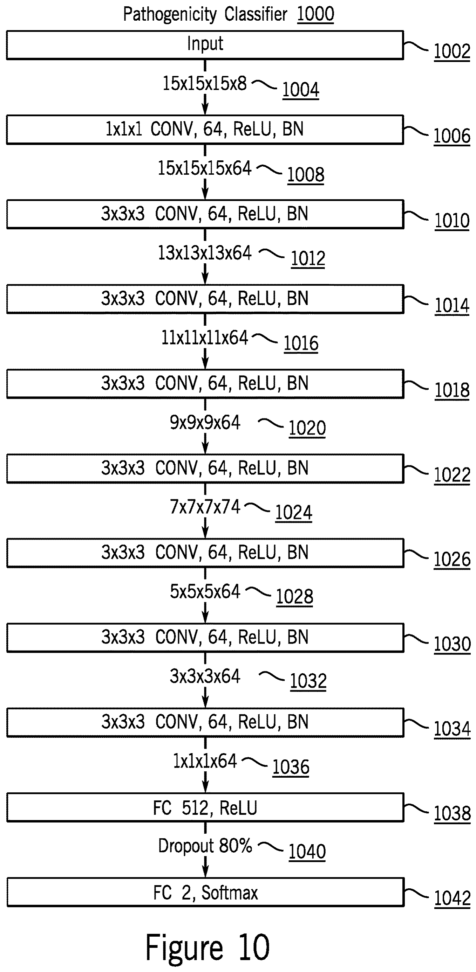

Figure 10 shows an example processing architecture 1000 of the pathogenicity classifier 900, in accordance with one implementation of the technology disclosed. The processing architecture 1000 includes a cascade of processing modules 1006, 1010, 1014, 1018, 1022, 1010, 1030, 1034, 1038, and 1042 each of which can include 1D convolutions (1x1x1 CONV), 3D convolutions (3x3x3 CONV), ReLU non-linearity, and batch normalization (BN). Other examples of the processing modules include fully-connected (FC) layers, a dropout layer, a flattening layer, and a final softmax layer that produces exponentially normalized scores for the target variant belonging to a benign class and a pathogenic class. In Figure 10, "64" denotes a number of convolution filters applied by a particular processing module. In Figure 10, the size of an input voxel 1002 is 15x15x15x8. Figure 10 also shows respective volumetric dimensionalities of the intermediate inputs 1004, 1008, 1012, 1016, 1020, 1024, 1028, 1032, 1036, and 1040 generated by the processing architecture 1000.

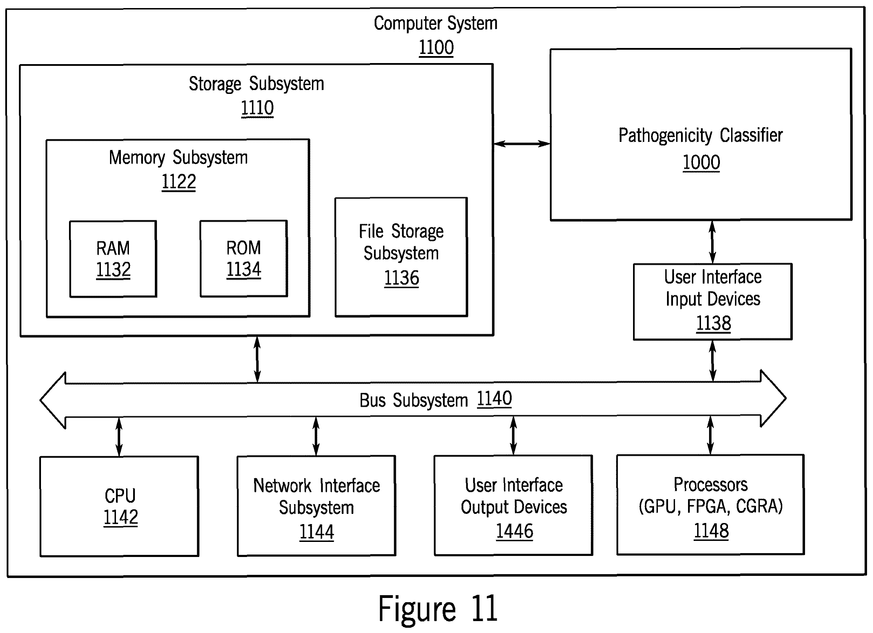

Figure 11 shows an example computer system 1100 that can be used to implement the technology disclosed. Computer system 1100 includes at least one central processing unit (CPU) 1144 that communicates with a number of peripheral devices via bus subsystem 1142. These peripheral devices can include a storage subsystem 1110 including, for example, memory devices and a file storage subsystem 1136, user interface input devices 1138, user interface output devices 1148, and a network interface subsystem 1146. The input and output devices allow user interaction with computer system 1100. Network interface subsystem 1146 provides an interface to outside networks, including an interface to corresponding interface devices in other computer systems.

In one implementation, the pathogenicity classifier 1000 is communicably linked to the storage subsystem 1110 and the user interface input devices 1138.

User interface input devices 1138 can include a keyboard; pointing devices such as a mouse, trackball, touchpad, or graphics tablet; a scanner; a touch screen incorporated into the display; audio input devices such as voice recognition systems and microphones; and other types of input devices. In general, use of the term "input device" is intended to include all possible types of devices and ways to input information into computer system 1100.

User interface output devices 1148 can include a display subsystem, a printer, a fax machine, or non-visual displays such as audio output devices. The display subsystem can include an LED display, a cathode ray tube (CRT), a flat-panel device such as a liquid crystal display (LCD), a projection device, or some other mechanism for creating a visible image. The display subsystem can also provide a non-visual display such as audio output devices. In general, use of the term "output device" is intended to include all possible types of devices and ways to output information from computer system 1100 to the user or to another machine or computer system.

Storage subsystem 1110 stores programming and data constructs that provide the functionality of some or all of the modules and methods described herein. These software modules are generally executed by processors 1148.

Processors 1148 can be graphics processing units (GPUs), field-programmable gate arrays (FPGAs), application-specific integrated circuits (ASICs), and/or coarse-grained reconfigurable architectures (CGRAs). Processors 1148 can be hosted by a deep learning cloud platform such as Google Cloud Platform™, Xilinx™, and Cirrascale™. Examples of processors 1148 include Google's Tensor Processing Unit (TPU)™, rackmount solutions like GX4 Rackmount Series™, GX11 Rackmount Series™, NVIDIA DGX-1™, Microsoft' Stratix V FPGA™, Graphcore's Intelligent Processor Unit (IPU)™, Qualcomm's Zeroth Platform™ with Snapdragon processors™, NVIDIA's Volta™, NVIDIA's DRIVE PX™, NVIDIA's JETSON TX1/TX11 MODULE™, Intel's Nirvana™, Movidius VPU™, Fujitsu DPI™, ARM's DynamicIQ™, IBM TrueNorth™, Lambda GPU Server with Testa V100s™, and others.

Memory subsystem 1122 used in the storage subsystem 1110 can include a number of memories including a main random-access memory (RAM) 1132 for storage of instructions and data during program execution and a read only memory (ROM) 1134 in which fixed instructions are stored. A file storage subsystem 1136 can provide persistent storage for program and data files, and can include a hard disk drive, a floppy disk drive along with associated removable media, a CD-ROM drive, an optical drive, or removable media cartridges. The modules implementing the functionality of certain implementations can be stored by file storage subsystem 1136 in the storage subsystem 1110, or in other machines accessible by the processor.

Bus subsystem 1140 provides a mechanism for letting the various components and subsystems of computer system 1100 communicate with each other as intended. Although bus subsystem 1142 is shown schematically as a single bus, alternative implementations of the bus subsystem can use multiple busses.

Computer system 1100 itself can be of varying types including a personal computer, a portable computer, a workstation, a computer terminal, a network computer, a television, a mainframe, a server farm, a widely-distributed set of loosely networked computers, or any other data processing system or user device. Due to the ever-changing nature of computers and networks, the description of computer system 1100 depicted in Figure 11 is intended only as a specific example for purposes of illustrating the preferred implementations of the present invention. Many other configurations of computer system 1100 are possible having more or less components than the computer system depicted in Figure 11.

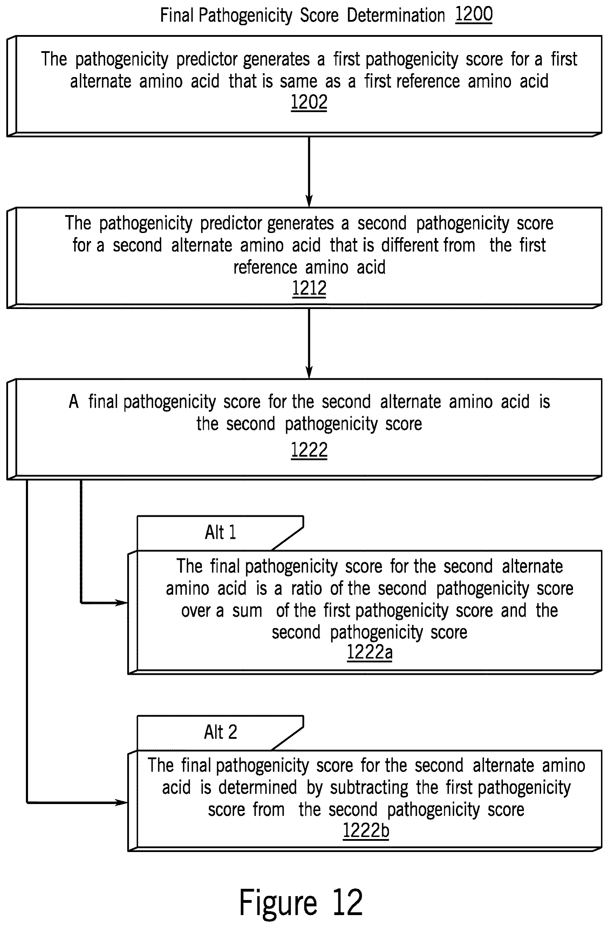

Figure 12 illustrates one implementation 1200 of determining a final pathogenicity score. At action 1202, in one implementation, the pathogenicity classifier 1000 generates a first pathogenicity score for a first alternate amino acid that is same as a first reference amino acid. At action 1212, in one implementation, the pathogenicity classifier 1000 generates a second pathogenicity score for a second alternate amino acid that is different from the first reference amino acid. At action 1222, in one implementation, a final pathogenicity score for the second alternate amino acid is the second pathogenicity score for the second alternate amino acid.

In other alternatives, the final pathogenicity score for the second alternate amino acid is based on a combination of the first pathogenicity score and the second pathogenicity score. In a first alternative at 1222a, in one implementation, the final pathogenicity score for the second alternate amino acid is a ratio of the second pathogenicity score over a sum of the first pathogenicity score and the second pathogenicity score. In a second alternative at 1222b, in one implementation, the final pathogenicity score for the second alternate amino acid is determined by subtracting the first pathogenicity score from the second pathogenicity score.

A person skilled in the art will appreciate that other current and future artificial intelligence, machine learning, and deep learning models, datasets, and training techniques can be incorporated in the disclosed variant pathogenicity classifier without deviating from the spirit of the technology disclosed.

## Correction for Multiple Testing

The discussion thus far has described an implementation of the technology disclosed comprising a method of performing an optimized burden test wherein a strength of association is quantified between genes associated with a phenotype and a contribution of rare variants to a phenotype response, wherein the burden test is optimized for a particular combination of an allele count and a pathogenicity score threshold that has a most significant $p$-value. The optimized burden test is based on a plurality of nested $t$-tests that maximize separation between carriers and non-carriers of at least one rare deleterious variant within a particular gene. In this implementation of the technology disclosed, the multiple $t$-tests performed within the optimization method warrants a necessity to correct for multiple testing within each gene.

The discussion now turns to a description of correcting the most significant $p$-value corresponding to the optimal combination of PST and AC values.

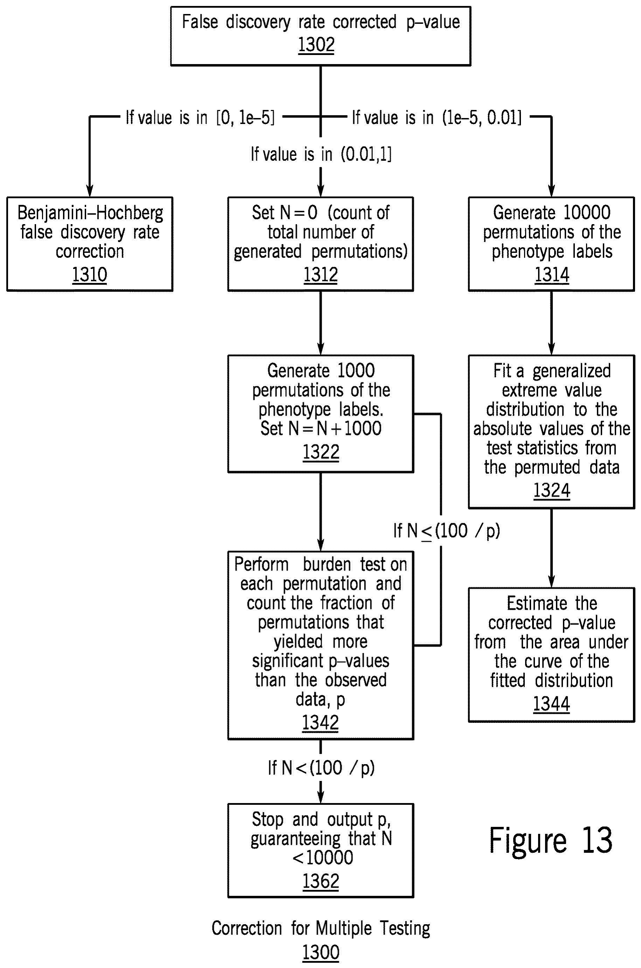

Figure 13 is a flow diagram 1300 of a process for correcting for multiple testing within each gene. Following false discovery rate (FDR) correcting, $p$-values are further corrected for multiple testing to account for burden test optimization for both PST and AC. FDR corrected $p$-values 1302 are separated into value ranges. In one implementation of the technology disclosed, the following algorithm 1300 for multiple testing correction is followed. In other implementations of the technology disclosed, other forms of multiple testing correction may be used. For values in the range (0, 1e-5], FDR corrected $p$-values 1302 undergo Benjamini-Hochberg FDR correction 1310.

For values in the range (0.01, 1], FDR corrected $p$-values 1302 undergo permutation testing step 1312. At step 1312, in one implementation, the count of total number of generated permutations ($N$) is set to zero. In other implementations, $N$ can be set to any predefined starting value. In other implementations, $N$ can be set to any predefined starting value. Following step 1312, step 1322 involves the generation of 1,000 permutations of the phenotype labels, setting $N$ to equal $N + 1000$. In the next step 1342, a burden test is performed on each permutation of the data and the fraction of permutations that yield more significant $p$-values than the original observed data are counted, $p$. If $N$ is larger than $100 / p,$ continue to step 1362. At step 1362, stop and output $p$. The stop point at step 1362 guarantees that $N < 10,000$, maintaining computational efficiency. That is, the number of permutations ($N$) is restricted to under 10,000, and thereby prevented from reaching a computationally-unfeasible or computationally-inefficient or computationally-expensive count.

For values in the range (1e-5, 0.01], FDR corrected $p$-values 1302 undergo permutation testing step 1314. At step 1314, 10,000 permutations are generated of the phenotype labels. Following step 1314, step 1324 involves the fitting of a generalized extreme value distribution to the absolute values of the test statistics for each respective burden test from the permuted data. In the next step 1344, the corrected $p$-value is estimated from the area under the curve of the fitted distribution. That is, at step 1362, the initial $p$-value is outputted, whereas, at step 1344, a corrected $p$-value is estimated.

## Correction of Phenotype Values for Covariates

Thus far, the discussion has covered an of quantifying a strength of association of genes associated with a phenotype and a contribution of rare variants to a phenotype response, wherein the effective strength score of the plurality of genes contributing to a rare polygenic risk score is determined by a Boolean carrier status variable and the rare polygenic risk score model *(i.e.,* burden test) is optimized for a particular optimum combination of an allele count and a pathogenicity score threshold that has a most significant $p$-value. The discussion now revisits the concept of phenotype measurements to introduce an additional method of optimization in which phenotype measurements are covariate-corrected and drug usage-corrected.

Note that the Application uses the terms "drug usage", "drug usage pattern", "drug category usage", and "drug category usage pattern" interchangeably. Also note that the Application uses the term "confounder" to describe a covariate associated to both one or more other covariates and the outcome of interest (*e.g.*, tobacco use is a confounder associated with drug usage patterns and phenotype measurements for cardiovascular disease). A person skilled in the art will appreciate that the technology disclosed can be applied to any disease, such as oncology diseases, diabetes, and so on.

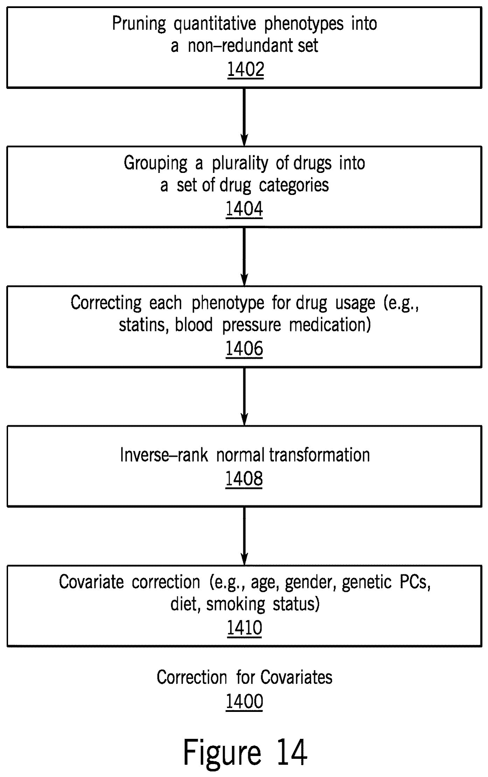

Figure 14 is a schematic diagram for a method 1400 of correcting phenotypic values for covariates. At step 1402, a dataset of quantitative phenotypes is pruned into a non-redundant dataset to filter out repeat or similar prototypes. At step 1404, a plurality of drugs of interest are grouped into a set of drug categories (the grouping can be based on, for example, similar target phenotype or disease). At step 1406, each phenotype within the non-redundant quantitative phenotype dataset is corrected for drug usage for each drug category within the set of drug categories (*e.g.*, statins, blood pressure medication, and so on). At step 1408, each corrected phenotype is inverse-rank normal transformed. At step 1410, the corrected phenotypes are further corrected for a plurality of confounders (*e.g.*, age, gender, genetic principal components, diet, smoking status, and so on). Regarding confounders, confounders are factors that confound the relationship between the trial drug and outcome. Some trials may permit adjunctive drugs during the trial or adjunctive treatments such as psychotherapy. These are confounders because results may be attributable to the adjuncts rather than to the drug being tested. As an example, trials of pain medications often ignore participants' use of nondrug pain relievers such as splints, creams, massage, hot baths, and chiropractors' manipulations. Response to these extra trial treatments may significantly confound the results of the trial. Common confounders are attributes of the participants; for example, body mass index, smoking status, age at onset of illness, socioeconomic status, educational status, and extent of support network. Life events are also potential confounders. They can cause major changes in participants' mood and symptom level, and this is particularly true of events, no matter how small, in the relationship between participant and rater. All known confounders that are not matched between experimental and control groups can be considered in the statistical analysis.

The discussion now turns to a detailed description of one implementation of the technology disclosed configured to predict phenotypic shift in response to usage of a plurality of drugs on a plurality of phenotypes of a cohort of individuals at a first and second time point, wherein individuals within the cohort are grouped based on permuted drug usage patterns measured at the two time points (*e.g.,* not taking a drug at the first and second time points, start taking the drug between first and second time points, stop taking the drug between the first and second time points, and taking the drug at the first and second time points). A person skilled in the art will appreciate that the technology disclosed can be extended to any number of time points, such as three, four, five, and so on.

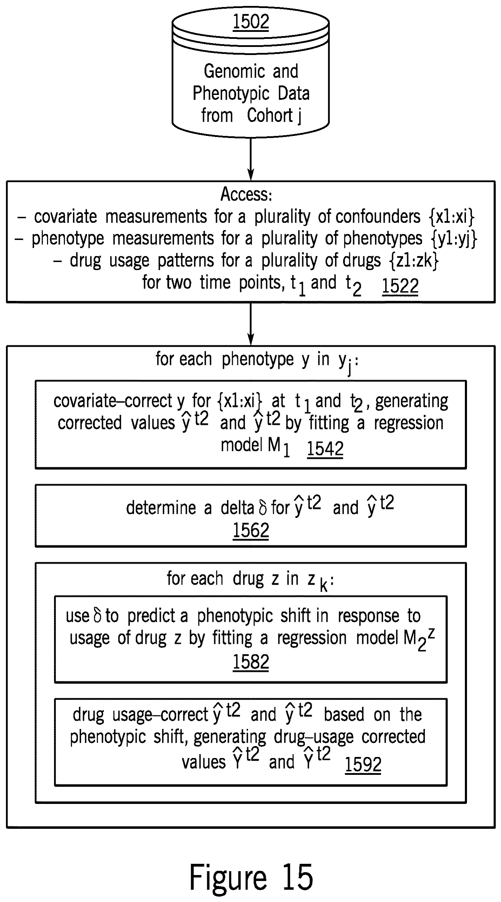

Figure 15 is a schematic diagram representing predicting phenotypic shift in response to usage of a plurality of drugs on a plurality of phenotypes. A database 1502 comprises genomic and phenotypic data corresponding to a group of individuals belonging to cohort *j*. At step 1522, covariate measurements for a plurality of confounders, phenotype measurements for a plurality of phenotypes, and drug usage patterns for a plurality of drug categories are accessed at two time points, $t_1$ and $t_2$, from database 1502. Steps 1542, 1562, 1582, and 1592 are performed for each phenotype within the plurality of phenotypes.

At step 1542, phenotype measurements are covariate-corrected for the plurality of confounders at $t_1$ and $t_2$, generating confounder-corrected values by fitting a first regression model. At step 1562, the first regression model is used to determine a delta $\delta$ for the confounder-corrected values. Hence, the covariate correction is implemented by regressing out the covariate measurements by fitting the first regression model. Steps 1582 and 1592 are performed for each drug category within the plurality of drug categories. At step 1582, $\delta$ is used to predict a phenotypic shift in response to usage of a particular drug category by fitting a second regression model. At step 1592, the confounder-corrected values are further corrected for drug-usage for the particular drug category to generate confounder-corrected, drug category-usage corrected values for the particular phenotype. Hence, the phenotypic shift prediction is implemented by fitting a second regression model that models a phenotypic shift for each of the drug usage patterns. The second regression model has a binary indicator variable for each of the drug usage patterns.

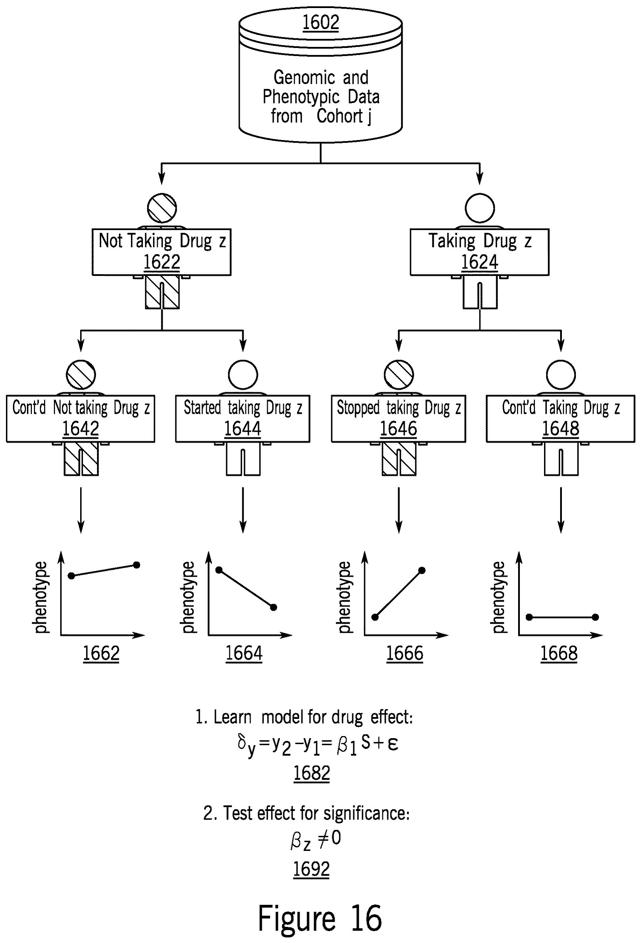

Figure 16 is a schematic diagram representing the experimental set-up to obtain drug usage patterns and phenotypic data for a particular cohort. A database 1602 comprises genomic and phenotypic data corresponding to a group of individuals belonging to cohort *j*. Database 1602 is segmented into two sub-cohorts comprising individuals not taking a particular drug category *z* 1622, and individuals taking the particular drug category z 1624 at a first timepoint $t_1$, respectively. The sub-cohort comprising individuals not taking the particular drug category z 1622 can be further segmented into smaller sub-cohorts comprising individuals who continued not taking the particular drug category z 1642, and individuals who started taking the particular drug category z 1644 following a first timepoint $t_1$ prior to a second timepoint $t_2$, respectively.

The sub-cohort comprising individuals taking the particular drug category *z* 1646 can be further segmented into smaller sub-cohorts comprising individuals who stopped taking the particular drug category *z* 1646, and individuals who continued taking the particular drug category *z* 1648 following a first timepoint $t_1$ prior to a second timepoint $t_2$, respectively.

Phenotype value trend graph 1662 illustrates an example of phenotype values measured at $t_1$ and $t_2$ for sub-cohort 1642 (*i.e.,* patients who were not taking drug category *z* at $t_1$ or $t_2$), where the particular phenotype value begins high on the y-axis at $t_1$ and increases slightly at $t_2$. Phenotype value trend graph 1664 illustrates an example of phenotype values measured at $t_1$ and $t_2$ for sub-cohort 1644 (*i.e.,* patients who were not taking drug category *z* at $t_1$, but started taking drug category *z* before $t_2$), where the particular phenotype value begins high on the y-axis at $t_1$ and decreases at $t_2$. Phenotype value trend graph 1666 illustrates an example of phenotype values measured at $t_1$ and $t_2$ for sub-cohort 1646 (*i.e.,* patients who were taking drug category *z* at $t_1$, but stopped taking drug category *z* before $t_2$), where the particular phenotype value begins low on the y-axis at $t_1$ and increases at $t_2$. Phenotype value trend graph 1668 illustrates an example of phenotype values measured at $t_1$ and $t_2$ for sub-cohort 1648 (*i.e.,* patients who were taking drug category *z* at $t_1$ and $t_2$), where the particular phenotype value begins low on the y-axis at $t_1$ and remains low at $t_2$.

In some implementations of the technology disclosed, the drug effect on phenotype value (*i.e.,* phenotypic shift in response to drug category *z*) can be learned by the model 1682, wherein the delta for phenotype values at $t_1$ and $t_2$ ($\delta_y$) is modeled as a regression with a $\beta$ coefficient for drug category z. This model can be tested for significance where the null hypothesis states that the $\beta$ coefficient for drug category z is equal to zero and the alternative hypothesis states that the $\beta$ coefficient for drug category z is not equal to zero.

In some implementations of the technology disclosed, regression models from steps 1542 and 1582 built on data from database 1602 are constructed via the following protocol:

Phenotypic shift effects of thirty-four drug categories (*e.g.*, statins, NSAIDs, opioids, and so on) were estimated from a cohort of participates who had their quantitative phenotype values and corresponding covariates measured at $t_1$ and $t_2$ separated by five years. For each quantitative phenotype, covariate-corrected values at $t_1$ and $t_2$ are generated by regressing out all covariates (*e.g.*, age, gender, genetic principal components, diet, smoking status, and so on) except drug usage. The difference between the two time points is computed as:

$$\delta = \tilde{Y}_{t2} - \tilde{Y}_{t1}$$

For each drug category X, all individuals within the cohort are partitioned into four groups (as described in sub-cohorts 1642, 1644, 1646, and 1648) according to their drug usage at $t_1$ and $t_2$ and a binary indicator variable is introduced for each group that encodes whether an individual belongs to that group:

1) Individuals who were not taking drug X at both time points, $t_1$ and $t_2$ (indicator variable: $X_{00}$)

2) Individuals who had started taking drug X between time point $t_1$ and $t_2$ (indicator variable: $X_{01}$)

3) Individuals who had stopped taking drug X between time point $t_1$ and $t_2$ (indicator variable: $X_{10}$)

4) Individuals who were taking drug X at both time points, $t_1$ and $t_2$ (indicator variable: $X_{11}$)

To determine which drugs have a significant effect on the phenotype Y, a forward selection stepwise regression of the form below is fitted, iterating over all drug categories:

$$\delta = \beta_{00}X_{00} + \beta_{01}X_{01} + \beta_{10}X_{10} + \beta_{11}X_{11} + \beta_t t + \beta_m \left(\tilde{Y}_{t1} - mean(\tilde{Y}_{t1})\right) + \beta_0$$

In the above equation, the term $\beta_t t$ models the effect of the time passed between $t_1$ and $t_2$ ($t = t_2 - t_1$ for each individual within the cohort), the term $\beta_m(\tilde{Y}_{t1} - mean(\tilde{Y}_{t1}))$ accounts for effects due to the regression to the mean between $t_1$ and $t_2$, and $\beta_0$ is the intercept. The stepwise procedure is stopped when the *p*-value of the coefficient $\beta_{01}$, which models the effect of starting drug X, has increased above $10^{-3}$.

After the set of relevant drugs *D* is determined, their individual effects can be estimated jointly by fitting the following regression:

$$\delta = \sum_{d \in D}(\beta_{00}^{(d)}X_{00}^{(d)} + \beta_{01}^{(d)}X_{01}^{(d)} + \beta_{10}^{(d)}X_{10}^{(d)} + \beta_{11}^{(d)}X_{11}^{(d)}) + \beta_t t + \beta_m \left(\tilde{Y}_{t1} - mean(\tilde{Y}_{t1})\right) + \beta_0$$

The raw values for phenotype Y at $t_1$ across all individuals in the cohort can b4e corrected as:

$$\tilde{Y}_{t1} = Y_{t1} - \sum_{d \in D}(\beta_{01}^{(d)} - \beta_{00}^{(d)})X^{(d)}$$

$X^{(d)}$ is a binary indicator variable that encodes whether an individual was taking drug X at the initial visit, $t_1$. After correcting for drug use, values $\tilde{Y}_{t1}$ can be inverse-rank normal transformed and all other covariates can be regressed out. The covariate-corrected-normalized-drug usage-corrected phenotype measurements can be used to generate rare variant polygenic risk scores.

In some implementations of the technology disclosed, drug usage correction is implemented by fitting a third regression model, including drug usage-correcting the phenotype measurement for the first time point based on a first binary indicator independent variable for a first drug usage pattern of start taking the drug between first and second time points, a second binary indicator independent variable for a second drug usage pattern of not taking a drug at the first and second time points, and a drug-specific binary indicator independent variable that encodes whether an individual was taking a particular drug at the first time point. A fourth regression model is fit, including drug usage-correcting the phenotype measurement for the second time point based on a third binary indicator independent variable for a third drug usage pattern of stop taking the drug between the first and second time points, a fourth binary indicator independent variable for a fourth drug usage pattern of taking the drug at the first and second time points, and a drug-specific binary indicator independent variable that encodes whether an individual was taking a particular drug at the second time point.

Rank-based inverse normal transformation may be applied to the drug usage-corrected phenotype measurements for the first and second time points, and generating normalized-drug usage-corrected phenotype measurements for the first and second time points. The normalized-drug usage-corrected phenotype measurements are then covariate-corrected for the first and second time points, and generating covariate-corrected-normalized-drug usage-corrected phenotype measurements for the first and second time points. The covariate-corrected-normalized-drug usage-corrected phenotype measurements may be used to generate rare variant polygenic risk scores wherein measurements for the phenotype of interest are corrected for phenotypic shift in response to covariates and drug usage patterns.

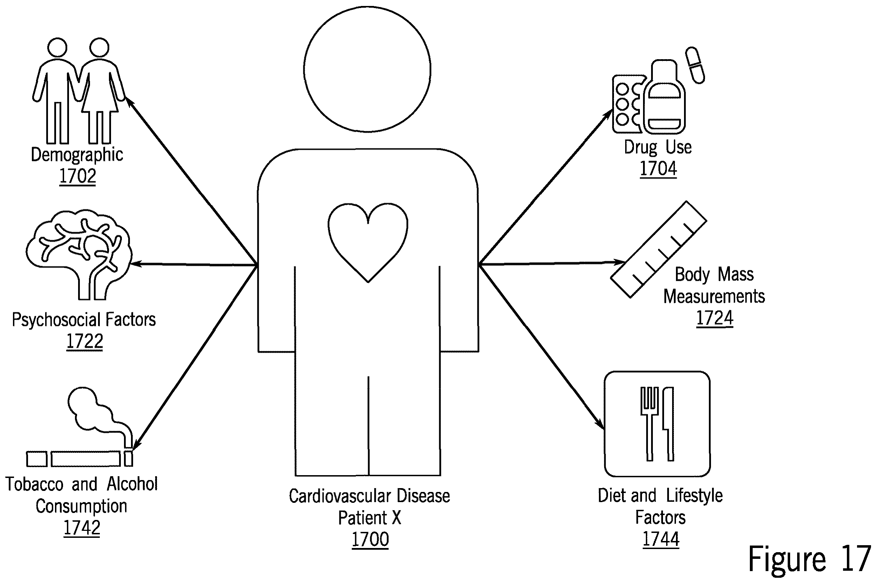

Figure 17 illustrates a plurality of phenotypes corresponding to a cardiovascular disease patient X 1700. Phenotypic values measured for cardiovascular disease patient X 1700 will be affected by a plurality of confounding factors. Examples of these confounding factors include demographic 1702 (*e.g.,* age, sex, ethnicity, and so on), psychosocial factors 1722 (mental health-related confounders, socioeconomic class, and so on), tobacco and alcohol consumption 1742, drug use 1704 (*e.g.,* drugs not included in formal drug effect analysis such as illicit drugs), body mass measurements 1724 (*e.g.*, body mass index, body fat percentage, abdominal fat density, and so on), or diet and lifestyle factors 1744 (*e.g.,* dietary restrictions, dietary habits, exercise, and so on). A person skilled in the art will recognize that these are nonlimiting confounders and a wide plurality of additional confounders exist that may influence measured phenotype values.

## Performance Measure Results as Objective Indicia of Non-Obviousness and Inventiveness

The discussion thus far has covered a plurality of implementations of the technology disclosed for rare variant burden testing comprising polygenic risk score models constructed for rare deleterious variant carrier status within a particular gene for a particular phenotype, wherein model parameters (*i.e.,* pathogenicity score threshold and maximum allele count) are optimized via nested *t*-tests (*i.e.,* grid search) and both phenotype values are covariate-corrected for a plurality of covariates and drug usage-corrected for a plurality of drug usage patterns. The discussion now turns to performance results of various implementations of the technology disclosed.

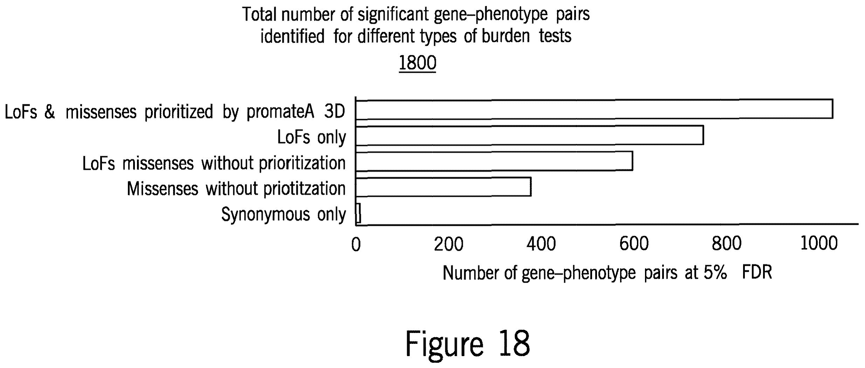

Figure 18 is a graph 1800 quantifying the total number of significant gene-phenotype pairs identified for different types of burden tests. Total number of significant gene-phenotype pairs identified across 90 phenotypes for different types of burden tests, showing that a combined test for rare LoF and deleterious missense variants prioritized by PrimateAI-3D outperforms other approaches. As a negative control, the number of significant genotype-phenotype pairs for a burden test conducted on synonymous variants is also shown.

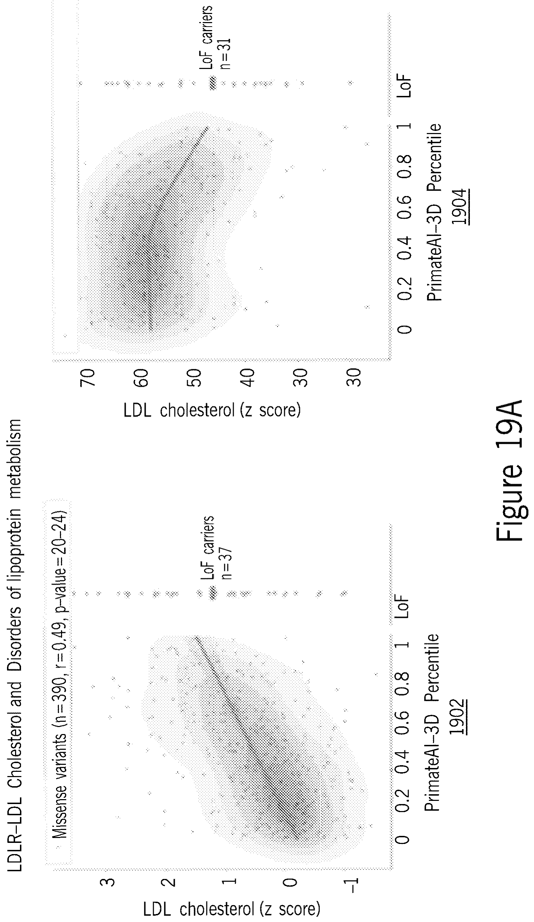

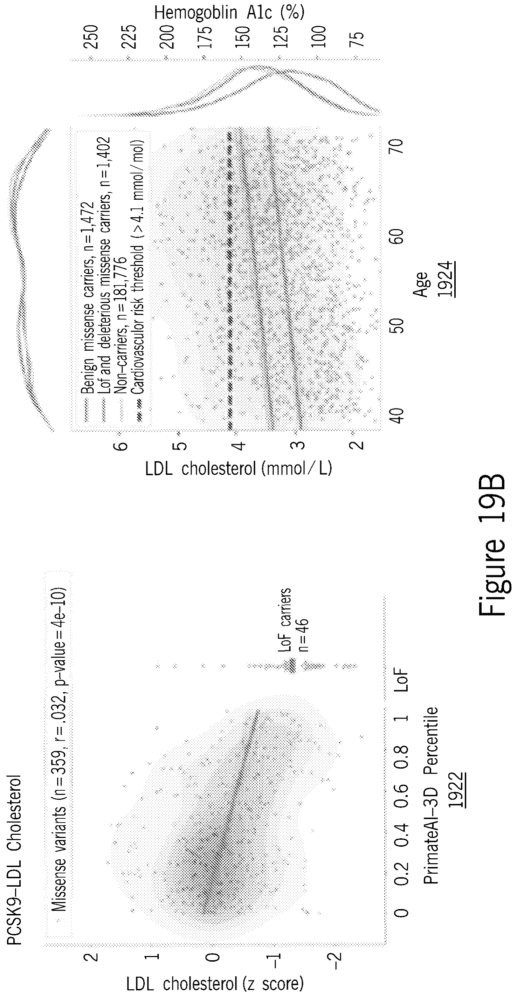

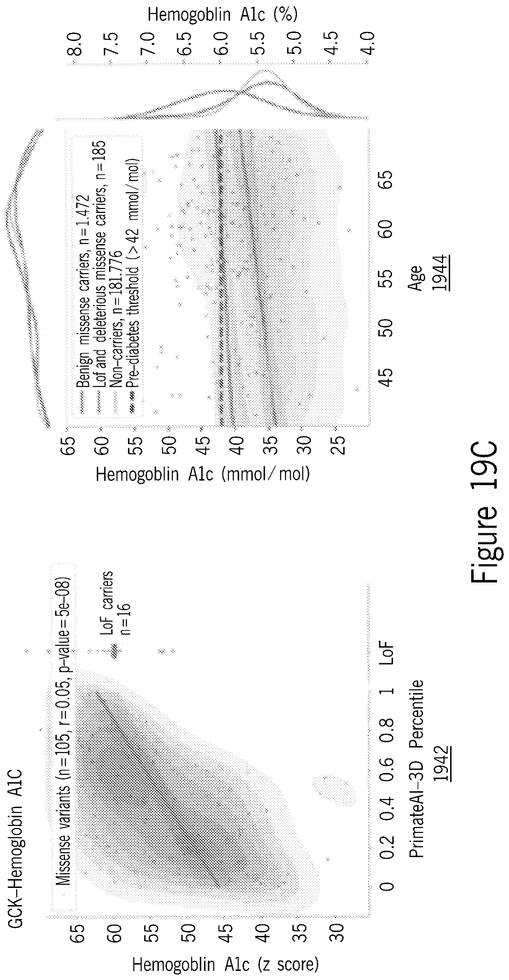

Figure 19 is a collection of graphs illustrating rare deleterious variants that affect disease severity and age of onset identified by the pathogenicity classifier PrimateAI-3D. Graph 1902 is respective to the *LDLR* gene and its association with LDL cholesterol and disorders of lipoprotein metabolism. For carriers of rare missense variants in the *LDLR* gene, LDL cholesterol levels (y-axis) correlate positively with PrimateAI-3D percentile scores (x-axis). Hereafter PrimateAI-3D scores refer to PrimateAI-3D percentile scores which have been normalized from 0 to 1 within each gene in order to facilitate comparisons between genes. Graph 1904 illustrates that PrimateAI-3D score is predictive of age of onset for dyslipidemia for carriers of rare missense variants in the *LDLR* gene.

Graph 1922 is respective to the *PCSK9* gene, a down-regulator of *LDLR.* LDL cholesterol levels of carriers of missense variants in *PCSK9,* a down-regulator of *LDLR,* correlate negatively with PrimateAI-3D score. Graph 1924 shows that carriers' LDL cholesterol levels increase with age at a similar rate as noncarriers, but are on average lower than non-carriers of the same age group. Graph 1942 is respective to the *GCK* gene, showing that for carriers of rare missense variants in the *GCK* gene, HbA1c levels correlate with PrimateAI-3D. Graph 1944 shows that Carriers' HbA1c levels increase with age at a similar rate as non-carriers, but on average reach pre-diabetic thresholds sooner in their lives.

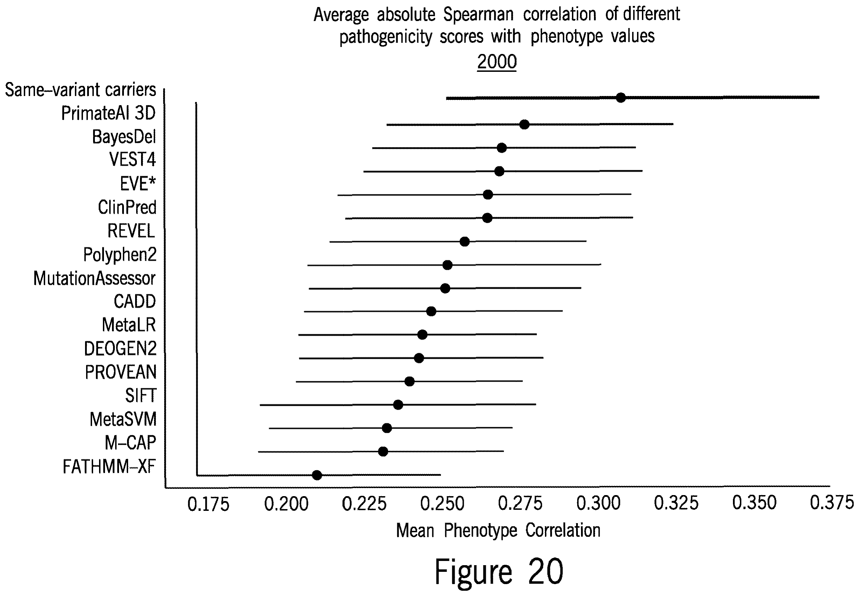

Figure 20 is a graph 2000 of the average absolute Spearman correlation of different pathogenicity scores with phenotype values. Graph 2000 shows that average absolute Spearman correlation of different pathogenicity scores with phenotype values on a benchmarking set of 34 gene-phenotype pairs, compared to the theoretical upper bound set by carriers of the same variant.

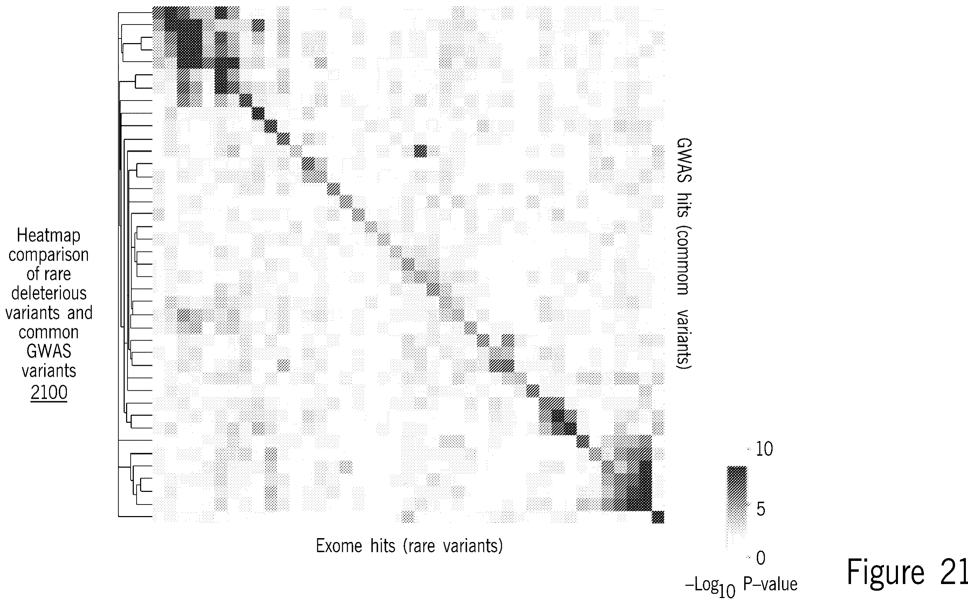

Figure 21 is a heatmap 2100 comparison of rare deleterious variants and common genome-wide association study variants. For each pair of phenotypes, the heatmap shows the statistical significance of the overlap between GWAS genes associated with the phenotype on the y-axis with the rare variant genes associated with the phenotype on the x-axis.

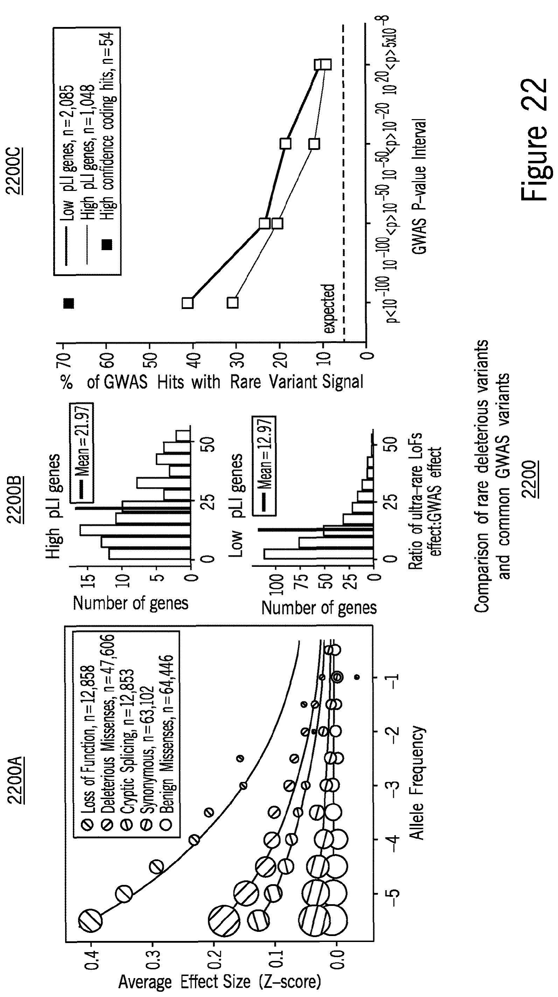

Figure 22 is a further comparison of rare deleterious variants and common genome-wide association study variants. Graph 2200A shows the Relationship of variant effect size with variant allele frequency for LoF, deleterious missense (PrimateAI-3D > 0.5) and cryptic splice (SpliceAI > 0.2) variants. Synonymous and benign missense (PrimateAI-3D < 0.5) variants are shown as negative controls. Dot sizes are proportional to the square root of the number of variants at each dot. Graph 2200B comprises the distribution of the effect sizes of the rarest LoF variants divided by the effect size of the lead GWAS variant for the same gene. Histograms for both high pLI (top plot) and low pLI (bottom plot) genes are shown. The red vertical line indicates the mean of each distribution. Graph 2200C shows the percentage of unambiguously-mapped GWAS genes with rare variant enrichment (nominal p-value $\leq$ 0.05), stratified by genes with different GWAS significance. Results are plotted separately for high pLI genes (pLI > 0.5) and low pLI genes (pLI < 0.5). High-confidence GWAS genes were defined as having a lead variant with *p*-value < 10-100 and strong LD ($r^2$ >= 0.9) with a coding variant in the associated gene. The dashed line shows the expected percentage of genes that would meet the nominal *p*-value threshold (*p* < 0.05) by chance.

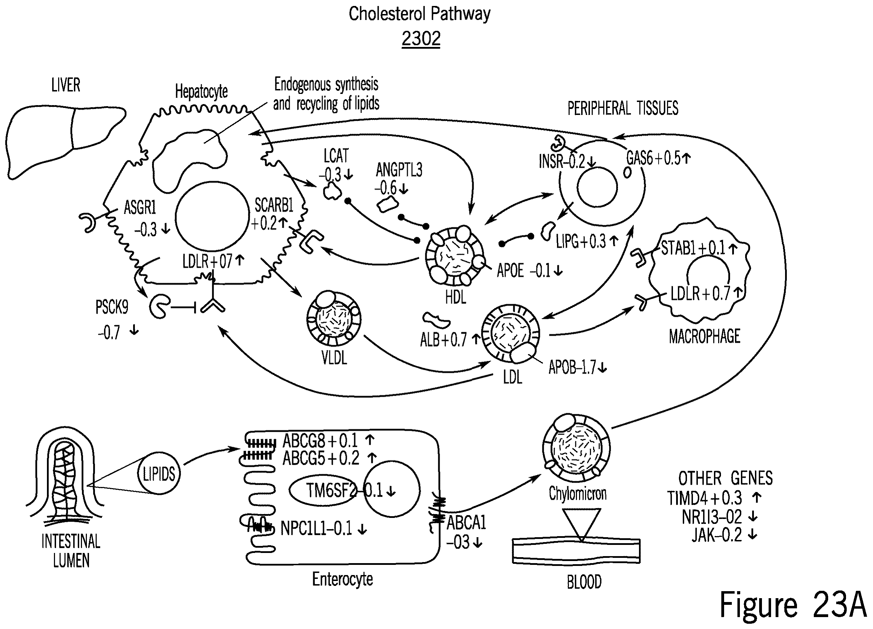

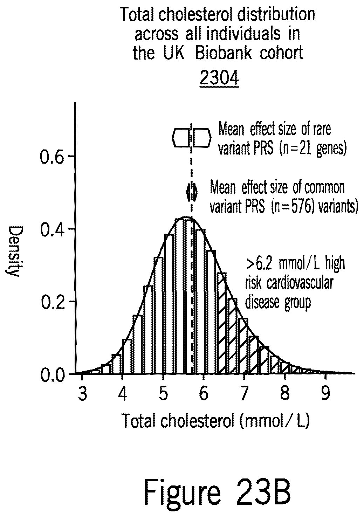

Figure 23 illustrates the cholesterol pathway and total cholesterol distribution across all individuals in the UK Biobank cohort. Illustration 2302 comprises the cholesterol pathway, with genes in the rare variant PRS model superimposed on the picture. For each gene, numbers and arrows indicate effect sizes and direction of effect. Graph 2304 shows the distribution of total cholesterol across all individuals in the UK Biobank cohort, with average effect sizes of common and rare variants shown.

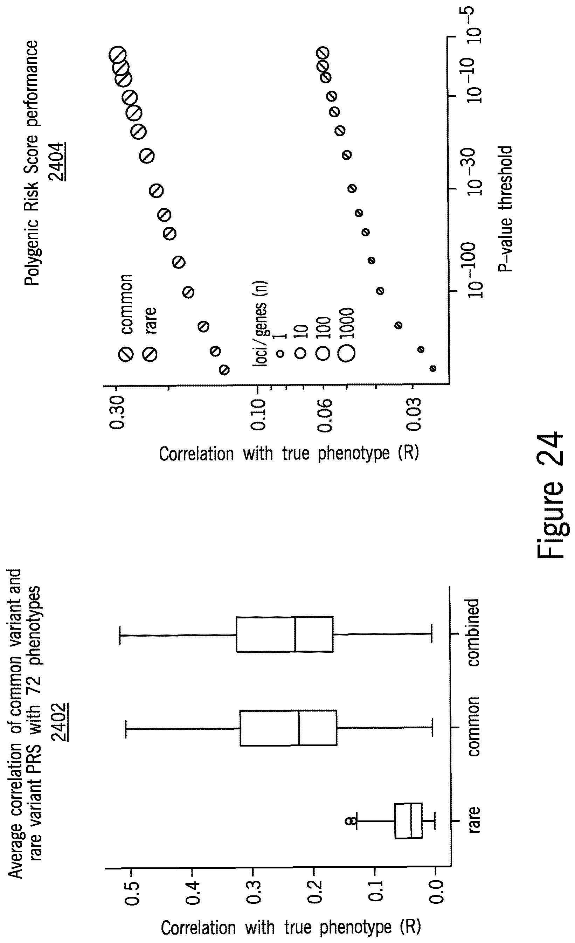

Figure 24 comprises measures of rare variant PRS performance. Graph 2402 shows the average correlation of common variant PRS (AF > 1%), rare variant PRS (AF < 0.1%), and combined PRS with 72 phenotypes, using 90% of the UKBB for PRS training, and 10% for testing. Box plots show distribution of Pearson correlations (R) between traits. Graph 2404 is a comparison of PRS performance (average correlation with phenotypes) using different significance cutoffs for determining inclusion of genes or loci into the model. Datapoint size corresponds to the number of genes or loci used in each PRS model.

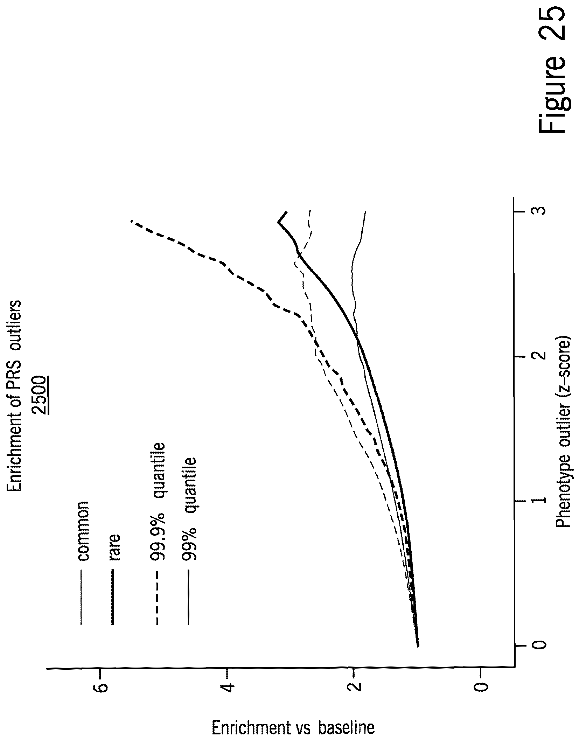

Figure 25 is a graph 2500 of the enrichment of PRS outliers. Graph 2500 shows the Enrichment of PRS outliers within individuals who are phenotype outliers. The x-axis shows the z-score threshold used to define phenotype outliers, and the y-axis shows the enrichment for PRS outliers in phenotype outlier individuals versus the baseline population.

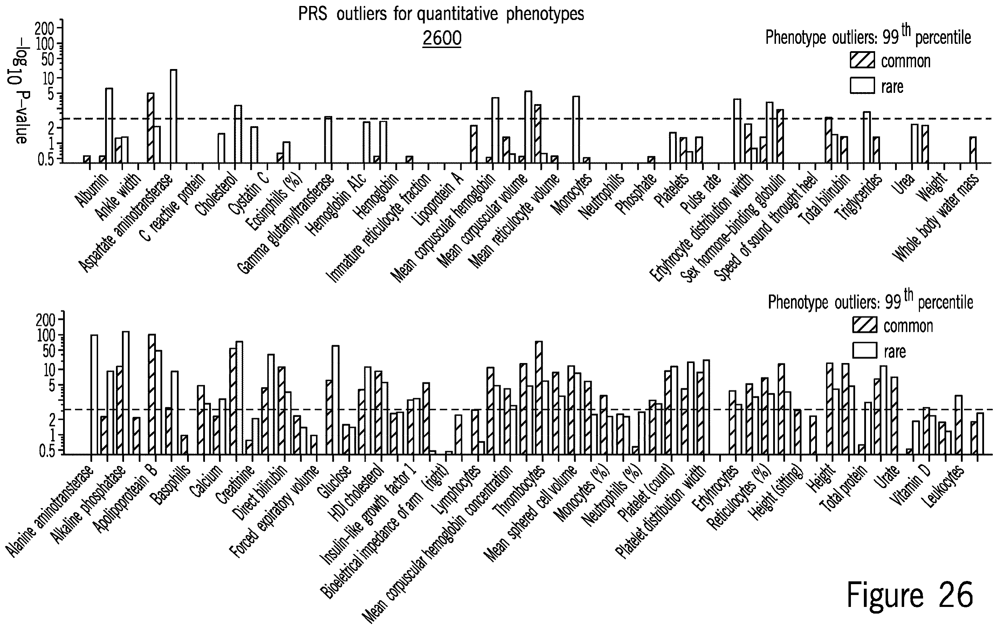

Figure 26 is a graph 2600 of PRS outliers for quantitative phenotypes. Graph 2600 shows PRS outliers for 56 quantitative phenotypes. Bars indicate statistical significance for either the common variant PRS or the rare variant PRS to explain individuals at the phenotype outlier thresholds of 99th and 99.9th percentiles.

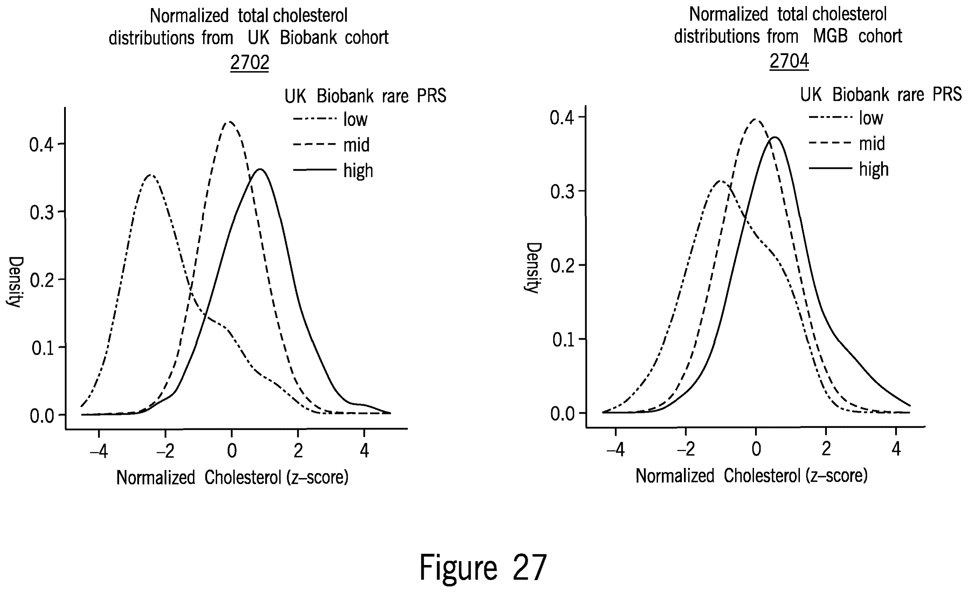

Figure 27 comprises graphs 2702 and 2704 of normalized total cholesterol distributions from two separate cohorts. Graph 2702 shows the normalized total cholesterol distributions from UK Biobank individuals who are in the bottom 0.5% (low), 0.5-99.5% (mid), and top 0.5% (high) groups for the cholesterol PRS, with 50% of the cohort used to train the PRS and the remainder for testing. Graph 2704 shows the normalized total cholesterol distributions from MGB individuals who are in the bottom 0.5% (low), 0.5-99.5% (mid), and top 0.5% (high) groups for the cholesterol PRS, with 50% of the cohort used to train the PRS and the remainder for testing.

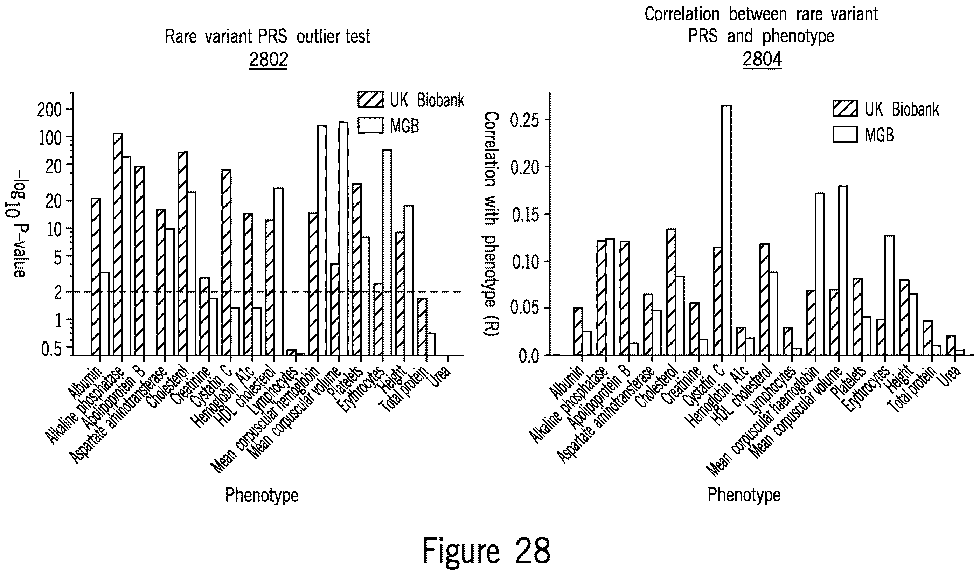

Figure 28 comprises graphs 2802 and 2804 comparing rare variant PRS outliers and phenotypes between two separate cohorts. Graph 2802 shows a rare variant PRS outlier test for 17 quantitative phenotypes that were measured in both cohorts. PRS and phenotype outliers were defined as individuals in the top 0.5% and bottom 0.5% of the population. Graph 2804 shows the correlation between rare variant PRS and phenotype for 17 quantitative phenotypes.

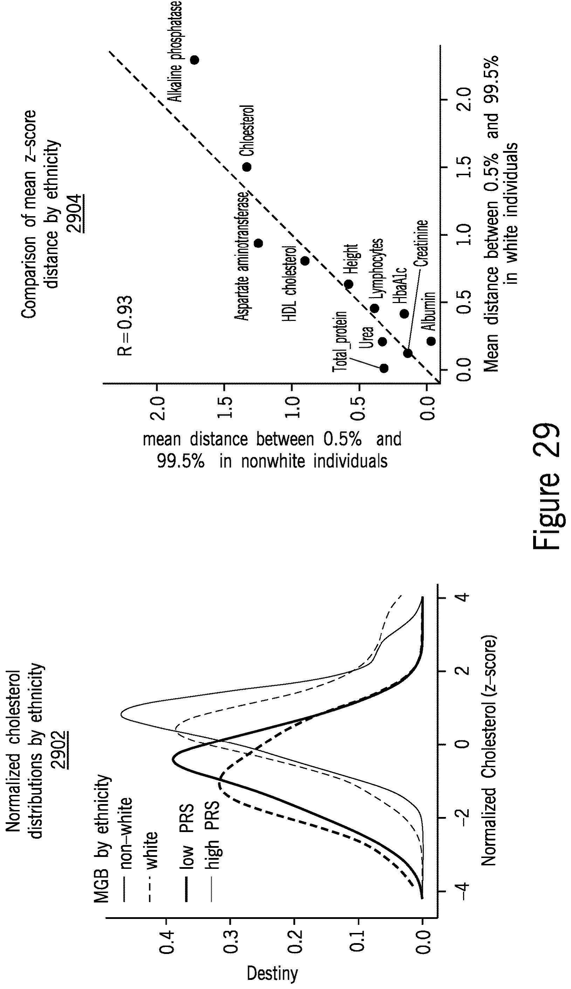

Figure 29 comprises graphs 2902 and 2904 illustrating performance results by ethnicity. Graph 2902 shows the normalized cholesterol distributions for low, and high PRS groups in MGB cohort, with results shown for both white and non-white individuals. Graph 2904 shows the comparison of mean z-score distance between low PRS (<0.5%) and high PRS (>99.5%) groups for each of the 11 phenotypes in MGB, for both white and non-white individuals.

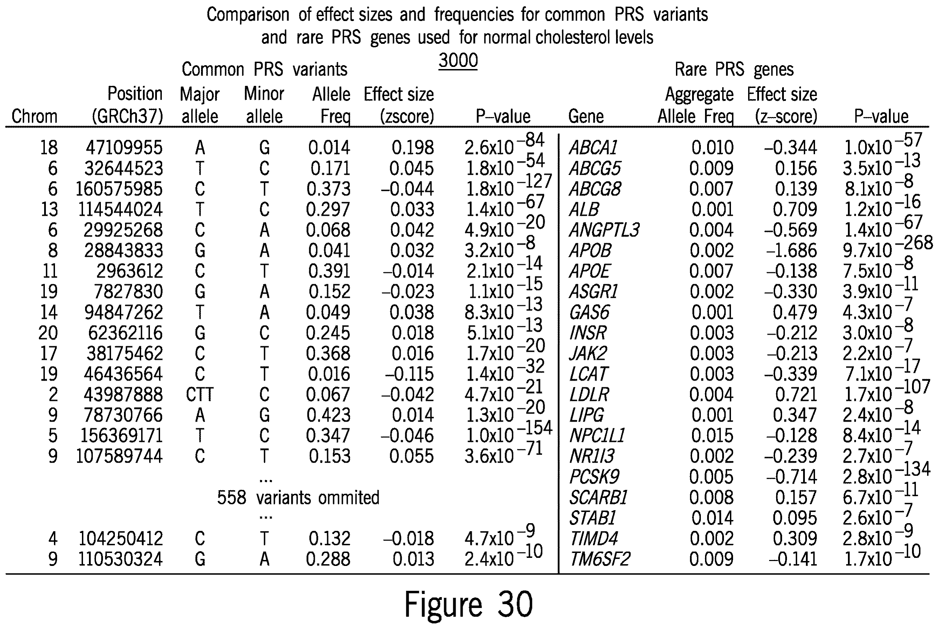

Figure 30 is a table 3000 comparing the effect sizes and frequencies for common PRS variants and rare PRS genes used for normal cholesterol levels.

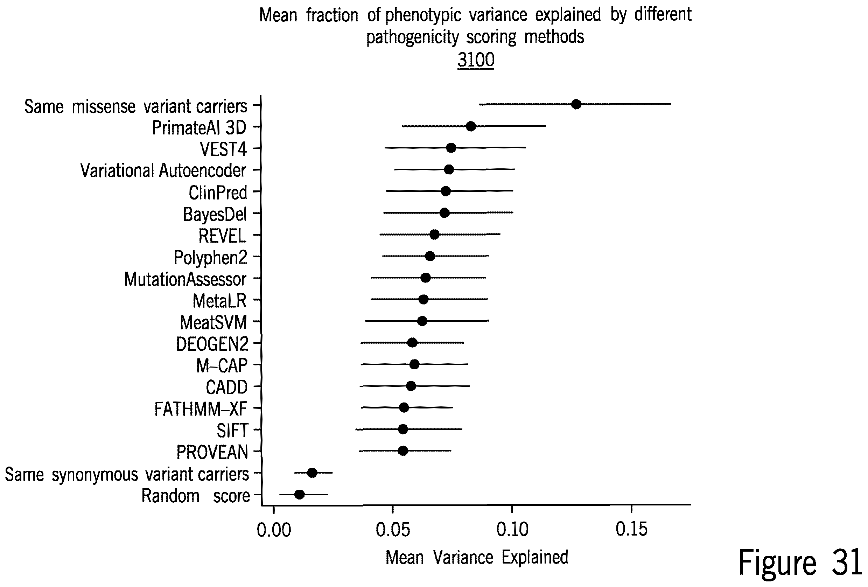

Figure 31 is a graph 3100 of the mean fraction of phenotypic variance explained by different pathogenicity scoring methods on a set of 34 gene-phenotype pairs, which were selected based on their enrichment for rare missense and LoF variants. The fraction phenotypic variance explained is computed as the squared Spearman correlation between each the scores from each scoring method and the phenotypic values of the carriers of the corresponding variants. The methods are compared in graph 3100 to a theoretical upper bound, which was computed by using phenotypic values of carriers of the same missense variants, and to two theoretical lower bounds - carriers of the same synonymous variants and random scores.

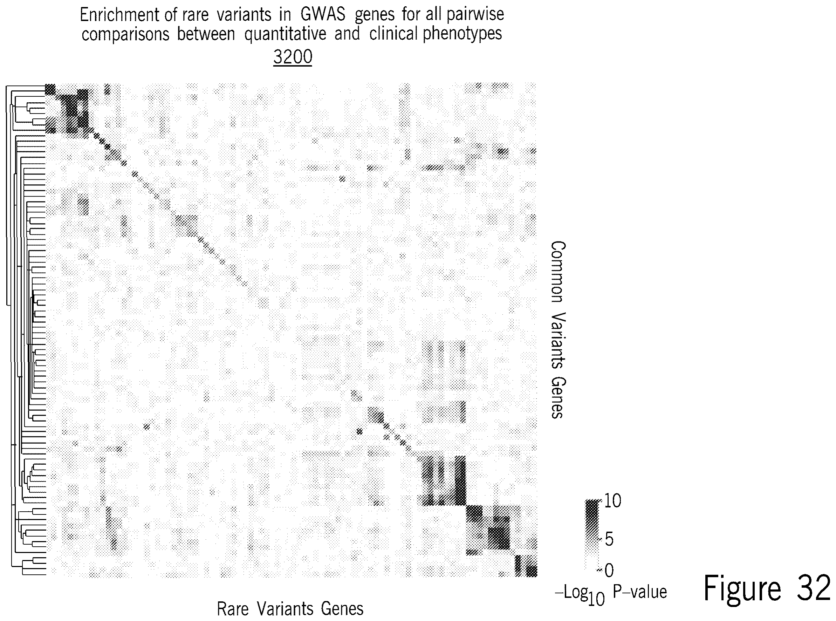

Figure 32 is a heatmap 3200 of the enrichment of rare variants in GWAS genes for all pairwise comparisons between quantitative and clinical phenotypes. For each pair of phenotypes, the heatmap 3200 shows the statistical significance of the ranking of the most significant subset of GWAS genes of one phenotype (y-axis) by their enrichment for rare deleterious variants affecting the second phenotype (x-axis). The burden tests for rare deleterious variants are computed after regressing out the effects of all significant GWAS variants from each phenotype. Heatmap 2100 shows a subset of the phenotypes which is enriched on the main diagonal in heatmap 3200.

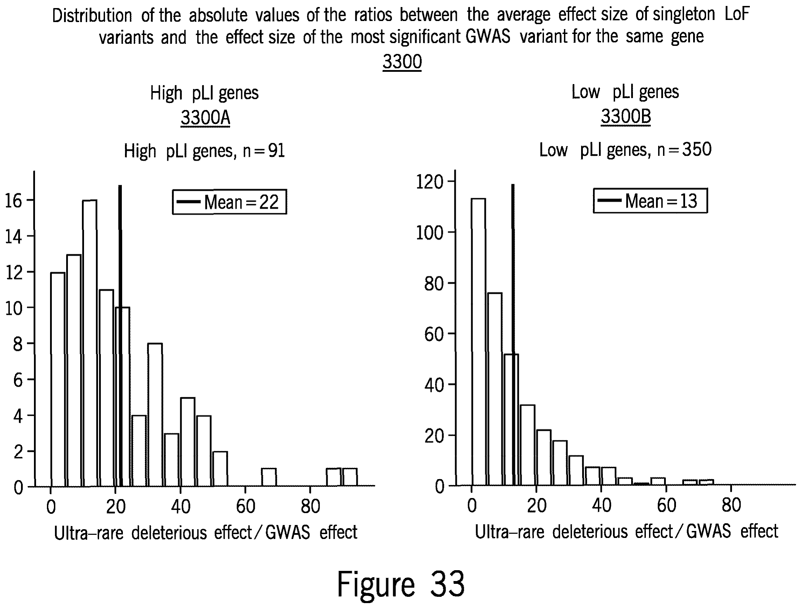

Figure 33 comprises graphs 3300 of the distribution of the absolute values of the ratios between the average effect size of singleton LoF variants and the effect size of the most significant GWAS variant for the same gene. Graph 3300A shows the distribution of the absolute values of the ratios between the average effect size of singleton LoF variants and the effect size of the most significant GWAS variant for the same gene for high pLI genes from graph 2200B. Graph 3300B shows the distribution of the absolute values of the ratios between the average effect size of singleton LoF variants and the effect size of the most significant GWAS variant for the same gene for low pLI genes from graph 2200B.

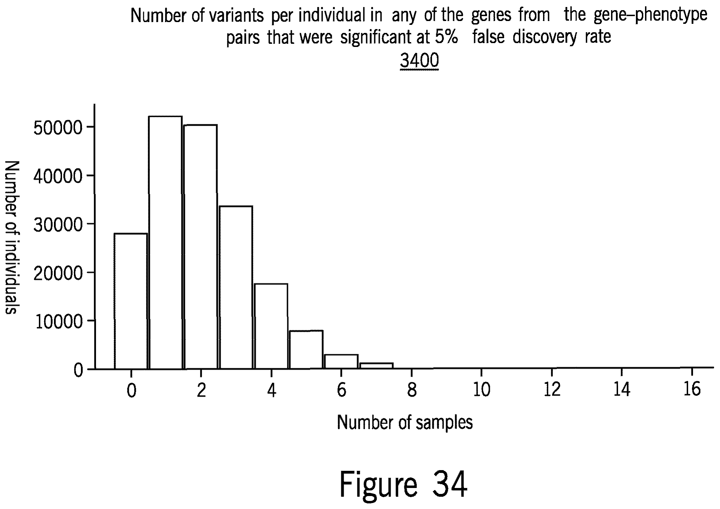

Figure 34 shows the number of variants per individual in any of the genes from the gene-phenotype pairs that were significant at 5% false discovery rate. Graph 3400 comprises 500 genes from 1031 gene-phenotype pairs that passed the significance threshold. The average number of variants per person is 2.03 and the standard deviation is 1.51.

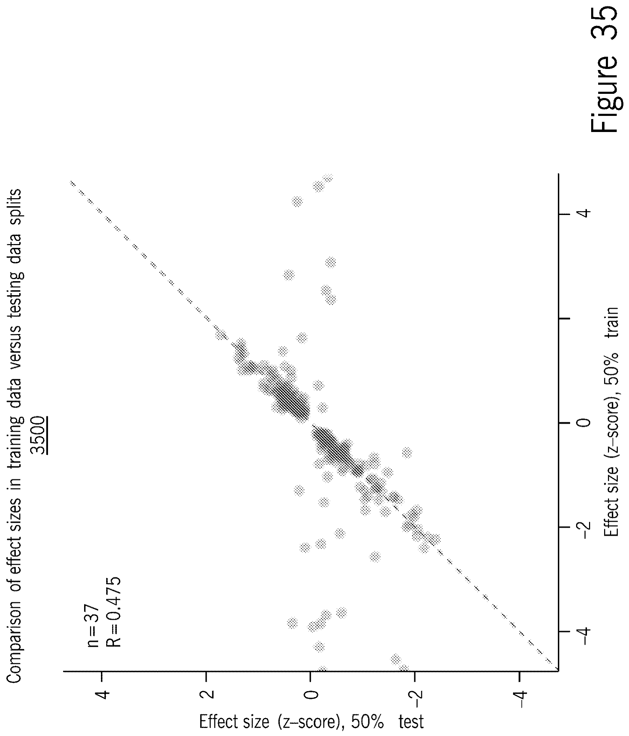

Figure 35 shows a comparison 3500 of effect sizes in training data versus testing data splits. Rare variant tests were performed using 50% of the cohort as a training set, and the other 50% as a testing set. In Graph 3500, effect sizes are compared from the training set versus the test set for genes with $p$-value $< 10^{-7}$ in the training set.

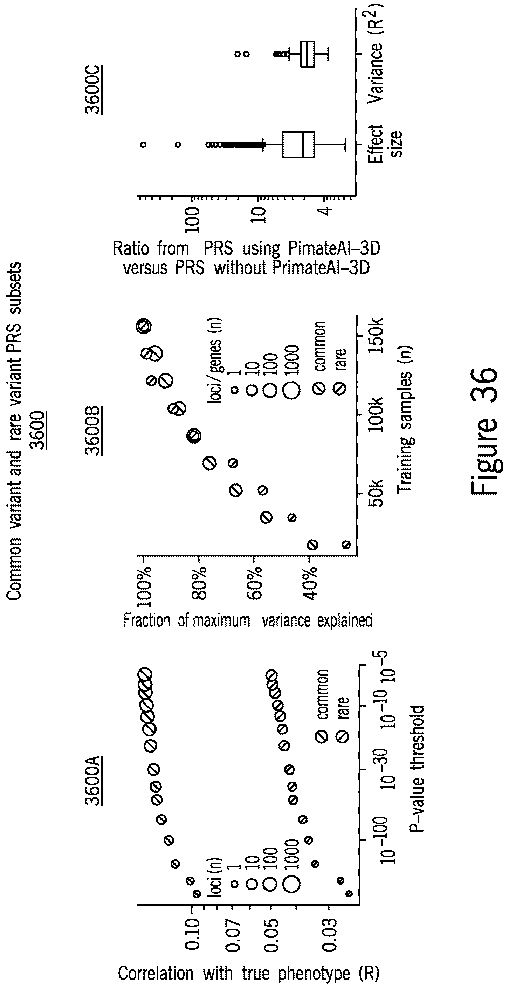

Figure 36 comprises graphs 3600 comparing common variant and rare variant PRS subsets. Graph 3600A shows common and rare PRS correlations when loci were required to be significant in both common and rare tests. PRSs were trained on 90%, tested on the remainder. Graph 3600B shows common and rare PRS correlations relative to maximum, by number of training samples. Graph 3600C shows the ratios of effect sizes and variance explained from rare PRS models which used PrimateAI-3D, versus rare variant PRS models constructed without PrimateAI-3D.

## Clauses

The technology disclosed, in particularly, the clauses disclosed in this section, can be practiced as a system, method, or article of manufacture. One or more features of an implementation can be combined with the base implementation. Implementations that are not mutually exclusive are taught to be combinable. One or more features of an implementation can be combined with other implementations. This disclosure periodically reminds the user of these options. Omission from some implementations of recitations that repeat these options should not be taken as limiting the combinations taught in the preceding sections - these recitations are hereby incorporated forward by reference into each of the following implementations.

One or more implementations and clauses of the technology disclosed or elements thereof can be implemented in the form of a computer product, including a non-transitory computer readable storage medium with computer usable program code for performing the method steps indicated. Furthermore, one or more implementations and clauses of the technology disclosed or elements thereof can be implemented in the form of an apparatus including a memory and at least one processor that is coupled to the memory and operative to perform exemplary method steps. Yet further, in another aspect, one or more implementations and clauses of the technology disclosed or elements thereof can be implemented in the form of means for carrying out one or more of the method steps described herein; the means can include (i) hardware module(s), (ii) software module(s) executing on one or more hardware processors, or (iii) a combination of hardware and software modules; any of (i)-(iii) implement the specific techniques set forth herein, and the software modules are stored in a computer readable storage medium (or multiple such media).

The clauses described in this section can be combined as features. In the interest of conciseness, the combinations of features are not individually enumerated and are not repeated with each base set of features. The reader will understand how features identified in the clauses described in this section can readily be combined with sets of base features identified as implementations in other sections of this application. These clauses are not meant to be mutually exclusive, exhaustive, or restrictive; and the technology disclosed is not limited to these clauses but rather encompasses all possible combinations, modifications, and variations within the scope of the claimed technology and its equivalents.

Other implementations of the clauses described in this section can include a non-transitory computer readable storage medium storing instructions executable by a processor to perform any of the clauses described in this section. Yet another implementation of the clauses described in this section can include a system including memory and one or more processors operable to execute instructions, stored in the memory, to perform any of the clauses described in this section.

We disclose the following clauses:

1. A computer-implemented method of performing an optimized burden test, including:
determining an optimal combination of a maximum allele count and a minimum pathogenicity score threshold that maximizes significance of burden testing effects of rare pathogenic variants in a particular gene on a particular phenotype, including:

grid searching a plurality of allele counts and a plurality of pathogenicity score thresholds, including:

generating a plurality of combinations of allele counts and pathogenicity score thresholds from the plurality of allele counts and the plurality of pathogenicity score thresholds;
identifying a plurality of groups of rare pathogenic variants corresponding to the plurality of combinations of allele counts and pathogenicity score thresholds; and
burden testing the plurality of groups of rare pathogenic variants in dependence upon a carrier status that separates carriers of a particular group of rare pathogenic variants in a cohort of individuals from non-carriers of the particular group of rare pathogenic variants in the cohort of individuals, and determining a plurality of effect sizes and p-values corresponding to the plurality of combinations of allele counts and pathogenicity score thresholds;

selecting, from the plurality of combinations of allele counts and pathogenicity score thresholds, a particular combination of an allele count and a pathogenicity score threshold that has a most significant p-value; and using the particular combination as the optimal combination.

2. The computer-implemented method of clause 1, wherein allele counts in the plurality of allele counts correspond to groups of rare pathogenic variants observed in the particular gene across the cohort of individuals.

3. The computer-implemented method of clause 2, wherein pathogenicity score thresholds in the plurality of pathogenicity score thresholds correspond to pathogenicity score quantiles of pathogenicity scores determined for rare pathogenic variants in the groups of rare pathogenic variants.

4. The computer-implemented method of clause 3, wherein the pathogenicity scores are generated by a convolutional neural network.

5. The computer-implemented method of clause 1, wherein the particular phenotype is a quantitative biomarker phenotype.

6. The computer-implemented method of clause 5, wherein the quantitative biomarker phenotype is burden tested using a two-tailed t-test.

7. The computer-implemented method of clause 6, wherein the two-tailed t-test is executed $a * p$ times in a nested fashion,

where $a$ is a number of allele counts in the plurality of allele counts, and
where $p$ is a number of the pathogenicity score thresholds in the plurality of pathogenicity score thresholds.

8. The computer-implemented method of clause 7, wherein intermediate statistics are shared between each execution of the two-tailed t-test.

9. The computer-implemented method of clause 1, wherein the particular phenotype is a categorical clinical diagnosis phenotype.

10. The computer-implemented method of clause 9, wherein the categorical clinical diagnosis phenotype is burden tested using logistic regression.

11. The computer-implemented method of clause 10, wherein the logistic regression is executed $a * p$ times in a nested fashion.

12. The computer-implemented method of clause 11, wherein intermediate statistics are shared between each execution of the logistic regression.

13. The computer-implemented method of clause 1, wherein the grid searching further includes performing a first grid search through the plurality of allele counts.

14. The computer-implemented method of clause 1, wherein the grid searching further includes performing a second grid search through the plurality of pathogenicity score thresholds.

15. The computer-implemented method of clause 1, further including correcting the most significant p-value using an adaptive permutation false discovery rate.

16. The computer-implemented method of clause 1, further including correcting the most significant p-value using a Benjamini-Hochberg false discovery rate.

17. A system including one or more processors coupled to memory, the memory loaded with computer instructions to perform an optimized burden test, the instructions, when executed on the processors, implement actions comprising:

determining an optimal combination of a maximum allele count and a minimum pathogenicity score threshold that maximizes significance of burden testing effects of rare pathogenic variants in a particular gene on a particular phenotype, including:

grid searching a plurality of allele counts and a plurality of pathogenicity score thresholds, including:

generating a plurality of combinations of allele counts and pathogenicity score thresholds from the plurality of allele counts and the plurality of pathogenicity score thresholds;
identifying a plurality of groups of rare pathogenic variants corresponding to the plurality of combinations of allele counts and pathogenicity score thresholds; and
burden testing the plurality of groups of rare pathogenic variants in dependence upon a carrier status that separates carriers of a particular group of rare pathogenic variants in a cohort of individuals from non-carriers of the particular group of rare pathogenic variants in the cohort of individuals, and determining a plurality of effect sizes and p-values corresponding to the plurality of combinations of allele counts and pathogenicity score thresholds;

selecting, from the plurality of combinations of allele counts and pathogenicity score thresholds, a particular combination of an allele count and a pathogenicity score threshold that has a most significant p-value; and
using the particular combination as the optimal combination.

18. The system of clause 17, wherein allele counts in the plurality of allele counts correspond to groups of rare pathogenic variants observed in the particular gene across the cohort of individuals.

19. The system of clause 18, wherein pathogenicity score thresholds in the plurality of pathogenicity score thresholds correspond to pathogenicity score quantiles of pathogenicity scores determined for rare pathogenic variants in the groups of rare pathogenic variants.

20. The system of clause 19, wherein the pathogenicity scores are generated by a convolutional neural network.

21. The system of clause 17, wherein the particular phenotype is a quantitative biomarker phenotype.

22. The system of clause 21, wherein the quantitative biomarker phenotype is burden tested using a two-tailed t-test.

23. The system of clause 22, wherein the two-tailed t-test is executed $a * p$ times in a nested fashion,

where $a$ is a number of allele counts in the plurality of allele counts, and
where $p$ is a number of the pathogenicity score thresholds in the plurality of pathogenicity score thresholds.

24. The system of clause 23, wherein intermediate statistics are shared between each execution of the two-tailed t-test.

25. The system of clause 17, wherein the particular phenotype is a categorical clinical diagnosis phenotype.

26. The system of clause 25, wherein the categorical clinical diagnosis phenotype is burden tested using logistic regression.

27. The system of clause 26, wherein the logistic regression is executed $a * p$ times in a nested fashion.

28. The system of clause 27, wherein intermediate statistics are shared between each execution of the logistic regression.

29. The system of clause 17, wherein the grid searching further includes performing a first grid search through the plurality of allele counts.

30. The system of clause 17, wherein the grid searching further includes performing a second grid search through the plurality of pathogenicity score thresholds.

31. The system of clause 17, further implementing actions comprising correcting the most significant p-value using an adaptive permutation false discovery rate.

32. The system of clause 17, further implementing actions comprising correcting the most significant p-value using a Benjamini-Hochberg false discovery rate.

33. A non-transitory computer readable storage medium impressed with computer program instructions perform an optimized burden test, the instructions, when executed on a processor, implement a method comprising:
determining an optimal combination of a maximum allele count and a minimum pathogenicity score threshold that maximizes significance of burden testing effects of rare pathogenic variants in a particular gene on a particular phenotype, including:

grid searching a plurality of allele counts and a plurality of pathogenicity score thresholds, including:

generating a plurality of combinations of allele counts and pathogenicity score thresholds from the plurality of allele counts and the plurality of pathogenicity score thresholds;
identifying a plurality of groups of rare pathogenic variants corresponding to the plurality of combinations of allele counts and pathogenicity score thresholds; and
burden testing the plurality of groups of rare pathogenic variants in dependence upon a carrier status that separates carriers of a particular group of rare pathogenic variants in a cohort of individuals from non-carriers of the particular group of rare pathogenic variants in the cohort of individuals, and determining a plurality of effect sizes and p-values corresponding to the plurality of combinations of allele counts and pathogenicity score thresholds;

selecting, from the plurality of combinations of allele counts and pathogenicity score thresholds, a particular combination of an allele count and a pathogenicity score threshold that has a most significant p-value; and
using the particular combination as the optimal combination.

34. The non-transitory computer readable storage medium of clause 33, wherein allele counts in the plurality of allele counts correspond to groups of rare pathogenic variants observed in the particular gene across the cohort of individuals.

35. The non-transitory computer readable storage medium of clause 34, wherein pathogenicity score thresholds in the plurality of pathogenicity score thresholds correspond to pathogenicity score quantiles of pathogenicity scores determined for rare pathogenic variants in the groups of rare pathogenic variants.

36. The non-transitory computer readable storage medium of clause 35, wherein the pathogenicity scores are generated by a convolutional neural network.

37. The non-transitory computer readable storage medium of clause 33, wherein the particular phenotype is a quantitative biomarker phenotype.

38. The non-transitory computer readable storage medium of clause 37, wherein the quantitative biomarker phenotype is burden tested using a two-tailed t-test.

39. The non-transitory computer readable storage medium of clause 38, wherein the two-tailed t-test regression is executed $a * p$ times in a nested fashion,

where $a$ is a number of allele counts in the plurality of allele counts, and
where p is a number of the pathogenicity score thresholds in the plurality of pathogenicity score thresholds.

40. The non-transitory computer readable storage medium of clause 39, wherein intermediate statistics are shared between each execution of the two-tailed t-test.

41. The non-transitory computer readable storage medium of clause 33, wherein the particular phenotype is a categorical clinical diagnosis phenotype.

42. The non-transitory computer readable storage medium of clause 41, wherein the categorical clinical diagnosis phenotype is burden tested using logistic regression.

43. The non-transitory computer readable storage medium of clause 42, wherein the logistic regression is executed $a * p$ times in a nested fashion.

44. The non-transitory computer readable storage medium of clause 43, wherein intermediate statistics are shared between each execution of the logistic regression.

45. The non-transitory computer readable storage medium of clause 33, wherein the grid searching further includes performing a first grid search through the plurality of allele counts.

46. The non-transitory computer readable storage medium of clause 33, wherein the grid searching further includes performing a second grid search through the plurality of pathogenicity score thresholds.

47. The non-transitory computer readable storage medium of clause 33, implementing the method further comprising correcting the most significant p-value using an adaptive permutation false discovery rate.

48. The non-transitory computer readable storage medium of clause 33, implementing the method further comprising correcting the most significant p-value using a Benjamini-Hochberg false discovery rate.


**Claims**

1. A method for determining a weighted variant polygenic risk score (PRS), comprising:

   accessing or acquiring genomic data and phenotypic data for a cohort of individuals, wherein the genomic data comprises gene sequence data for each individual and the phenotypic data comprises phenotypic measurement data for each individual, wherein the gene sequence data further comprises a respective carrier status for each variant and for each individual;
   for each gene of a plurality of genes, obtaining a plurality of gene-specific effective strength scores by:

      modeling a relationship between genotypes determined from the genomic data and phenotypes determined from the phenotypic data;
      determining, based on the relationship, an effective strength score of a strength of association in the cohort between carrier status of a plurality of variants of each gene and a phenotypic response;

   determining a weighted burden score based on the plurality of gene-specific effective strength scores, wherein the weighted burden score comprises a variant polygenic risk score determined by summing respective products of effect size and carrier status for each gene of the plurality of genes.

2. The method of claim 1, wherein the variant polygenic risk score comprises a rare variant polygenic risk score, wherein a respective variant is determined to be rare based on an occurrence rate of the respective variant in a population being below a threshold.

3. The method of claim 2, wherein the threshold is gene-specific.

4. The method of any one of claims 1-3 wherein the phenotypic measurement data comprises quantitative biomarker phenotype measurement data.

5. The method of claim 4, wherein the quantitative biomarker phenotype is burden tested using a two-tailed t-test.

6. The method of any one of claims 1-4 wherein the phenotypic measurement data comprises categorical clinical diagnosis phenotype data.

7. The method of any one of claims 1-6, wherein the respective carrier status for each variant and for each individual is determined on a gene-resolution basis

8. The method of any one of claims 1-7, wherein a respective individual that possesses at least one rare deleterious variant within a particular gene is a carrier at the particular gene.

9.  The method of any one of claims 1-8, wherein a respective individual that does not possesses at least one rare deleterious variant within a particular gene is not a carrier at the particular gene.

10. The method of any one of claims 1-9, wherein determining the effective strength score comprises applying a t-test.

11. The method of claim 10, wherein a respective t-test is performed for the plurality of genes to obtain a plurality of gene-specific effective strength scores for a particular shared phenotypic response.

12. The method of claim 11, wherein the weighted burden score is determined based on the plurality of gene-specific effective strength scores.

13. The method of any one of claims 1-12, wherein the genomic data further comprises allele counts corresponding to groups of variants observed in a particular gene across the cohort of individuals.

14. A system including one or more processors coupled to memory, the memory loaded with computer instructions to determine a weighted variant polygenic risk score (PRS), the instructions, when executed on the processors, implement actions comprising the method according to any one of claims 1 to 13.

15. A non-transitory computer readable storage medium impressed with computer program instructions to determine a weighted variant polygenic risk score (PRS), the instructions, when executed on a processor, implement a method according to any one of claims 1 to 13.

### Cohort i 104

| | Variant A Carrier? | Variant B Carrier? | Variant C Carrier? | Phenotype D |
|---|---|---|---|---|
| Individual 1 | Y | Y | N | $x_1$ |
| Individual 2 | N | N | Y | $x_2$ |
| ... | | | | |
| Individual n | Y | N | Y | $x_n$ |

**102**
Genomic and Phenotypic Data from Cohort i

**124**

**Model relationship between a particular genotype and a particular phenotype 122**

Phenotype D

p-value
$\beta$

noncarrier        carrier
Rare deleterious variant carrier status

**Determine effective strength score of the strength of association in the cohort between carrier status of a plurality of rare variants and a phenotypic response 142**

**144**

t-test

$H_0: \Delta(|\,\overline{X}_{carrier} - \overline{X}_{noncarrier}\,|) = 0$

$H_0: \Delta(|\,\overline{X}_{carrier} - \overline{X}_{noncarrier}\,|) \neq 0$

**Output weighted burden score 162**

**164**

Rare Variant Polygenic Risk Score = $\sum$ (Effect Size * Carrier Status)

## Figure 1A

| 108 | Gene | Effect Size | Carrier Status |
|-----|------|-------------|----------------|
| 118 | Gene A | Effect Size A | Carrier |
| 128 | Gene B | Effect Size B | NonCarrier |
| 138 | Gene C | Effect Size C | Carrier |
| 148 | Gene D | Effect Size F | Carrier |
| 158 | Gene E | Effect Size E | Carrier |

Output weighted burden score
162

164
Rare Variant Polygenic Risk Score = $\Sigma$ (Effect Size * Carrier Status)

182
Rare Variant Polygenic Risk Score for Phenotype Y = Effect Size A + Effect Size C + Effect Size D + Effect Size E

Figure 1B

Computer System
200

Storage Subsystem
210

Memory Subsystem
222

| RAM 232 | ROM 234 |

File Storage
Subsystem
236

Rare Variant Polygenic Risk Score
Model
240

User Interface
Input Devices
238

Bus Subsystem 242

CPU
244

Network Interface
Subsystem
246

User Interface
Output Devices
248

Processors
(GPU, FPGA, CGRA)
249

Figure 2

Genetic Sequence A 302

| 1 | 2 | 2 | 4 | 5 | 6 | 7 | 8 | ... | $N^{th}$ |
|---|---|---|---|---|---|---|---|-----|----------|
| A | ... | ... | ... | C | ... | ... | ... | ... | G |

— 304

Single Nucleotide
Variant

Variant 1A 322

| 1 | 2 | 2 | 4 | 5 | 6 | 7 | 8 | ... | $N^{th}$ |
|---|---|---|---|---|---|---|---|-----|----------|
| A | ... | ... | ... | A | ... | ... | ... | ... | G |

— 324

Single Nucleotide
Variant

Variante 1B 342

| 1 | 2 | 2 | 4 | 5 | 6 | 7 | 8 | ... | $N^{th}$ |
|---|---|---|---|---|---|---|---|-----|----------|
| A | ... | ... | ... | T | ... | ... | ... | ... | G |

— 344

Figure 3

EP 4 742 250 A2

Genetic Sequence A <u>402</u>

| 1 | 4 | 4 | 4 | 5 | 6 | 7 | 4 | ... | N<sup>th</sup> |
|---|---|---|---|---|---|---|---|-----|-----|
| A | ... | ... | ... | C | ... | ... | ... | ... | G |

<u>404</u>
Single Nucleotide
Variant

Variant 1A <u>422</u>

| 1 | 4 | 4 | 4 | 5 | 6 | 7 | 4 | ... | N<sup>th</sup> |
|---|---|---|---|---|---|---|---|-----|-----|
| A | ... | ... | ... | <u>A</u> | ... | ... | ... | ... | G |

<u>424</u>
Single Nucleotide
Variant

Variant 1B <u>442</u>

| 1 | 4 | 4 | 4 | 5 | 6 | 7 | 4 | ... | N<sup>th</sup> |
|---|---|---|---|---|---|---|---|-----|-----|
| A | ... | ... | ... | <u>T</u> | ... | ... | ... | ... | G |

<u>444</u>
Single Nucleotide
Variant

Variant 1C <u>462</u>

| 1 | 4 | 4 | 4 | 5 | 6 | 7 | 4 | ... | N<sup>th</sup> |
|---|---|---|---|---|---|---|---|-----|-----|
| A | ... | ... | ... | <u>G</u> | ... | ... | ... | ... | G |

<u>464</u>

Protein A <u>406</u>

Native

Protein 1A <u>426</u>

Missense

Protein 1B <u>446</u>

Synonymous

Protein 1C <u>466</u>

Nonsense

Phenotype A <u>408</u>

Phenotype 1A <u>428</u>

Phenotype 1B <u>448</u>

Phenotype 1C <u>468</u>

Figure 4

Figure 5

Figure 6

Figure 7

| Cohort i 804 | | | | |
|---|---|---|---|---|
| | Variant A Carrier? | Variant B Carrier? | Variant C Carrier? | Phenotype D |
| Individual 1 | Y | Y | N | $x_1$ |
| Individual 2 | N | N | Y | $x_1$ |
| ... | | | | |
| Individual n | Y | N | Y | $x_n$ |

802

Genomic and Phenotypic Data from Cohort i

Filter by Pathogenicity Score Thresholds and Allele Counts to grid search over $(PST_m, AC_n)$

Pathogenicity Score Thresholds

822

Allele Count

$(PST_m, AC_n)$ ☐ = 824

t-test

$H_0$ : The difference in phenotypic value for individuals who are carriers for a group of variants and phenotypic value for individuals who are not carriers for the group of variants is not statistically significant.

$H_a$: The difference in phenotypic value for individuals who are carriers for a group of variants and phenotypic value for individuals who are not carriers for the group of variants is statistically significant

For each (PST, AC) in $(PST_m, AC_n)$, obtain p-value

842

| $(PST_1, AC_1)$ | $p_1$ |
|---|---|
| $(PST_2, AC_1)$ | $p_2$ |
| ... | ... |
| $(PST_m, AC_m)$ | $p_n$ |

| Sub-Cohort $(PST_m, AC_n)$  844 | | | | |
|---|---|---|---|---|
| | Variant A Carrier? | Variant B Filtered Out | Variant C Carrier? | Phenotype D |
| Individual 1 | Y | | N | $x_1$ |
| Individual 2 | N | | Y | $x_1$ |
| ... | | | | |
| Individual n | Y | | Y | $x_n$ |

Identify combination of PST and AC corresponding to the most significant p-value

862

$(PST_{OPTIMAL}, AC_{OPTIMAL})$

# Figure 8

Protein Structure-Based Pathogenicity Determination 900

| Access reference and alternative amino acid sequences 902 | ← | Sequence Accessor 904 |

| Generate 3D protein structures for a reference amino acid sequence 912 | ← | 9D Structure Generator 914 |

| Classify atomic coordinates of the 9D protein structures on an amino acid-basis 922 | ← | Coordinate Classifier 924 |

| Instantiate a voxel grid 932 | ← | Voxel Grid Generator 934 |

| Center the voxel grid at the reference amino acid experiencing a target variant 942 | ← | Voxel Grid Centerer 944 |

| Generate amino acid-wise distance channels for voxels in the voxel grid 952 | ← | Distance Channel Generator 954 |

| Generate a reference one-hot encoding and an alternative one-hot encoding 962 | ← | One-Hot Encoder 964 |

| Concatenate the amino acid-wise distance channels and the reference and alternative one-hot encodings 972 | ← | Concatenator 974 |

| Process the concatenated amino acid-wise distance channels and the reference and alternative one-hot encodings through a pathogenicity classifier to determine a pathogenicity of the target variant 982 | ← | Runtime Logic 984 |

Figure 9

Pathogenicity Classifier  1000

| | |
|---|---|
| Input | 1002 |

15x15x15x8  1004

| | |
|---|---|
| 1x1x1 CONV, 64, ReLU, BN | 1006 |

15x15x15x64  1008

| | |
|---|---|
| 3x3x3 CONV, 64, ReLU, BN | 1010 |

13x13x13x64  1012

| | |
|---|---|
| 3x3x3 CONV, 64, ReLU, BN | 1014 |

11x11x11x64  1016

| | |
|---|---|
| 3x3x3 CONV, 64, ReLU, BN | 1018 |

9x9x9x64  1020

| | |
|---|---|
| 3x3x3 CONV, 64, ReLU, BN | 1022 |

7x7x7x74  1024

| | |
|---|---|
| 3x3x3 CONV, 64, ReLU, BN | 1026 |

5x5x5x64  1028

| | |
|---|---|
| 3x3x3 CONV, 64, ReLU, BN | 1030 |

3x3x3x64  1032

| | |
|---|---|
| 3x3x3 CONV, 64, ReLU, BN | 1034 |

1x1x1x64  1036

| | |
|---|---|
| FC 512, ReLU | 1038 |

Dropout 80%  1040

| | |
|---|---|
| FC 2, Softmax | 1042 |

Figure 10

Computer System
1100

Storage Subsystem
1110

Memory Subsystem
1122

RAM
1132

ROM
1134

File Storage
Subsystem
1136

Pathogenicity Classifier
1000

User Interface
Input Devices
1138

Bus Subsystem 1140

CPU
1142

Network Interface
Subsystem
1144

User Interface
Output Devices
1446

Processors
(GPU, FPGA, CGRA)
1148

Figure 11

Final Pathogenicity Score Determination 1200

The pathogenicity predictor generates a first pathogenicity score for a first alternate amino acid that is same as a first reference amino acid
1202

The pathogenicity predictor generates a second pathogenicity score for a second alternate amino acid that is different from the first reference amino acid
1212

A final pathogenicity score for the second alternate amino acid is the second pathogenicity score
1222

Alt 1

The final pathogenicity score for the second alternate amino acid is a ratio of the second pathogenicity score over a sum of the first pathogenicity score and the second pathogenicity score
1222a

Alt 2

The final pathogenicity score for the second alternate amino acid is determined by subtracting the first pathogenicity score from the second pathogenicity score
1222b

Figure 12

False discovery rate corrected p-value
1302

If value is in [0, 1e-5]

If value is in (1e-5, 0.01]

If value is in (0.01,1]

Benjamini–Hochberg false discovery rate correction
1310

Set N = 0 (count of total number of generated permutations)
1312

Generate 10000 permutations of the phenotype labels
1314

Generate 1000 permutations of the phenotype labels. Set N = N + 1000
1322

Fit a generalized extreme value distribution to the absolute values of the test statistics from the permuted data
1324

If N ≤ (100 / p)

Perform burden test on each permutation and count the fraction of permutations that yielded more significant p-values than the observed data, p
1342

Estimate the corrected p-value from the area under the curve of the fitted distribution
1344

If N < (100 / p)

Stop and output p, guaranteeing that N < 10000
1362

Figure 13

Correction for Multiple Testing
1300

Pruning quantitative phenotypes into
a non-redundant set
1402

Grouping a plurality of drugs into
a set of drug categories
1404

Correcting each phenotype for drug usage (e.g.,
statins, blood pressure medication)
1406

Inverse-rank normal transformation
1408

Covariate correction (e.g., age, gender, genetic PCs,
diet, smoking status)
1410

Correction for Covariates
1400

# Figure 14

Figure 15

Figure 16

Demographic
1702

Psychosocial Factors
1722

Tobacco and Alcohol
Consumption
1742

Cardiovascular Disease
Patient X
1700

Drug Use
1704

Body Mass
Measurements
1724

Diet and Lifestyle
Factors
1744

Figure 17

Total number of significant gene–phenotype pairs
identified for different types of burden tests

<u>1800</u>

Figure 18

LDLR–LDL Cholesterol and Disorders of lipoprotein metabolism

PrimateAI–3D Percentile
1902

PrimateAI–3D Percentile
1904

Figure 19A

PCSK9–LDL Cholesterol

Figure 19B

Figure 19C

Average absolute Spearman correlation of different pathogenicity scores with phenotype values

2000

Figure 20

Figure 21

Heatmap comparison of rare deleterious variants and common GWAS variants 2100

GWAS hits (commom variants)

Exome hits (rare variants)

$-Log_{10}$ P-value

Comparison of rare deleterious variants
and common GWAS variants

2200

Figure 22

EP 4 742 250 A2

Cholesterol Pathway
2302

Figure 23A

Figure 23B

Average correlation of common variant and
rare variant PRS with 72 phenotypes
2402

Polygenic Risk Score performance
2404

Figure 24

EP 4 742 250 A2

Enrichment of PRS outliers

2500

Figure 25

Figure 26

Normalized total cholesterol
distributions from UK Biobank cohort
2702

Normalized total cholesterol
distributions from MGB cohort
2704

Figure 27

Figure 28

Comparison of mean z-score
distance by ethnicity
2904

R=0.93

Alkaline phosphatase

Chloesterol

Aspartate aminotransferase

HDL cholesterol

Height

Lymphocytes

Total_protein

Urea

HbaA1c

Creatinine

Albumin

mean distance between 0.5% and
99.5% in nonwhite individuals

Mean distance between 0.5% and 99.5%
in white individuals

Normalized cholesterol
distributions by ethnicity
2902

MGB by ethnicity
—— non-white
--- white
—— low PRS
—— high PRS

Destiny

Normalized Cholesterol (z-score)

Figure 29

Comparison of effect sizes and frequencies for common PRS variants
and rare PRS genes used for normal cholesterol levels

| | | Common PRS variants | | | | 3000 | | Rare PRS genes | | |
|---|---|---|---|---|---|---|---|---|---|---|
| Chrom | Position (GRCh37) | Major allele | Minor allele | Allele Freq | Effect size (zscore) | P–value | Gene | Aggregate Allele Freq | Effect size (z–score) | P–value |
| 18 | 47109955 | A | G | 0.014 | 0.198 | $2.6 \times 10^{-84}$ | ABCA1 | 0.010 | −0.344 | $1.0 \times 10^{-57}$ |
| 6 | 32644523 | T | C | 0.171 | 0.045 | $1.8 \times 10^{-54}$ | ABCG5 | 0.009 | 0.156 | $3.5 \times 10^{-13}$ |
| 6 | 160575985 | C | T | 0.373 | −0.044 | $1.8 \times 10^{-127}$ | ABCG8 | 0.007 | 0.139 | $8.1 \times 10^{-8}$ |
| 13 | 114544024 | T | C | 0.297 | 0.033 | $1.4 \times 10^{-67}$ | ALB | 0.001 | 0.709 | $1.2 \times 10^{-16}$ |
| 6 | 29925268 | C | A | 0.068 | 0.042 | $4.9 \times 10^{-20}$ | ANGPTL3 | 0.004 | −0.569 | $1.4 \times 10^{-67}$ |
| 8 | 28843833 | G | A | 0.041 | 0.032 | $3.2 \times 10^{-8}$ | APOB | 0.002 | −1.686 | $9.7 \times 10^{-268}$ |
| 11 | 2963612 | C | T | 0.391 | −0.014 | $2.1 \times 10^{-14}$ | APOE | 0.007 | −0.138 | $7.5 \times 10^{-8}$ |
| 19 | 7827830 | G | A | 0.152 | −0.023 | $1.1 \times 10^{-15}$ | ASGR1 | 0.002 | −0.330 | $3.9 \times 10^{-11}$ |
| 14 | 94847262 | T | A | 0.049 | 0.038 | $8.3 \times 10^{-13}$ | GAS6 | 0.001 | 0.479 | $4.3 \times 10^{-7}$ |
| 20 | 62362116 | G | C | 0.245 | 0.018 | $5.1 \times 10^{-13}$ | INSR | 0.003 | −0.212 | $3.0 \times 10^{-8}$ |
| 17 | 38175462 | C | T | 0.368 | 0.016 | $1.7 \times 10^{-20}$ | JAK2 | 0.003 | −0.213 | $2.2 \times 10^{-7}$ |
| 19 | 46436564 | C | T | 0.016 | −0.115 | $1.4 \times 10^{-32}$ | LCAT | 0.003 | −0.339 | $7.1 \times 10^{-17}$ |
| 2 | 43987888 | CTT | C | 0.067 | −0.042 | $4.7 \times 10^{-21}$ | LDLR | 0.004 | 0.721 | $1.7 \times 10^{-107}$ |
| 9 | 78730766 | A | G | 0.423 | 0.014 | $1.3 \times 10^{-20}$ | LIPG | 0.001 | 0.347 | $2.4 \times 10^{-8}$ |
| 5 | 156369171 | T | C | 0.347 | −0.046 | $1.0 \times 10^{-154}$ | NPC1L1 | 0.015 | −0.128 | $8.4 \times 10^{-14}$ |
| 9 | 107589744 | C | T | 0.153 | 0.055 | $3.6 \times 10^{-71}$ | NR1I3 | 0.002 | −0.239 | $2.7 \times 10^{-7}$ |
| | | | | | | | PCSK9 | 0.005 | −0.714 | $2.8 \times 10^{-134}$ |
| | | ... 558 variants ommited ... | | | | | SCARB1 | 0.008 | 0.157 | $6.7 \times 10^{-11}$ |
| | | | | | | | STAB1 | 0.014 | 0.095 | $2.6 \times 10^{-7}$ |
| 4 | 104250412 | C | T | 0.132 | −0.018 | $4.7 \times 10^{-9}$ | TIMD4 | 0.002 | 0.309 | $2.8 \times 10^{-9}$ |
| 9 | 110530324 | G | A | 0.288 | 0.013 | $2.4 \times 10^{-10}$ | TM6SF2 | 0.009 | −0.141 | $1.7 \times 10^{-10}$ |

Figure 30

Mean fraction of phenotypic variance explained by different
pathogenicity scoring methods

3100

Figure 31

Enrichment of rare variants in GWAS genes for all pairwise
comparisons between quantitative and clinical phenotypes
3200

Common Variants Genes

Rare Variants Genes

10

5

0

$-Log_{10}$ P-value

Figure 32

Distribution of the absolute values of the ratios between the average effect size of singleton LoF
variants and the effect size of the most significant GWAS variant for the same gene

<u>3300</u>

High pLI genes
<u>3300A</u>

Low pLI genes
<u>3300B</u>

Figure 33

Figure 34

Comparison of effect sizes in training data versus testing data splits
3500

n=37
R=0.475

Effect size (z-score), 50% test

Effect size (z-score), 50% train

Figure 35

Common variant and rare variant PRS subsets
3600

3600A

3600B

3600C

Figure 36

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63294813
- US 63294816
- US 63294820
- US 63294827
- US 63294828
- US 63294830
- US 63351283
- US 63351299
- US 63351317
- US 62573144
- US 62573149
- US 62573153
- US 62582898
- US 16090318
- US 16098618
- US 16096818
- US 16097818
- US 40714919
- US 23205621
- US 63175495
- US 63175767
- US 46841121
- US 63253122
- US 63281579
- US 63281592
- US 160903
- US 16160986
- US 16160968
- US 16407149 B

**Non-patent literature cited in the description**

- **SUNDARAM, L. et al.** Predicting the clinical impact of human mutation with deep neural networks.. *Nat. Genet.*, 2018, vol. 50, 1161-1170
- **JAGANATHAN, K. et al.** Predicting splicing from primary sequence with deep learning.. *Cell*, 2019, vol. 176, 535-548